# EUROPEAN PATENT APPLICATION

(11) **EP 1 842 855 A2**
(43) Date of publication of application: **10.10.2007**
(21) Application number: 07105121.3
(22) Date of filing: 28.03.2007
(51) Int. Cl.: C07H 9/04, C07H 15/00, C07H 3/02, A61K 31/70, A61K 31/7004, A61K 31/7028, A61K 31/7042, A61P 37/00, A61P 35/00, A61P 17/00, A61P 11/06

(54) **MONOSACCHARIDE DERIVATIVES AS ANTI-INFLAMMATORY AND/OR ANTI-CANCER AGENTS**

(30) Priority: 29.03.2006 IN DE08752006
(71) Applicant: Ranbaxy Laboratories Limited, Gurgaon, Haryana 122001 (IN)
(72) Inventor: Sattigeri, Viswajanani Jitendra, 122001, Haryana (IN); Arora, Sudershan K., 411008, Maharashtra (IN); Salman, Mohammad, Princeton, New Jersey 08540 (US); Palle, Venkata P., 411007, Maharashtra (IN); Mukherji, Ashis, 110070, Delhi (IN); Verma, Ashwani Kumar, 110018, Dehli (IN); Malhotra, Sanjay, 110075, Dehli (IN); Dharmarahan, Sankaranarayanan, 122001, Haryana (IN); Ray, Abhijit, 110070, Dehli (IN); Shirumalla, Raj Kumar, 110018, Dehli (IN)
(74) Representative: Cronin, Brian Harold John

(57) **Abstract**

The present invention relates to monosaccharide derivatives as anti-inflammatory agents. The compounds disorder herein can be useful for inhibition and prevention of inflammation and associated pathologies including inflammatory and autoimmune diseases such as bronchial asthma, rheumatoid arthritis, type I diabetes, multiple sclerosis, allograft rejection, psoriasis, inflammatory bowel disease, Ulcerative colitis, acne, atherosclerosis, cancer, pruritis and allergic rhinitis. Pharmacological compositions containing compounds disclosed herein and the methods of treating bronchial asthma, chronic obstructive pulmonary disease, rheumatoid arthritis, multiple sclerosis, type 1 diabetes, psoriasis, allograft rejection, inflammatory bowel disease, Ulcerative colitis, acne, atherosclerosis, cancer, pruritis, allergic rhinitis and other inflammatory and/or autoimmune disorders, using the compounds are also provided.

## Description

### Field of the Invention

The present invention relates to monosaccharide derivatives as anti-inflammatory agents. The compounds disclosed herein can be useful for inhibition and prevention of inflammation and associated pathologies including inflammatory and autoimmune diseases such as bronchial asthma, rheumatoid arthritis, type I diabetes, multiple sclerosis, allograft rejection, psoriasis, inflammatory bowel disease, Ulcerative colitis, acne, atherosclerosis, cancer, pruritis or allergic rhinitis. Pharmacological compositions containing compounds disclosed herein and the methods of treating bronchial asthma, chronic obstructive pulmonary disease, rheumatoid arthritis, multiple sclerosis, type I diabetes, psoriasis, allograft rejection, inflammatory bowel disease, Ulcerative colitis, acne, atherosclerosis, cancer, pruritis, allergic rhinitis and other inflammatory and/or autoimmune disorders, using the compounds are also provided.

### Background of the Invention

Inflammation is a key defence mechanism of the body that is activated as a result of tissue injury. The inflammatory process is self-containing, however, under certain pathophysiological conditions the inflammatory process tends to perpetuate itself, giving rise to chronic inflammatory diseases like bronchial asthma, rheumatoid arthritis etc.

Although the exact cellular and molecular bases of most chronic inflammatory disease remain unclear, it has become apparent that several inflammatory cells act in concert towards initiation and perpertuation of an inflammatory response by releasing a wide range of chemokine, cytokine, proteolytic enzymes and other bioactive molecules. Mast cells primed by lymphocytes interact with environmental allergens and release mediators like histamine, prostaglandin, leukotrienes etc *(*Clin. Exp. Allergy 32, 1682, 2002) to initiate an early inflammatory response. This is followed by a delayed inflammatory response due to release of cytokines (IL-4, IL-5, IL-6, IL-8, IL-13, GM-CSF and TNFalpha), chemokines and proteolytic enzymes (chymase, tryptase) (Chest 112, 523, 1997; Lancet 350, 59, 1997) that not only bring about tissue damage, but attract other inflammatory cells and initiate tissue fibrosis, and the cycle continues. Eosinophils infiltrate inflamed tissue following allergen - mast cell interaction in bronchial asthma and allergic rhinitis. Evidence is emerging that mast cells also interact with bacterial endotoxins leading to generation of cytokines like TNFalpha, that encourage neutrophil influx into the site of inflammation *(*Br. J. Pharmacol.123, 31, 1998; Br. J. Pharmacol. 128, 700, 1999; Br. J. Pharmacol. 136, 111, 2002; J. Clin. Invest. 109, 1351, 2002). Involvement of mast cells in the inflammatory response of chronic obstructive pulmonary disease *(*New Eng. J. Med. 347, 1040, 2002; Thorax 57, 649, 2002), inflammatory bowel disease *(*Gut. 45 Suppl. 116, 1999) as well as in rheumatoid arthritis *(*Science 297, 1626, 2002), pathologies with prominent neutrophilic inflammation, has been proposed.

U.S. Patent 6,329,344B1 discloses several monosaccharide derivatives said to be useful as cell adhesion inhibitors. It generally relates to substituted pentose and hexose monosaccharide derivatives, which are said to exhibit cell adhesion inhibitory and anti-inflammatory activities. U.S. Patent 6,590,085B1 discloses several monosaccharide derivatives described as inhibitors of cell adhesion and cell adhesion mediated pathologies, including inflammatory and autoimmune diseases. U.S. Patent Application US 2002/0173632 A1 discloses furanose and amino furanose compounds reportedly useful for rheumatoid, arthritis, immunomodulatory diseases inflammatory and proliferative diseases. U.S. Patent 5,298,494 discloses derivatives of monosaccharides, which are said to exhibit anti-proliferative and/or anti-inflammatory activity and are useful for treating mammals having inflammatory disorders and/or autoimmune disorders. U.S Patent 4,996,195 discloses derivatives of α,D-glucofuranose and α,D-allofuranose described as useful for treating animals and mammals with inflammatory and/or autoimmune disorders.

WO 93/13117 and U.S. Patent 5,360,792 disclose 5- or 6-deoxy hexose monosaccharides having a saturated nitrogen containing heterocycle described as useful as anti-proliferative and anti-inflammatory compounds. WO 94/28910 discloses 5,6-dideoxy-5-amino derivatives of idose and 6-deoxy-6-amino derivatives of glucose, which reportedly exhibit immunomodulatory, anti-inflammatory and anti-proliferative activity. WO 94/11381 discloses derivatives of pentose monosaccharides described as useful as anti-proliferative and anti-inflammatory compounds. U.S. Patent 5,010,058 discloses 3,5,6-disubstituted derivatives of 1,2-O-isopropylidene-α,O-glucofuranoside described as useful for treating inflammatory and autoimmune disorders. U.S. Patent 4,849,512 discloses 3-acylamino-3-deoxyallose derivatives. U.S. Patent 5,367,062 discloses disubstituted and deoxy disubstituted derivatives of α-D-lyxofuranosides reportedly having anti-inflammatory and anti-proliferative activity. U.S. Patent 5,360,794 discloses disubstituted derivatives of α-D-mannofuranoside reportedly having anti-inflammatory and anti proliferative activity. WO 03/029263 discloses 3-deoxy-3-amide derivatives of carbohydrates described as useful as inducers of erythroid cell differentiation. FR 2735130 discloses regiospecific synthesis of new carbamic polyesters.

### Summary of the Invention

Monosaccharide derivatives which can be used for the inhibition and prevention of inflammation and associated pathologies, including inflammatory and autoimmune diseases such as bronchial asthma, rheumatoid arthritis, type I diabetes, multiple sclerosis, allograft rejection or psoriasis are provided herein. Pharmaceutically acceptable salts, pharmaceutically acceptable solvates, enantiomers, diastereomers or N-oxides of these compounds having the same type of activity are also provided. Pharmaceutical compositions containing the compounds, and which may also contain pharmaceutically acceptable carriers or diluents, which may be used for the treatment of inflammatory and autoimmune diseases such as bronchial asthma, rheumatoid arthritis, type I diabetes, multiple sclerosis, allograft rejection, psoriasis, inflammatory bowel disease, Ulcerative colitis, acne, atherosclerosis, cancer, pruritis and allergic rhinitis are provided herein.

Other aspects will be set forth in accompanying description which follows and in part will be apparent from the description or may be learnt by the practice of the invention.

In accordance with one aspect, there are provided compounds having the structure of Formula I. 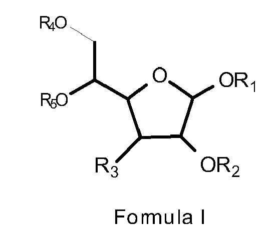 R₁ and R₂ can together form a five-membered acetal, wherein the carbon atom joining the oxygens can be substituted with R_{L} and Rₘ [wherein R_{L} and Rₘ are independently selected from hydrogen, alkyl, alkenyl, alkynyl, cycloalkyl, aryl, or aralkyl; or R_{L} and Rₘ can together join to form a 3-8 membered ring, wherein the ring may optionally contain one or more heteroatoms selected from O, N or S, and the ring may be optionally substituted with one or more of alkyl, alkenyl, alkynyl, amino, substituted amino, cycloalkyl, oxo, hydroxy, carboxy, -COQR₆ (wherein Q is O or NH and R₆ is selected from alkyl, alkenyl, alkynyl, aryl, aralkyl, and cycloalkyl), alkoxy, aryloxy, halogen (F,Cl, Br, I), aryl, aralkyl, heteroaryl, heterocyclyl, heteroarylalkyl, or heterocyclylalkyl; or R_{L} and Rₘ can together join to form an oxo group].
R₃ can be
A) -(CH₂)ₙG wherein n is an integer from 0-5 and G is selected from
   1) ORₑ {wherein Rₑ is selected from
      a) acyl (with the proviso that n cannot be 0), and
      b) -C(=O)NR_{f}R_{q} [wherein R_{f} and Rq can be independently selected from hydrogen, hydroxy (with the restriction that both R_{f} and Rq cannot both be hydroxy), alkyl, alkenyl, alkynyl, cycloalkyl, aryl, aralkyl, heteroaryl, heterocyclyl, and S(O)₂R₇ (wherein R₇ is selected from alkyl, alkenyl, alkynyl, cycloalkyl, aryl, aralkyl, heteroaryl, heterocyclyl, heterocyclylalkyl, heteroarylalkyl, and optionally substituted amino)]; and R_{f} and R_{q} may also together join to form a heterocyclyl ring; also, when n is zero, then R_{f} and Rq cannot be hydrogen, alkyl, alkenyl, alkynyl, cycloalkyl and R_{f} and Rq together cannot join to form a heterocyclyl ring};
   2) -NRⱼC(=O)ORₛ, (wherein Rⱼ is selected from hydrogen, lower (C₁-C₆) alkyl, lower (C₂-C₆) alkenyl, lower (C₂-C₆) alkynyl, lower (C₃-C₆) cycloalkyl, aryl, heteroaryl (with the proviso that the heteroaryl ring is not linked through a heteroatom), aralkyl (C₁-C₄), heteroarylalkyl (C₁-C₄), and heterocyclylalkyl (C₁-C₄), and Rₛ is selected from alkyl, alkenyl, alkynyl, cycloalkyl, aryl, aralkyl, heterocyclylalkyl, or heteroarylalkyl);
   3) NRⱼYRᵤ (wherein Rj is the same as defined above and Y is -C(=O), -C(=S) or SO₂ and Rᵤ is selected from alkyl, alkenyl, alkynyl, cycloalkyl, aryl, aralkyl, heteroaryl, heterocyclyl, heteroarylalkyl, and heterocyclylalkyl; and when n is 0 then Y cannot be - C(=O));
   4) -NRⱼC(=T)NRₜRₓ (wherein Rₜ is OH or Rₓ and T is O, S, -N(CN), -N(NO₂), CH(NO₂) Rⱼ is the same as defined above and Rₓ is selected from hydrogen, alkyl, alkenyl, alkynyl, cycloalkyl, aryl, aralkyl, heteroaryl, heterocyclyl, heteroarylalkyl, heterocyclylalkyl, and S(O)₂R₇ wherein R₇ is the same as defined above);
   5) heterocyclyl;
   6) heteroaryl; and
   7) -(C=O)NRₐR_{b} (wherein Rₐ and R_{b} are independently selected from hydrogen, and Rᵤ wherein Rᵤ is same as defined earlier, also, Rₐ and R_{b} together with the nitrogen atom carrying them can be the N-terminus of an amino acid or di-tetrapeptide or Rₐ and R_{b} may together join to form a heterocyclyl ring).
   R₃ can also be
B) -NRⱼRₘ (wherein Rⱼ is the same as defined above and Rₘ is selected from alkyl, cycloalkyl, aryl, aralkyl, heteroaryl, heterocyclyl, heteroarylalkyl, and heterocyclylalkyl);
C) -O(CH₂)_{w}G₁ [wherein w is an integer from 1-5 (and G₁ is selected from ORₑ (wherein Re is the same as defined above), -NRⱼC(=O)ORₛ (wherein Rⱼ and Rₛ are the same as defined above), -NRⱼC(=T)NRₜRₓ (wherein Rⱼ, T, Rₜ and Rₓ are the same as defined above), -NRⱼYRᵤ (wherein Y, Rᵤ and Rⱼ are the same as defined above), heterocyclyl, and heteroaryl)];
D) -NRⱼ(CH₂)_{w}G₁ (wherein w, Rⱼ and G₁ are the same as defined above);
E) -O(CH₂)_{w}G₂ [wherein w is the same as defined above (and G₂ is selected from -C(=O)NRₐR_{b} (wherein Rₐ and R_{b} are the same as defined above), and -C(=O)ORₖ (wherein Rₖ is H or R₆ and R₆ is the same as defined above); or
F) -NRⱼ(CH₂)_{w}G₂ (wherein w is as defined above, Rⱼ and G₂ are the same as defined above))].

Also, when R₃ is ORₑ then R₂ and Rₑ may together join to form a five membered acetal wherein the carbon linking the two oxygens is substituted with R_{L} and Rₘ (wherein R_{L} and Rₘ are the same as defined earlier) (and R₁ is independently selected from
a) -(CH₂)ₜG₁ (wherein t is an integer from 2-4 and G₁ are the same as defined above and also when G₁ is heterocyclylalkyl group then the said group cannot be 4-(1-pyrrolidinyl) butyl),
b) -(CH₂)_{w}G₂ (wherein w and G₂ are the same as defined above),
c) aryl,
d) aralkyl (with the proviso that aralkyl cannot be phenylpropyl),
e) heteroaryl, and
f) heterocyclyl (wherein the heteroaryl and heterocyclyl rings are not linked through a heteroatom), and cycloalkyl (with the proviso that cycloalkyl cannot be cyclooctyl).
R₄ and R₅ can independently be selected from hydrogen, lower (C₁-C₆) alkyl, lower (C₂-C₆) alkenyl, lower (C₂-C₆) alkynyl, lower (C₃-C₈) cycloalkyl, aryl, acyl, heterocyclyl, heteroaryl, lower (C₁-C₄) heterocyclylalkyl, and lower (C₁-C₄) heteroarylalkyl; or R₄ and R₅ may together form a five-membered acetal wherein the carbon linking the two oxygens is substituted with R_{L} and Rₘ (wherein R_{L} and Rₘ are the same as defined earlier) with the proviso that when R₃ is ORₑ then the acetal must be isopropylidene acetal.

The following definitions apply to terms as used herein.

The term "alkyl," unless otherwise specified, refers to a monoradical branched or unbranched saturated hydrocarbon chain having from 1 to 20 carbon atoms. Alkyl groups can be optionally interrupted by atom(s) or group(s) independently selected from oxygen, sulfur, a phenylene, sulphinyl, sulphonyl group or -NR_{α}-, wherein R_{α} can be hydrogen, alkyl, cycloalkyl, alkenyl, cycloalkenyl, alkynyl, aryl, acyl, aralkyl, -C(=O)OR_{λ}, SOₘR_{ψ} or -C(=O)NR_{λ}R_{π}. This term can be exemplified by groups such as methyl, ethyl, n-propyl, iso-propyl, n-butyl, iso-butyl, sec-butyl, t-butyl, n-pentyl, isopentyl, neopentyl, n-hexyl, n-decyl, tetradecyl, and the like. Alkyl groups may be substituted further with one or more substituents selected from alkenyl, alkynyl, alkoxy, cycloalkyl, cycloalkenyl, acyl, acylamino, acyloxy, alkoxycarbonylamino, azido, cyano, halogen, hydroxy, keto, oxo, thiocarbonyl, carboxy, carboxyalkyl, aryl, heterocyclyl, heteroaryl, (heterocyclyl)alkyl, cycloalkoxy, -CH=N-O(C₁₋₆alkyl), -CH=N-NH(C₁₋₆alkyl), -CH=N-NH(C₁₋₆alkyl)-C₁₋₆-alkyl, arylthio, thiol, alkylthio, aryloxy, nitro, aminosulfonyl, aminocarbonylamino, - NHC(=O)R_{λ}, -NR_{λ}R_{π}, -C(=O)NR_{λ}R_{π}, -NHC(=O)NR_{λ}R_{π}, -C(=O)heteroaryl, C(=O)heterocyclyl, -O-C(=O)NR_{λ}R_{π} {wherein R_{λ} and R_{π} are independently selected from hydrogen, halogen, hydroxy, alkyl, alkenyl, alkynyl, alkenyl, alkoxy, cycloalkyl, cycloalkenyl, aryl, aralkyl, heterocyclyl, heteroaryl, heterocyclylalkyl, heteroarylalkyl or carboxy}, nitro or -SOₘR_{ψ} (wherein m is an integer from 0-2 and R_{ψ} is hydrogen, alkyl, alkenyl, alkynyl, cycloalkyl, aralkyl, aryl, heterocyclyl, heteroaryl, heteroarylalkyl or heterocyclylalkyl). Unless otherwise constrained by the definition, alkyl substituents may be further substituted by 1-3 substituents selected from alkyl, alkenyl, alkynyl, carboxy, - NR_{λ}R_{π}, -C(=O)NR_{λ}R_{π}, -OC(=O)NR_{λ}R_{π},-NHC(=O)NR_{λ}R_{π}, hydroxy, alkoxy, halogen, CF₃, cyano, and -SOₘR_{ψ}; or an alkyl group also may be interrupted by 1-5 atoms of groups independently selected from oxygen, sulfur or -NR_{α}- (wherein R_{α}, R_{λ}, R_{π}, m and R_{ψ}, are the same as defined earlier). Unless otherwise constrained by the definition, all substituents may be substituted further by 1-3 substituents selected from alkyl, alkenyl, alkynyl, carboxy, carboxyalkyl, -NR_{λ}R_{π}, -C(=O)NR_{λ}R_{π}, -O-C(=O)NR_{λ}R_{π}, hydroxy, alkoxy, halogen, CF₃, cyano, and -SOₘR_{ψ} (wherein R_{λ}, R_{π}, m and R_{ψ} are the same as defined earlier); or an alkyl group as defined above that has both substituents as defined above and is also interrupted by 1-5 atoms or groups as defined above.

The term "alkylene," as used herein, refers to a diradical branched or unbranched saturated hydrocarbon chain having from 1 to 6 carbon atoms and one or more hydrogen can optionally be substituted with alkyl, hydroxy, halogen or oximes. This term can be exemplified by groups such as methylene, ethylene, propylene isomers (e.g., -CH₂CH₂CH₂ and -CH(CH₃)CH₂) and the like. Alkylene may further be substituted with one or more substituents such as alkyl, alkenyl, alkynyl, alkoxy, cycloalkyl, acyl, acylamino, acyloxy, alkoxycarbonylamino, azido, cyano, halogen, hydroxy, oxo, thiocarbonyl, carboxy, arylthio, thiol, alkylthio, aryloxy, heteroaryloxy, aminosulfonyl, -COOR_{ψ}, -NHC(=O)R_{λ}, -NR_{λ}R_{π}, -C(=O)NR_{λ}R_{π}, -NHC(=O)NR_{λ}R_{π}, -C(=O)heteroaryl, C(=O)heterocyclyl, -O-C(=O)NR_{λ}R_{π}, nitro, -S(O)ₘR_{λ} (wherein R_{λ}, R_{π}, m and R_{ψ} are the same as defined earlier). Unless otherwise constrained by the definition, all substituents may be further substituted by 1-3 substituents chosen from alkyl, alkenyl, alkynyl, carboxy, -COOR_{ψ}, -NR_{λ}R_{π}, -C(=O)NR_{λ}R_{π}, -OC(=O)NR_{λ}R_{π}, -NHC(=O)NR_{λ}R_{π}, hydroxy, alkoxy, halogen, CF₃, cyano, and -S(O)ₘR_{ψ} (wherein R_{λ}, R_{π}, m and R_{ψ} are the same as defined earlier). Alkylene can also be optionally interrupted by 1-5 atoms of groups independently chosen from oxygen, sulfur and -NR_{α} (wherein R_{α} is the same as defined earlier). Unless otherwise constrained by the definition, all substituents may be further substituted by 1-3 substituents selected from hydrogen, alkyl, cycloalkyl, alkenyl, alkynyl, aryl, acyl, aralkyl, alkoxy, hydroxy, carboxy, -C(=O)OR_{ψ}, halogen, CF₃, cyano, -NR_{λ}R_{π}, -S(O)ₘR_{ψ}, -C(=O)NR_{λ}R_{π}, -OC(=O)NR_{λ}R_{π}, -CONH-, -C=O or -C=NOH (wherein R_{λ}, R_{π}, m and R_{ψ} are the same as defined earlier).

The term "alkenyl," unless otherwise specified, refers to a monoradical of a branched or unbranched unsaturated hydrocarbon group having from 2 to 20 carbon atoms with cis, trans or geminal geometry. Alkenyl groups can be optionally interrupted by atom(s) or group(s) independently chosen from oxygen, sulfur, phenylene, sulphinyl, sulphonyl and -NR_{α}- (wherein R_{α} is the same as defined earlier). In the event that alkenyl is attached to a heteroatom, the double bond cannot be alpha to the heteroatom. Alkenyl groups may be substituted further with one or more substituents selected from alkyl, alkenyl, alkynyl, alkoxy, cycloalkyl, cycloalkenyl, acyl, acylamino, acyloxy, -NHC(=O)R_{λ}, -NR_{λ}R_{π}, -C(=O)NR_{λ}R_{π}, -NHC(=O)NR_{λ}R_{π}, -O-C(=O)NR_{λ}R_{π}, alkoxycarbonylamino, azido, cyano, halogen, hydroxy, oxo, keto, carboxyalkyl, thiocarbonyl, carboxy, arylthio, thiol, alkylthio, aryl, aralkyl, aryloxy, heterocyclyl, heteroaryl, heterocyclyl alkyl, heteroaryl alkyl, aminosulfonyl, aminocarbonylamino, alkoxyamino, hydroxyamino, alkoxyamino, nitro or SOₘR_{ψ} (wherein R_{λ}, R_{π}, m and R_{ψ} are as defined earlier). Unless otherwise constrained by the definition, alkenyl substituents optionally may be substituted further by 1-3 substituents selected from alkyl, alkenyl, alkynyl, carboxy, hydroxy, alkoxy, halogen, -CF₃, cyano, -NR_{λ}R_{π}, -C(=O)NR_{λ}R_{π}, -O-C(=O)NR_{λ}R_{π} and -SOₘR_{ψ} (wherein R_{λ}, R_{π}, m and R_{ψ} are as defined earlier). Groups, such as ethenyl or vinyl (CH=CH₂), 1-propylene or allyl (-CH₂CH=CH₂), iso-propylene (-C(CH₃)=CH₂), bicyclo[2.2.1]heptene, and the like, exemplify this term.

The term "alkenylene" unless otherwise specified, refers to a diradical of a branched or unbranched unsaturated hydrocarbon group preferably having from 2 to 6 carbon atoms with cis, trans or geminal geometry. In the event that alkenylene is attached to the heteroatom, the double bond cannot be alpha to the heteroatom. The alkenylene group can be connected by two bonds to the rest of the structure of compound of Formula I. Alkenylene may further be substituted with one or more substituents such as alkyl, alkenyl, alkynyl, alkoxy, cycloalkyl, acyl, acylamino, acyloxy, -NHC(=O)R_{λ}, -NR_{λ}R_{π}, -C(=O)NR_{λ}R_{π}, -NHC(=O)NR_{λ}R_{π},-OC(=O)NR_{λ}R_{π} (wherein R_{λ} and R_{π} are the same as defined earlier), alkoxycarbonylamino, azido, cyano, halogen, hydroxy, oxo, thiocarbonyl, carboxy, -COOR_{ψ} (wherein R_{ψ} is the same as defined earlier), arylthio, thiol, alkylthio, aryl, aralkyl, aryloxy, heterocyclyl, heteroaryl, heterocyclyl alkyl, heteroaryl alkyl, aminosulfonyl, alkoxyamino, nitro, -S(O)ₘR_{ψ} (wherein R_{ψ} and m are the same as defined earlier). Unless otherwise constrained by the definition, all substituents may optionally be further substituted by 1-3 substituents chosen from alkyl, alkenyl, alkynyl, carboxy, -COOR_{ψ} (wherein R_{ψ} is the same as defined earlier), hydroxy, alkoxy, halogen, -CF₃, cyano, -NR_{λ}R_{π}, -C(=O)NR_{λ}R_{π}, -OC(=O)NR_{λ}R_{π} (wherein R_{λ} and R_{π} are the same as defined earlier) and -S(O)ₘR_{ψ} (wherein R_{ψ} and m are the same as defined earlier).

The term "alkynyl," unless otherwise specified, refers to a monoradical of an unsaturated hydrocarbon, having from 2 to 20 carbon atoms. Alkynyl groups can be optionally interrupted by atom(s) or group(s) independently chosen from oxygen, sulfur, phenylene, sulphinyl, sulphonyl and -NR_{α}- (wherein R_{α} is the same as defined earlier). In the event that alkynyl groups are attached to a heteroatom, the triple bond cannot be alpha to the heteroatom. Alkynyl groups may be substituted further with one or more substituents selected from alkyl, alkenyl, alkoxy, cycloalkyl, cycloalkenyl, acyl, acylamino, acyloxy, alkoxycarbonylamino, azido, cyano, halogen, hydroxy, keto, oxo, thiocarbonyl, carboxy, carboxyalkyl, arylthio, thiol, alkylthio, aryl, aralkyl, aryloxy, aminosulfonyl, aminocarbonylamino, hydroxyamino, alkoxyamino, nitro, heterocyclyl, heteroaryl, heterocyclylalkyl, heteroarylalkyl, -NHC(=O)R_{λ}, -NR_{λ}R_{π}, -NHC(=O)NR_{λ}R_{π}, -C(=O)NR_{λ}R_{π}, -O-C(=O)NR_{λ}R_{π} or -SOₘR_{ψ}, (wherein R_{λ}, R_{π}, m and R_{ψ}, are the same as defined earlier). Unless otherwise constrained by the definition, alkynyl substituents optionally may be substituted further by 1-3 substituents selected from alkyl, alkenyl, alkynyl, carboxy, carboxyalkyl, hydroxy, alkoxy, halogen, CF₃, -NR_{λ}R_{π}, -C(=O)NR_{λ}R_{π}, -NHC(=O)NR_{λ}R_{π}, -C(=O)NR_{λ}R_{π}, cyano or -SOₘR_{ψ}, (wherein R_{λ}, R_{π}, m and R_{ψ} are the same as defined earlier).

The term "alkynylene" unless otherwise specified, refers to a diradical of a triply-unsaturated hydrocarbon, preferably having from 2 to 6 carbon atoms. In the event that alkynylene is attached to the heteroatom, the triple bond cannot be alpha to the heteroatom. The alkenylene group can be connected by two bonds to the rest of the structure of compound of Formula I. Alkynylene may further be substituted with one or more substituents such as alkyl, alkenyl, alkoxy, cycloalkyl, acyl, acylamino, acyloxy, alkoxycarbonylamino, azido, cyano, halogen, hydroxy, oxo, thiocarbonyl, carboxy, arylthio, thiol, alkylthio, aryl, aralkyl, aryloxy, aminosulfonyl, nitro, heterocyclyl, heteroaryl, heterocyclyl alkyl, heteroarylalkyl, -NHC(=O)R_{λ}, -NR_{λ}R_{π}, -NHC(=O)NR_{λ}R_{π}, -C(=O)NR_{λ}R_{π}, -OC(=O)NR_{λ}R_{π} (wherein R_{λ} and R_{π} are the same as defined earlier), -S(O)ₘR_{ψ} (wherein R_{ψ} and m are the same as defined earlier). Unless otherwise constrained by the definition, all substituents may optionally be further substituted by 1-3 substituents chosen from alkyl, alkenyl, alkynyl, carboxy, -COOR_{ψ} (wherein R_{ψ} is the same as defined earlier), hydroxy, alkoxy, halogen, CF₃, -NR_{λ}R_{π}, -C(=O)NR_{λ}R_{π}, -NHC(=O)NR_{λ}R_{π},-C(=O)NR_{λ}R_{π} (wherein R_{λ} and R_{π} are the same as defined earlier), cyano, and -S(O)ₘR_{ψ} (wherein R_{ψ} and m are the same as defined earlier).

The term "cycloalkyl," unless otherwise specified, refers to cyclic alkyl groups of from 3 to 20 carbon atoms having a single cyclic ring or multiple condensed rings, which may optionally contain one or more olefinic bonds, unless otherwise constrained by the definition. Such cycloalkyl groups can include, for example, single ring structures, including cyclopropyl, cyclobutyl, cyclooctyl, cyclopentenyl, and the like or multiple ring structures, including adamantanyl, and bicyclo [2.2.1] heptane or cyclic alkyl groups to which is fused an aryl group, for example, indane, and the like. Spiro and fused ring structures can also be included. Cycloalkyl groups may be substituted further with one or more substituents selected from alkyl, alkenyl, alkynyl, alkoxy, cycloalkyl, cycloalkenyl, acyl, acylamino, acyloxy, alkoxycarbonylamino, azido, cyano, halogen, hydroxy, oxo, thiocarbonyl, carboxy, carboxyalkyl, arylthio, thiol, alkylthio, aryl, aralkyl, aryloxy, aminosulfonyl, aminocarbonylamino, -NR_{λ}R_{π}, -NHC(=O)NR_{λ}R_{π}, -NHC(=O)R_{λ},-C(=O)NR_{λ}R_{π}, -O-C(=O)NR_{λ}R_{π}, nitro, heterocyclyl, heteroaryl, heterocyclylalkyl, heteroarylalkyl or SOₘR_{ψ} (wherein R_{λ}, R_{π}, m and R_{ψ} are the same as defined earlier). Unless otherwise constrained by the definition, cycloalkyl substituents optionally may be substituted further by 1-3 substituents selected from alkyl, alkenyl, alkynyl, carboxy, hydroxy, alkoxy, halogen, CF₃, -NR_{λ}R_{π}, -C(=O)NR_{λ}R_{π}, -NHC(=O)NR_{λ}R_{π},-OC(=O)NR_{λ}R_{π}, cyano or -SOₘR_{ψ} (wherein R_{λ}, R_{π}, m and R_{ψ} are the same as defined earlier). "Cycloalkylalkyl" refers to alkyl-cycloalkyl group linked through alkyl portion, wherein the alkyl and cycloalkyl are the same as defined earlier.

The term "alkoxy" denotes the group O-alkyl wherein alkyl is the same as defined above.

The term "aryl," unless otherwise specified, refers to aromatic system having 6 to 14 carbon atoms, wherein the ring system can be mono-, bi- or tricyclic and are carbocyclic aromatic groups. For example, aryl groups include, but are not limited to, phenyl, biphenyl, anthryl or naphthyl ring and the like, optionally substituted with 1 to 3 substituents selected from halogen (e.g., F, Cl, Br, I), hydroxy, alkyl, alkenyl, alkynyl, cycloalkyl, alkoxy, acyl, aryloxy, CF₃, cyano, nitro, COOR_{ψ}, NHC(=O)R_{λ}, -NR_{λ}R_{π}, -C(=O)NR_{λ}R_{π}, -NHC(=O)NR_{λ}R_{π}, -O-C(=O)NR_{λ}R_{π}, -SOₘR_{ψ}, carboxy, heterocyclyl, heteroaryl, heterocyclylalkyl, heteroarylalkyl or amino carbonyl amino, mercapto, haloalkyl, optionally substituted aryl, optionally substituted heterocyclylalkyl, thioalkyl, -CONHR_{π}, -OCOR_{π}, -COR_{π}, -NHSO₂R_{π} or -SO₂NHR_{π} (wherein R_{λ}, R_{π}, m and R_{ψ}, are the same as defined earlier). Aryl groups optionally may be fused with a cycloalkyl group, wherein the cycloalkyl group may optionally contain heteroatoms selected from O, N or S. Groups such as phenyl, naphthyl, anthryl, biphenyl, and the like exemplify this term.

The term "aralkyl," unless otherwise specified, refers to alkyl-aryl linked through an alkyl portion (wherein alkyl is as defined above) and the alkyl portion contains 1-6 carbon atoms and aryl is as defined below. Examples of aralkyl groups include benzyl, ethylphenyl, propylphenyl, naphthylmethyl and the like.

The term "aryloxy" denotes the group O-aryl wherein aryl is the same as defined above.

The term "carboxy" as defined herein refers to -C(=O)OH.

The term "heteroaryl," unless otherwise specified, refers to an aromatic ring structure containing 5 or 6 ring atoms or a bicyclic or tricyclic aromatic group having from 8 to 10 ring atoms, with one or more heteroatom(s) independently selected from N, O or S optionally substituted with 1 to 4 substituent(s) selected from halogen (e.g., F, Cl, Br, I), hydroxy, alkyl, alkenyl, alkynyl, cycloalkyl, acyl, carboxy, aryl, alkoxy, aralkyl, cyano, nitro, heterocyclyl, heteroaryl, -NR_{λ}R_{π}, CH=NOH, -(CH₂)_{w}C(=O)R_{η} {wherein w is an integer from 0-4 and R_{η} is hydrogen, hydroxy, OR_{λ}, NR_{λ}R_{π}, -NHOR_{ω} or -NHOH}, -C(=O)NR_{λ}R_{π} -NHC(=O)NR_{λ}R_{π}, -SOₘR_{ψ}, -O-C(=O)NR_{λ}R_{π}, -O-C(=O)R_{λ}, or -O-C(=O)OR_{λ} (wherein m, R_{ψ}, R_{λ} and R_{π} are as defined earlier and R_{ω} is alkyl, cycloalkyl, aryl, heteroaryl, heterocyclyl, heteroarylalkyl or heterocyclylalkyl). Unless otherwise constrained by the definition, the substituents are attached to a ring atom, i. e., carbon or heteroatom in the ring. Examples of heteroaryl groups include oxazolyl, imidazolyl, pyrrolyl, 1,2,3-triazolyl, 1,2,4-triazolyl, tetrazolyl, thiazolyl, oxadiazolyl, benzoimidazolyl, thiadiazolyl, pyridinyl, pyridazinyl, pyrimidinyl, pyrazinyl, thienyl, isoxazolyl, triazinyl, furanyl, benzofuranyl, indolyl, benzthiazinyl, benzthiazinonyl, benzoxazinyl, benzoxazinonyl, quinazonyl, carbazolyl phenothiazinyl, phenoxazinyl, benzothiazolyl or benzoxazolyl, and the like.

The term "heterocyclyl," unless otherwise specified, refers to a non-aromatic monocyclic or bicyclic cycloalkyl group having 5 to 10 atoms wherein 1 to 4 carbon atoms in a ring are replaced by heteroatoms selected from O, S or N, and optionally are benzofused or fused heteroaryl having 5-6 ring members and/or optionally are substituted, wherein the substituents are selected from halogen (*e.g.,* F, Cl, Br, I), hydroxy, alkyl, alkenyl, alkynyl, cycloalkyl, acyl, optionally substituted aryl, alkoxy, alkaryl, cyano, nitro, oxo, carboxy, optionally substituted heterocyclyl, optionally substituted heterocyclylalkyl, optionally substituted heteroaryl, -O-C(=O)R_{λ}, -O-C(=O)OR_{λ}, -C(=O)NR_{λ}R_{π}, SOₘR_{ψ}, -O-C(=O)NR_{λ}R_{π}, -NHC(=O)NR_{λ}R_{π}, -NR_{λ}R_{π}, mercapto, haloalkyl, thioalkyl, -COOR_{ψ}, -COONHR_{λ}, -COR_{λ}, -NHSO₂R_{λ} or SO₂NHR_{λ} (wherein m, R_{ψ}, R_{λ} and R_{π} are as defined earlier) or guanidine. Heterocyclyl can optionally include rings having one or more double bonds. Such ring systems can be mono-, bi- or tricyclic. Carbonyl or sulfonyl group can replace carbon atom(s) of heterocyclyl. Unless otherwise constrained by the definition, the substituents are attached to the ring atom, i.e., carbon or heteroatom in the ring. Also, unless otherwise constrained by the definition, the heterocyclyl ring optionally may contain one or more olefinic bond(s). Examples of heterocyclyl groups include oxazolidinyl, tetrahydrofuranyl, dihydrofuranyl, benzoxazinyl, benzthiazinyl, imidazolyl, benzimidazolyl, tetrazolyl, carbaxolyl, indolyl, phenoxazinyl, phenothiazinyl, dihydropyridinyl, dihydroisoxazolyl, dihydrobenzofuryl, azabicyclohexyl, thiazolidinyl, dihydroindolyl, pyridinyl, isoindole 1,3-dione, piperidinyl, tetrahydropyranyl, piperazinyl, 3H-imidazo[4,5-b]pyridine, isoquinolinyl, 1H-pyrrolo[2,3-b]pyridine or piperazinyl and the like.

"Heteroarylalkyl" refers to alkyl-heteroaryl group linked through alkyl portion, wherein the alkyl and heteroaryl are the same as defined earlier.

"Heterocyclylalkyl" refers to alkyl-heterocyclyl group linked through alkyl portion, wherein the alkyl and heterocyclyl are the same as defined earlier.

"Acyl" refers to -C(=O)R" wherein R" is selected from the group alkyl, cycloalkyl, aryl, aralkyl, heteroaryl, heterocyclyl, heteroarylalkyl or heterocyclylalkyl.

"Amine," unless otherwise specified, refers to -NH₂. "Substituted amino" unless otherwise specified, refers to a group -N(Rₖ)₂ wherein each Rₖ is independently selected from the group hydrogen provided that both Rₖ groups are not hydrogen (defined as "amino"), alkyl, alkenyl, alkynyl, aralkyl, cycloalkyl, aryl, heteroaryl, heterocyclyl, heterocyclylalkyl, heteroarylalkyl, acyl, S(O)ₘR_{ψ} (wherein m and R_{ψ} are the same as defined above), -C(= Rᵥ)NR_{λ}R_{y} (wherein Rᵥ is O or S & R_{λ} and R_{y} are the same as defined earlier) or NHC(=Rᵥ)NR_{y}R_{λ} (wherein Rᵥ, R_{y} and R_{λ} are the same as defined earlier). Unless otherwise constrained by the definition, all amino substituents may optionally be further substituted by 1-3 substituents chosen from alkyl, aralkyl, cycloalkyl, aryl, heteroaryl, heterocyclyl, carboxy, -COOR_{ψ} (wherein R_{ψ} is the same as defined earlier), hydroxy, alkoxy, halogen, CF₃, cyano, -C(=Rᵥ)NR_{λ}R_{y} (wherein Rᵥ is the same as defined earlier), -O(C=O)NR_{λ}R_{y}, -OC(=Rᵥ)NR_{λ},R_{y} (wherein R_{λ}, R_{y} and Rᵥ are the same as defined earlier), -S(O)ₘR_{ψ} (wherein R_{ψ} and m are the same as defined above).

The term "leaving group" refers to groups that exhibit or potentially exhibit the properties of being labile under the synthetic conditions and also, of being readily separated from synthetic products under defined conditions. Examples of leaving groups include, but are not limited to, halogen (e.g., F, Cl, Br, I), triflates, tosylate, mesylates, alkoxy, thioalkoxy, or hydroxy radicals and the like.

The term "activated derivative of a carboxylic acid," can include, for example, protected amino acids, aliphatic acids or aromatic acids converted to their corresponding acyl halides (e.g., acid fluoride, acid chloride and acid bromide), corresponding activated esters (e.g., nitro phenyl ester, the ester of 1-hydroxybenzotriazole or the ester of hydroxysuccinimide, HOSu) or mixed anhydrides, for example, anhydride with ethyl chloroformate and other derivatives within the skill of the art.

The term "protecting groups" is used herein to refer to moieties which have the property of preventing specific chemical reaction at a site on the molecule undergoing chemical modification intended to be left unaffected by the particular chemical modification. Also the term protecting group, unless otherwise specified, may be used with groups such as hydroxy, amino and carboxy. Examples of such groups are found in T.W. Greene and P.G.M. Wuts, "Protective Groups in Organic Synthesis", 2nd Ed., John Wiley and Sons, New York, N.Y. The species of the carboxylic protecting groups, amino protecting groups or hydroxy protecting group employed are not critical, so long as the derivatised moieties/moiety is/are stable to conditions of subsequent reactions and can be removed without disrupting the remainder of the molecule.

"Amino acid" refers to both natural and unnatural amino acids. The term "natural amino acid," as used hereind, is intended to represent the twenty two naturally-occurring amino acids glycine, alanine, valine, leucine, isoleucine, serine, methionine, threonine, phenylalanine, tyrosine, trytophan, cysteine, proline, proline, histidine, aspartic acid, asparagines, glutamic acid, glutamine, γ-carboxyglutamic acid, arginine, ornithine and lysine in their L form. The term "unnatural amino acid," as used herein, is intended to represent the 'D' form of the twenty two naturally-occurring amino acids described above. It is further understood that the term unnatural amino acid includes homologues of the natural amino acids, and synthetically modified form of the natural amino acids commonly utilized by those in the peptide chemistry arts when preparing synthetic analogues of naturally occurring peptides, including D and L forms. The synthetically modified forms include amino acids having alkylene chains shortened or lengthened by up to two carbon atoms, amino acids comprising optionally substituted aryl groups, and amino acids comprised halogenated groups preferably halogenated alkyl and aryl groups. The term "unnatural amino acids" as used herein is also intended to represent beta amino acids.

The term "peptide" refers to a molecule comprising amino acids linked through amide linkages. Dipeptide comprises of 2 amino acids, tripeptide refers to a peptide having 3 amino acids and tetrapeptide refers to one having four amino acids, wherein the term amino acid is as defined earlier. "LDVP" refers to a tetrapeptide leucyl-aspartyl-valyl-prolyl. "DVP" refers to a tripeptide aspartyl-valyl-prolyl. "VP" refers to a dipeptide valyl-prolyl.

Compounds disclosed herein contain one or more asymmetric carbon atoms and thus can exist as racemates and racemic mixtures, single enantiomers, diastereomieric mixtures and individual diastereomers. All such isomeric forms of these compounds are expressly included herein. Each stereogenic carbon may be of the R or S configuration. Although the specific compounds exemplified in this application may be depicted in a particular stereochemical configuration, compounds having either the opposite stereochemistry at any given chiral center or mixtures thereof are envisioned. Although amino acids and amino acid side chains may be depicted in a particular configuration, both natural and unnatural forms are envisioned.

### Detailed Description of the Invention

Compounds disclosed herein may be prepared by techniques well known in the art and familiar to a practitioner of ordinary skill in art. In addition, compounds disclosed herein may be prepared by the processes described herein, although these processes are not the only means by which the compounds described may be synthesised. Further, synthetic steps described herein may be performed in an alternate sequence or order to give the desired compounds.

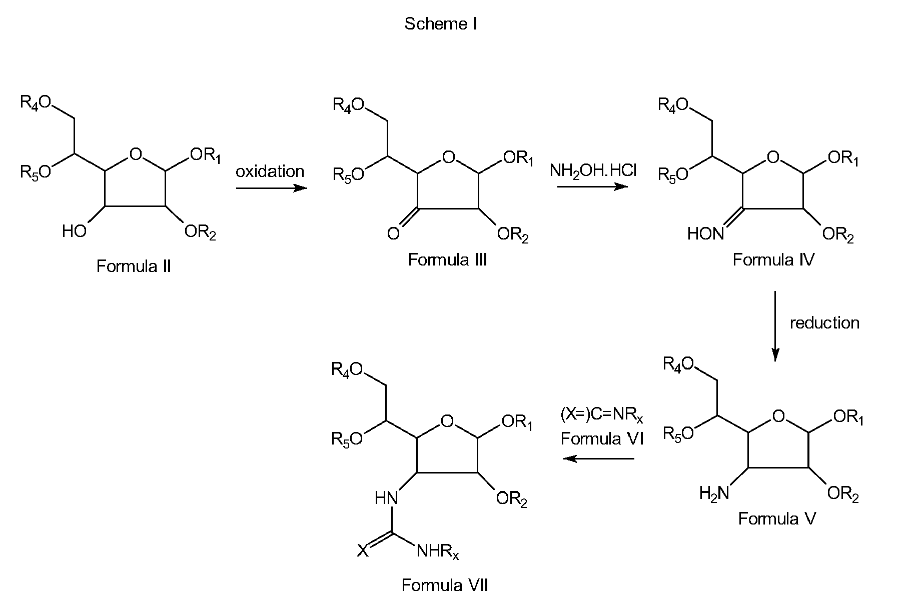

Compounds of Formula VII can be prepared by Scheme I. Thus, a compound of Formula II (wherein R₁, R₂, R₄ and R₅ are the same as defined earlier) can be oxidized to form a compound of Formula III, which can be reacted with , for example, hydroxylamine hydrochloride to form a compound of Formula IV, which can undergo reduction to form a compound of Formula V, which can be reacted with a compound of Formula VI (wherein X is O or S and Rₓ the same as defined earlier) to furnish a compound of Formula VII.

The oxidation of a compound of Formula II to form a compound of Formula III can be carried out under various conditions. For example, one may use Swern's oxidation utilizing dimethyl sulphoxide and acetic anhydride or oxalyl chloride, optionally in either dimethyl sulphoxide or dichloromethane as solvents. One may also utilize oxidizing agents such as pyridinium chlorochromate, pyridinium dichromate, pyridine-sulfurtrioxide or periodinane in an organic solvent such as dichloromethane, chloroform for the oxidation of a compound of Formula II to form a compound of Formula III.

Thus, the oxidation of a compound of Formula II can be carried out utilizing dimethyl sulphoxide and acetic anhydride to furnish a compound of Formula III. The reaction of a compound of Formula III with hydroxylamine hydrochloride to form a compound of Formula IV can be carried out in an organic solvent such as ethanol, methanol, propanol or isopropyl alcohol, in the presence of an organic base such as pyridine, triethylamine or diisopropylethylamine.

The reduction of a compound of Formula IV to yield a compound of Formula V can be carried out in an organic solvent such as tetrahydrofuran, dimethylformamide, diethylether or dioxane, with a reducing agent such as lithium aluminium hydride or sodium borohydride.

The reaction of a compound of Formula V with an isocyanate or isothiocyanate of Formula VI to yield a compound of Formula VII can be carried out in an organic solvent such as acetonitrile, dichloromethane, dichloroethane, chloroform or carbon tetrachloride.

Alternatively, a compound of Formula VII can also be prepared by reacting a compound of Formula V with an appropriate amine in the presence of reagents such as carbonyldiimidazole (CDI) or with carbamates such as phenyl carbamate or p-nitrophenyl carbamate of an amine. Also, optionally thiocarbonyldiimidazole or an isothiocyanate can be used in place of carbonyldiimidazole or isocyanate, respectively in the reaction.

Particular illustrative compounds which may be prepared following Scheme I include, for example:
(3R or 3S)-1,2;5,6-Di-O-isopropylidene-3-deoxy-3-[{(4-chloro-phenyl)-amino}-carbonyl]-amino-α-D-allofuranoside (Compound No. 1);
1,2;5,6-Di-O-isopropylidene-3-deoxy-3-[{(4-methoxy-phenyl)-amino}-carbonyl]-amino-α-D-allofuranoside (Compound No. 3);
1,2;5,6-Di-O-isopropylidene-3-deoxy-3-[{phenyl-sulphonylamino}-carbonyl]-amino-α-D-allofuranoside (Compound No. 5);
1,2;5,6-Di-O-isopropylidene-3-deoxy-3-[{(4-methyl-phenyl)-sulphonylamino}-carbonyl]-amino-α-D-allofuranoside (Compound No. 7);
(3R or 3S)-1,2;5,6-Di-O-isopropylidene-3-deoxy-3-[{(4-nitro-phenyl)-amino}-carbonyl]-amino-α-D-allofuranoside (Compound No. 9);
(3R or 3S)-1,2;5,6-Di-O-isopropylidene-3-deoxy-3-[{4-methylphenyl}-amino}-carbonyl]-amino-α-D-allofuranoside (Compound No. 11);
1,2;5,6-Di-O-isopropylidene-3-deoxy-3-[{(4-trifluoromethyl-phenyl)-amino}-carbonyl]-amino-α-D-allofuranoside (Compound No. 47);
1,2;5,6-Di-O-isopropylidene-3-deoxy-3-{[(2-phenylethyl)-amino]thiocarbonyl}-amino-α-D-allofuranoside (Compound No. 49);
1,2;5,6-Di-O-isopropylidene-3-deoxy-3-{[(4-fluorophenyl)-amino]thiocarbonyl}-amino-α-D-allofuranoside (Compound No. 50),
1,2;5,6-Di-O-isopropylidene-3-deoxy-3-{[(4-fluorophenyl)-amino]thiocarbonyl}-amino-α-D-allofuranoside (Compound No. 55);
1,2;5,6-Di-O-isopropylidene-3-deoxy-3-{[(4-trifluoromethoxyphenyl)-amino]thiocarbonyl}-amino-α-D-allofuranoside (Compound No. 56);
1,2;5,6-Di-O-isopropylidene-3-deoxy-3-{[(4-nitrophenyl)-amino]thiocarbonyl}-amino-α-D-allofuranoside (Compound No. 58);
1,2;5,6- Di-O-isopropylidene-3-deoxy-3- {[(2,4-difluorophenyl)-amino]thiocarbonyl}-amino-α-D-allofuranoside (Compound No. 59);
1,2;5,6-Di-O-isopropylidene-3-deoxy-3-{[(3,4-dichlorophenyl)-amino]thiocarbonyl}-amino-α-D-allofuranoside (Compound No. 60);
1,2;5,6-Di-O-isopropylidene-3-deoxy-3-{[(4-trinuoromethylphenyl)-amino]thiocarbonyl}-amino-α-D-allofuranoside (Compound No. 61);
1,2;5,6-Di-O-isopropylidene-3-deoxy-3- {[(4-methoxyphenyl)-amino]thiocarbonyl}-amino-α-D-allofuranoside (Compound No. 62);
1,2;5,6-Di-O-isopropylidene-3-deoxy-3- {[(4-benzyloxyphenyl)-amino]thiocarbonyl}-amino-α-D-allofuranoside (Compound No. 63);
1,2;5,6-Di-O-isopropylidene-3-deoxy-3-{[(3-nitrophenyl)-amino]thiocarbonyl}-amino-α-D-allofuranoside (Compound No. 64);
1,2;5,6-Di-O-isopropylidene-3-deoxy-3-{[(2-fluorophenyl)-amino]thiocarbonyl}-amino-α-D-allofuranoside (Compound No. 65);
1,2;5,6-Di-O-isopropylidene-3-deoxy-3-{[(2-trifluoromethylphenyl)-amino]thiocarbonyl}-amino-α-D-allofuranoside (Compound No. 66);
1,2;5,6-Di-O-isopropylidene-3-deoxy-3-{[(2-nitrophenyl)-amino]thiocarbonyl}-amino-α-D-allofuranoside (Compound No. 67);
1,2;5,6-Di-O-isopropylidene-3-deoxy-3-{[(4-cyanophenyl)-amino]thiocarbonyl}-amino-α-D-allofuranoside (Compound No. 68);
1,2;5,6-Di-O-isopropylidene-3-deoxy-3-{[(4-difluoromethoxyphenyl)-amino]thiocarbonyl}-amino-α-D-allofuranoside (Compound No. 69);
1,2;5,6-Di-O-isopropylidene-3-deoxy-3-{[(2-difluoromethoxyphenyl)-amino]thiocarbonyl}-amino-α-D-allofuranoside (Compound No. 70);
1,2;5,6-Di-O-isopropylidene-3-deoxy-3- ([(naphthyl)-amino]thiocarbonyl) -amino-α-D-allofuranoside (Compound No. 71);
1,2,5,6-Di-O-isopropylidene-3-deoxy-3- {[(4-thiomethylphenyl)-amino]thiocarbonyl}-amino-α-D-allofuranoside (Compound No. 72);
1,2,5,6-Di-O-isopropylidene-3-deoxy-3-{[(4-(4-propylcyclohexyl)phenyl)-amino]carbonyl}-amino-α-D-allofuranoside (Compound No. 88);
1,2,5,6-Di-O-isopropylidene-3-deoxy-3-{[(4-(4-hexylcyclohexyl)phenyl)-amino]carbonyl}-amino-α-D-allofuranoside (Compound No. 89);
1,2,5,6-Di-O-isopropylidene-3-deoxy-3- {[(pyridin-3-yl)-amino]thiocarbonyl}-amino-α-D-allofuranoside (Compound No. 90);
1,2,5,6-Di-O-isopropylidene-3-deoxy-3-{[(4-chlorophenyl)-amino]thiocarbonyl}-amino-α-D-allofuranoside (Compound No. 91);
1,2;5,6-Di-O-isopropylidene-3-deoxy-3-{[(2-chlorophenyl)-amino]thiocarbonyl}-amino-α-D-allofuranoside (Compound No. 92);
1,2;5,6-Di-O-isopropylidene-3-deoxy-3-{[(3-chlorophenyl)-amino]thiocarbonyl}-amino-α-D-allofuranoside (Compound No. 93);
1,2;5,6-Di-O-isopropylidene-3-deoxy-3-{[(2,6-dichlorophenyl)-amino]thiocarbonyl}-amino-α-D-allofuranoside (Compound No. 94);
1,2;5,6-Di-O-isopropylidene-3-deoxy-3- {[(2,6-dimethylphenyl)-amino]thiocarbonyl}-amino-α-D-allofuranoside (Compound No. 95);
1,2;5,6-Di-O-isopropylidene-3-deoxy-3-{[(2,5-difluorophenyl)-amino]thiocarbonyl}-amino-α-D-allofuranoside (Compound No. 96);
1,2,5,6-Di-O-isopropylidene-3-deoxy-3-{[(4-iodophenyl)-amino]thiocarbonyl}-amino-α-D-allofuranoside (Compound No. 97);
1,2,5,6-Di-O-isopropylidene-3-deoxy-3-{[(3-methoxyphenyl)-amino]thiocarbonyl}-amino-α-D-allofuranoside (Compound No. 98);
1,2;5,6-Di-O-isopropylidene-3-deoxy-3- {[(2-methoxyphenyl)-amino]thiocarbonyl}-amino-α-D-allofuranoside (Compound No. 99);
1,2,5,6-Di-O-isopropylidene-3-deoxy-3-{[(4-fluorophenyl)-amino]thiocarbonyl}-amino-α-D-glucofuranoside (Compound No. 101);
1,2,5,6-Di-O-isopropylidene-3-deoxy-3-{[(3-nitrophenyl)-amino]thiocarbonyl}-amino-α-D-glucofuranoside (Compound No. 102);
1,2;5,6-Di-O-isopropylidene-3-deoxy-3-{[(2-fluorophenyl)-amino]thiocarbonyl}-amino-α-D-glucofuranoside (Compound No. 103);
1,2;5,6-Di-O-isopropylidene-3-deoxy-3-{[(2-trifluoromethylphenyl)-amino]thiocarbonyl}-amino-α-D-glucofuranoside (Compound No. 104);
1,2;5,6-Di-O-isopropylidene-3-deoxy-3-{[(2,4-difluorophenyl)-amino]thiocarbonyl}-amino-α-D-glucofuranoside (Compound No. 105);
1,2;5,6-Di-O-isopropylidene-3-deoxy-3-{[(2-difluoromethoxyphenyl)-amino]thiocarbonyl}-amino-α-D-glucofuranoside (Compound No. 106); and
1,2;5,6-Di-O-isopropylidene-3-deoxy-3-1[(4-difluoromethoxyphenyl)-amino]thiocarbonyl}-amino-α-D-glucofuranoside (Compound No. 107).

An alternative route for the synthesis of compounds of Formula VII is outlined, for example, in Scheme II below:

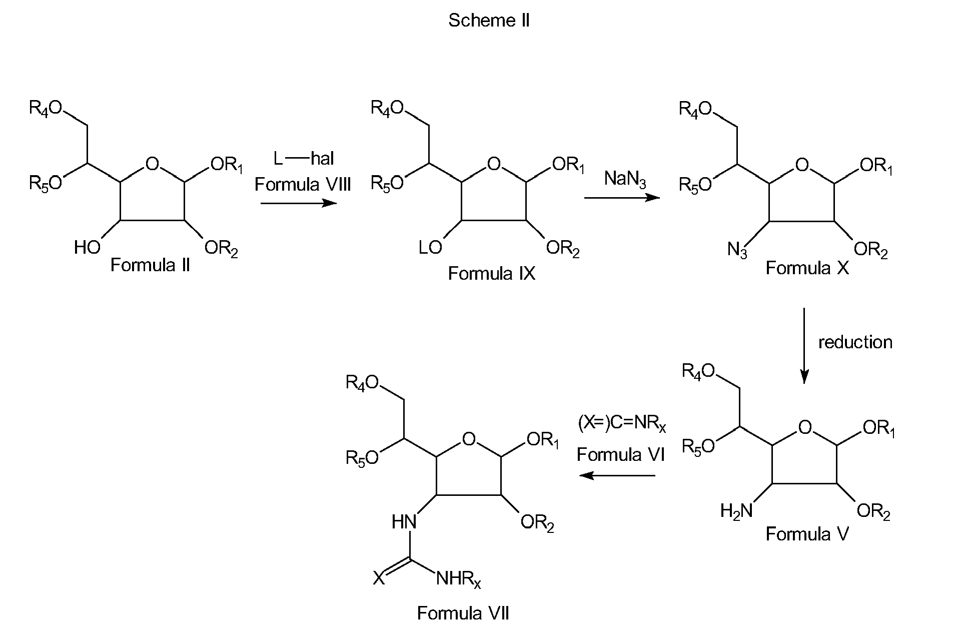

Thus, a compound of Formula II (wherein R₁, R₂, R₄ and R₅ are the same as defined earlier) can be reacted with a compound of Formula VIII [wherein L is a leaving group such as tosyl or mesyl and hal is a halogen (Cl, Br, I)] to form a compound of Formula IX, which can be reacted with , for example, sodium azide to form a compound of Formula X, which can undergo reduction to yield a compound of Formula V, which can be reacted with a compound of Formula VI (wherein X is O or S and Rₓ is the same as defined earlier) to furnish a compound of Formula VII. A compound of Formula II can be reacted with a compound of Formula VIII to form a compound of Formula IX in the presence of an organic base, such as pyridine, triethylamine or diisopropylethylamine. Alternatively, the hydroxyl group in a compound of Formula II can also be converted to a triflyl group with triflic anhydride.

A compound of Formula IX can be reacted with , for example, sodium azide in an organic solvent such as dimethylformamide, tetrahydrofuran, dioxane or diethyl ether.

Alternatively, one may also use trimethylsilyl azide or lithium azide in place of sodium azide. Similarly one may also use benzyl amine in place of azido moiety, which on debenzylation would furnish a compound of Formula V.

A compound of Formula X can be reduced to give a compound of Formula V in an organic solvent such as tetrahydrofuran, dioxane, ethanol or diethyl ether, with a reducing agent such as lithium aluminum hydride or sodium borohydride. Alternatively, the reduction of a compound of Formula X can also be carried out by hydrogenation in the presence of catalytic palladium on carbon.

A compound of Formula V can be reacted with a compound of Formula VI to yield a compound of Formula VII in an organic solvent such as acetonitrile, dichloromethane, dichloroethane, chloroform or carbon tetrachloride.

Also, the alternate strategies mentioned in Scheme I are applicable for the syntheses of compounds of Formula VII.

Particular compounds which may be prepared following, for example, Scheme II include:
1,2;5,6-Di-O-isopropylidene-3-deoxy-3-([{4-(2-methoxy-2-oxoethyl)-phenyl}-amino]- carbonyl)-amino-α-D-glucofuranoside (Compound No. 4);
1,2;5,6-Di-O-isopropylidene-3-deoxy-3-([{4-(2-hydroxy-2-oxoethyl)-phenyl}-amino]-carbonyl)-amino-α-D-glucofuranoside (Compound No. 6);
(3R or 3S)-1,2;5,6-Di-O-isopropylidene-3-deoxy-3-[{(4-methyl-phenyl)-amino}-carbonyl]-amino-α-D-glucofuranoside (Compound No. 8);
(3R or 3S)-1,2;5,6-Di-O-isopropylidene-3-deoxy-3-[{(4-chloro-phenyl)-amino}-carbonyl]-amino-α-D- glucofuranoside (Compound No. 10);
1,2;5,6-Di-O-isopropylidene-3-deoxy-3-[phenyl-amino-carbonyl]-amino-α-D-glucofuranoside (Compound No. 12);
(3R or 3S)-1,2;5,6-Di-O-isopropylidene-3-deoxy-3-[{(4-nitro-phenyl)-amino}-carbonyl]-amino-α-D-glucofuranoside (Compound No. 14); and
1,2;5,6-Di-O-isopropylidene-3-deoxy-3-[{(4-fluoro-phenyl)-amino}-carbonyl]-amino-α-D-allofuranoside (Compound No. 51).

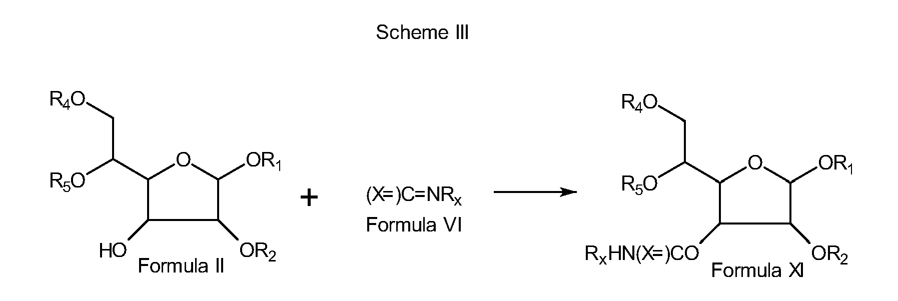

A compound of Formula XI can be prepared, for example, by following Scheme III. Thus, a compound of Formula II (wherein R₁, R₂, R₄ and R₅ are the same as defined earlier) can be reacted with a compound of Formula VI (wherein X is O or S and Rₓ is the same as defined earlier) to form a compound of Formula XI.

A compound of Formula II can be reacted with a compound of Formula VI to furnish a compound of Formula XI in an organic solvent such as dichloromethane, dichloroethane, chloroform or carbon tetrachloride.

Particular illustrative compounds which may be prepared following for example, Scheme III include:
1,2;5,6-Di-O-isopropylidene-3-O-[(4-fluoro-phenyl)-amino]-carbonyl]-α-D-glucofuranoside (Compound No. 48);
1,2;5,6-Di-O-isopropylidene-3-O-[(4-methyl-phenyl)-amino]-carbonyl]-α-D-glucofuranoside (Compound No. 13);
1,2;5,6-Di-O-isopropylidene-3-O-[(4-methoxy-phenyl)-amino]-carbonyl-α-D-glucofuranoside (Compound No. 15); and
1,2;5,6-Di-O-isopropylidene-3-O-[(4-chloro-phenyl)-amino]-carbonyl-α-D-glucofuranoside (Compound No. 16).

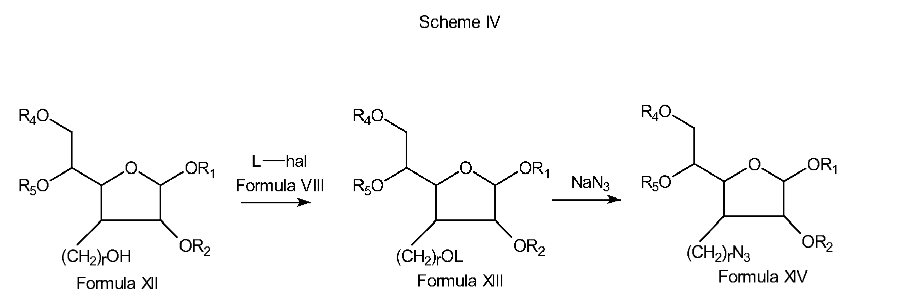

A compound of Formula XVI can be prepared, for example, by Scheme IV. Thus, a compound of Formula XII (wherein R₁, R₂, R₄ and R₅ are the same as defined earlier and r is an integer from 1 to 3) can be reacted with a compound of Formula VIII [wherein L is a leaving group such as mesyl or tosyl and hal is a halogen (Cl, Br, I)] to form a compound of Formula XIII, which can be reacted with , for example, sodium azide to form a compound of Formula XIV, which can undergo reduction to give a compound of Formula XV, which can be reacted with a compound of Formula VI to furnish a compound of Formula XVI.

A compound of Formula XII can be reacted with a compound of Formula VIII to form a compound of Formula XIII in the presence of a base such as pyridine, triethylamine or diisopropylethylamine, in an organic solvent selected from the group such as dichloromethane, tetrahydrofuran or dimethylformamide. Thus for example, a compound of Formula XII can be reacted with p-toluenesuphonyl chloride to form the tosyl compound, a compound of Formula XIII.

A compound of Formula XIII can be reacted with, for example, sodium azide to form a compound of Formula XIV in an organic solvent such as dimethylformamide, tetrahydrofuran, dioxane or diethyl ether.

A compound of Formula XIV can be reduced to yield a compound of Formula XV in an organic solvent such as tetrahydrofuran, dimethylformamide, dioxane or diethyl ether, with a reducing agent such as lithium aluminum hydride, sodium borohydride.

Alternatively, a compound of Formula XV can also be prepared by hydrogenation of a compound of Formula XIV in the presence, for example, of palladium on carbon.

A compound of Formula XV can be reacted with a compound of Formula VI in a organic solvent selected from, for example, dichloromethane, dichloroethane, carbon tetrachloride or chloroform.

Alternative methods as provided for the synthesis of a compound of Formula VII in Scheme I, are also applicable for the synthesis of a compound of Formula XVI.

Particular illustrative compounds which may be prepared following, for example, Scheme IV include:
1,2;5,6-Di-O-isopropylidene-3-deoxy-3-[2-{3-(4-chloro-phenyl)-ureido}-ethyl]-α-D-allofuranoside (Compound No. 2);
1,2;5,6-Di-O-isopropylidene-3-deoxy-3-[2-{3-(4-methyl-phenyl)-ureido}-ethyl]-α-D-allofuranoside (Compound No. 17); and
1,2;5,6-Di-O-isopropylidene-3-deoxy-3-{2-[3-(4-methoxy-phenyl)-ureido]-ethyl}-α-D-allofuranoside (Compound No. 18).

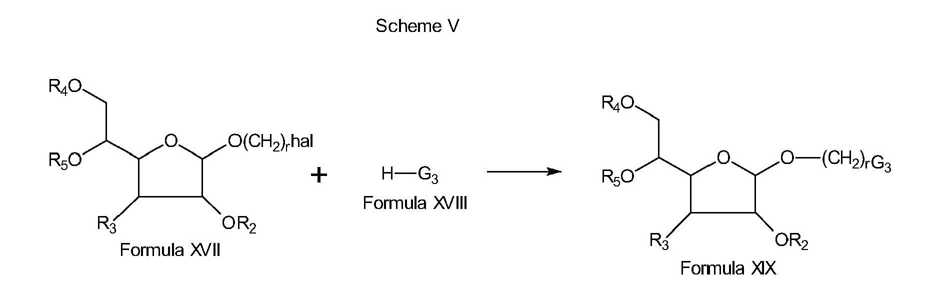

Compounds of Formula XIX can be prepared, for example, by Scheme V. Thus, a compound of Formula XVII (wherein R₂, R₃, R₄ and R₅ are the same as defined earlier, r is an integer from 1 to 3 and hal is (Cl, Br, I) can be reacted with a compound of Formula XVIII (wherein G₃ is a heterocyclyl ring attached to H through N) to yield a compound of Formula XIX.

A compound of Formula XVII can be reacted with a compound of Formula XVIII to form a compound of Formula XIX in an organic solvent such as dimethylformamide, tetrahydrofuran, dioxane or diethyl ether, in the presence of a base such as potassium carbonate, sodium bicarbonate, triethyl amine, pyridine or diisopropylethylamine.

Particular illustrative compounds which may be prepared following for example Scheme V include:
2,3;5,6-Di-O-isopropylidene-1-O-{3-[1-(4-[3-chloro-phenyl]-piperazinyl)]-propyl}-α-D-mannofuranoside (Compound No. 29);
2,3;5,6-Di-O-isopropylidene-1-O- {2-[1-(4-[4-chloro-phenyl]-piperazinyl)]-ethyl}-α-D-mannofuranoside (Compound No. 30);
2,3;5,6-Di-O-isopropylidene-1-O-{2-[1-(4-[4-methoxy-phenyl]-piperazinyl)]-ethyl}-α-D-mannofuranoside (Compound No. 31);
2,3;5,6-Di-O-isopropylidene-1-O- {2-[1-(4-[2-pyrimidinyl]-piperazinyl)]-ethyl}-α-D-mannofuranoside (Compound No. 32);
2,3;5,6-Di-O-isopropylidene-1-O-{2-[4-morpholinyl]-ethyl}-α-D-mannofuranoside (Compound No. 33);
2,3;5,6-Di-O-isopropylidene-1-O- {2-[1-(4-benzyl-piperazinyl)]-ethyl}-α-D-mannofuranoside (Compound No. 34);
2,3;5,6-Di-O-isopropylidene-1-O-{2-[1-(4-[4-chloro-phenyl-amino-carbonyl]-piperazinyl)]-ethyl}-α-D-mannofuranoside (Compound No. 35); and
2,3;5,6-Di-O-isopropylidene-1-O- {2-(1-piperazinyl)-ethyl}-α-D-mannofuranoside (Compound No. 36).

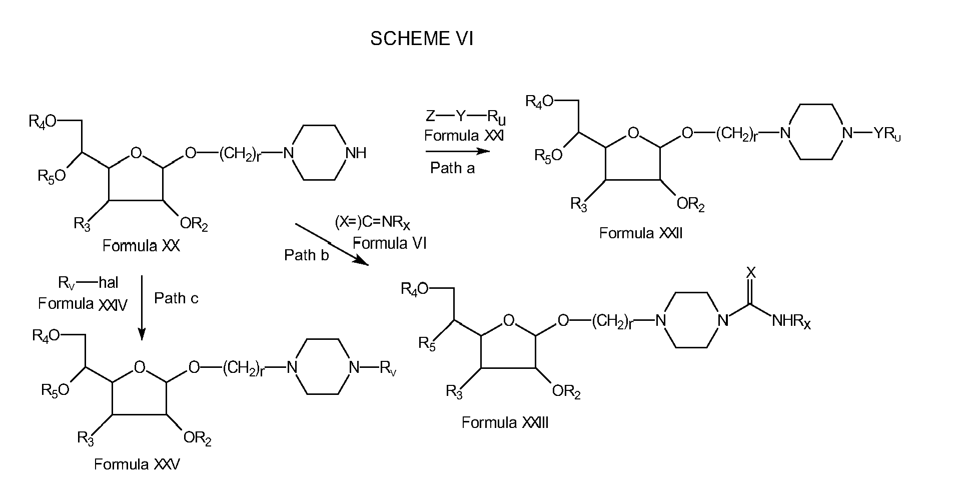

Compounds of Formulae XXII, XXIII and XXV can be prepared, for example, by Scheme VI.
*Path a:* A compound of Formula XX (wherein r, R₂, R₃, R₄, R₅ are the same as defined earlier) can reacted with a compound of Formula XXI (wherein Z is a halogen (Cl, Br, I) or OH, Y and Rᵤ are the same as defined earlier) to yield a compound of Formula XXII.
*Path b:* A compound of Formula XX can be reacted with a compound of Formula VI (wherein X is O or S and Rₓ is the same as defined earlier) to form a compound of Formula XXIII.
*Path* c: A compound of Formula XX can be reacted with a compound of Formula XXIV (wherein Rᵥ is alkyl and hal (Cl, Br, I) to form a compound of Formula XXV.

A compound of Formula XX (Path a) can be reacted with a compound of Formula XXI [when Y is -C(=O)] to furnish a compound of Formula XXII an organic solvent such as dichloromethane, dichloroethane, carbon tetrachloride or chloroform, in the presence of an organic base such as triethylamine, pyridine or diisopropylamine. Alternatively, when Y is -C(=O), a compound of Formula XX may react with "an activated derivative of a carboxylic acid" to furnish a compound of Formula XXII.

A compound of Formula XX (Path b) can be reacted with a compound of Formula VI to yield a compound of Formula XXIII in an organic solvent such as dichloromethane, chloroform, carbon tetrachloride or tetrahydrofuran.

Alternative methods as provided for the synthesis of a compound of Formula VII in Scheme I, are also applicable for the synthesis of a compound of Formula XXIII.

A compound of Formula XX (Path c) can be reacted with a compound of Formula XXIV to form a compound of Formula XXV in the presence of a base such as potassium carbonate, sodium bicarbonate, triethylamine, pyridine or diisopropylethylamine, in an organic solvent such as dimethylformamide, tetrahydrofuran, dioxane or diethyl ether.

Particular illustrative compounds which may be prepared following, for example, Scheme VI, *path a* include:
2,3;5,6-Di-O-isopropylidene-1-O-{2-[1-(4-{4-methyl-phenyl-sulphonyl}-piperazinyl)]-ethyl}-α-D-mannofuranoside (Compound No. 40);
2,3;5,6-Di-O-isopropylidene-1-O-{2-[1-(4-[2-thienyl-methyl-carbonyl]-piperazinyl)]-ethyl}-α-D-mannofuranoside (Compound No. 42);
2,3;5,6-Di-O-isopropylidene-1-O-{2-[1-(4-[4-fluoro-phenyl-carbonyl]-piperazinyl)]-ethyl}-α-D-mannofuranoside (Compound No. 43).

Particular illustrative compounds which may be prepared following, for example, Scheme VI, *path b* include:
2,3,5,6-Di-O-isopropylidene-1-O-{2-[1-(4-[isopropylamino-thiocarbonyl]-piperazinyl)]-ethyl}-α-D-mannofuranoside (Compound No. 38);
2,3,5,6-Di-O-isopropylidene-1-O-{2-[1-(4-{(1-naphthyl)-amino-carbonyl}-piperazinyl)]-ethyl}-α-D-mannofuranoside (Compound No. 39).

Particular illustrative compounds which may be prepared following, for example, Scheme VI, *path* c include:
2,3;5,6-Di-O-isopropylidene-1-O-{2-[1-(4-{[3,3']-bithiophenyl-5-ylmethyl}-piperazinyl)]-ethyl}-α-D-mannofuranoside (Compound No. 37); and
2,3;5,6-Di-O-isopropylidene-1-O-{2-[1-(4-[(2-chloro-3,4-methylenedioxyphenyl)-methyl]-piperazinyl)]-ethyl}-α-D-mannofuranoside (Compound No. 45).

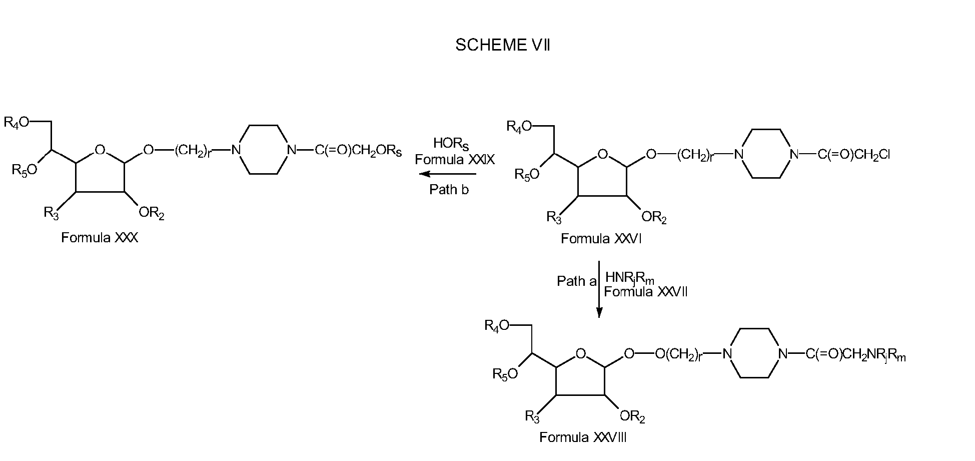

Particular illustrative compounds of Formula XXVIII and XXX can be prepared, for example, by Scheme VII.
*Path a:* A compound of Formula XXVI (wherein R₂, R₃, R₄ and R₅ are the same as defined earlier and r is an integer from 1 to 3) can reacted with a compound of Formula XXVII (wherein Rⱼ and Rₘ are the same as defined earlier) to form a compound of Formula XXVIII.
*Path b:* A compound of Formula XXVI can be reacted with a compound of Formula XXIX (wherein Rₛ is the same as defined earlier) to give a compound of Formula XXX.

A compound of Formula XXVI (path a) can be reacted with a compound of Formula XXVII to form a compound of Formula XXVIII in the presence of a base such as potassium carbonate, sodium bicarbonate, triethylamine, pyridine or diisopropylethylamine in an organic solvent such as dimethylformamide, tetrahydrofuran, diethyl ether, or dioxane.

A compound of Formula XXVI (path b) can be reacted with a compound of Formula XXIX to form a compound of Formula XXX in the presence of a base such as potassium hydroxide, cesium carbonate, potassium carbonate, sodium hydride, potassium tert-butoxide, in an organic solvent such as dimethylformamide, tetrahydrofuran, dioxane and diethyl ether.

Particluar illustrative compounds which may be prepared following, for example, Scheme VII, *path a* include:
2,3,5,6-Di-O-isopropylidene-1-O- {2-[1-(4-[2-(2,6-dioxo-1-piperidinyl)-acetyl]-piperazinyl)]-ethyl}-α-D-mannofuranoside (Compound No. 41)
2,3,5,6-Di-O-isopropylidene-1-O-{2-[1-(4-{2-(1-[1*H*-1,2,4-triazolyl])-acetyl}-piperazinyl]-ethyl}-α-D-mannofuranoside (Compound No. 44)

Particular illustrative compounds which may be prepared, for example, following Scheme VII, path b include:
2,3;5,6-Di-O-isopropylidene-1-O-{2-[1-(4-{2-[4-chloro-phenoxy]-acetyl}-piperazinyl)]-ethyl}-α-D-mannofuranoside (Compound No. 46).

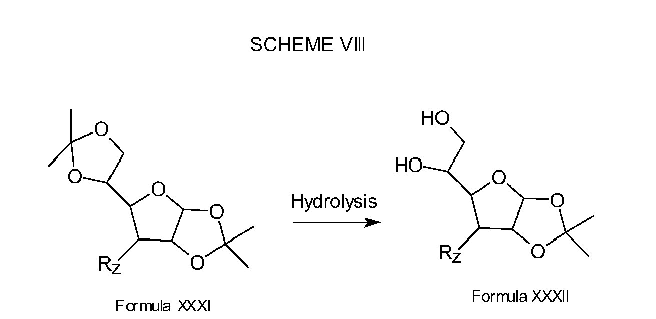

A compound of Formula XXXII, can be prepared by Scheme VIII. Thus, a compound of Formula XXXI (wherein R₃ is the same as defined earlier) can be hydrolysed to yield a compound of Formula XXXII.

A compound of Formula XXXI can be hydrolyzed with the reagents, for example aqueous perchloric acid, aqueous acetic acid, aqueous sulphuric acid or Dowex 50W-8X (commercially available) to form a compound of Formula XXXII in an organic solvent such as methanol, tetrahydrofuran, dimethylformamide, dioxane or diethyl ether.

Particular illustrative compounds which may be prepared following, for example, Scheme VIII include:
1,2-O-Isopropylidene-3-deoxy-3-{[(4-methoxy-phenyl)-amino]-carbonyl}-amino-α-D-allofuranoside (Compound No. 19);
1,2-O-Isopropylidene-3-deoxy-3-{2-[3-(4-methoxy-phenyl)-ureido]-ethyl}-α-D-allofuranoside (Compound No. 20);
1,2-O-Isopropylidene-3-O-{(4-chloro-phenyl)-amino}-carbonyl-α-D-allofuranoside (Compound No. 21);
1,2-O-Isopropylidene-3-deoxy-3-{[(4-nitro-phenyl)-amino]-carbonyl}-amino-α-D-allofuranoside (Compound No. 22);
1,2-O-Isopropylidene-3-deoxy-3-{[(4-chloro-phenyl)-amino]-carbonyl}-amino-α-D-allofuranoside (Compound No. 23);
1,2-O-Isopropylidene-3-O- {(4-methyl-phenyl)-amino}-carbonyl-α-D-allofuranoside (Compound No. 24);
1,2-O-Isopropylidene-3-deoxy-3-[2-{3-(4-methyl-phenyl)-ureido}-ethyl]-α-D-allofuranoside (Compound No. 25);
1,2-O-Isopropylidene-3-deoxy-3-{2-[3-(4-{2-methoxy-2-oxo-ethyl}-phenyl)-ureido]-ethyl}-α-D-allofuranoside (Compound No. 26);
1,2-O-Isopropylidene-3-deoxy-3-{2-[(4-methyl-phenyl)-amino]-carbonyl}-amino-α-D-allofuranoside (Compound No. 27); and
1,2-O-Isopropylidene-3-deoxy-3-{2-[3-(4-{2-hydroxy-2-oxo-ethyl}-phenyl)-ureido]-ethyl}-α-D-allofuranoside (Compound No. 28).

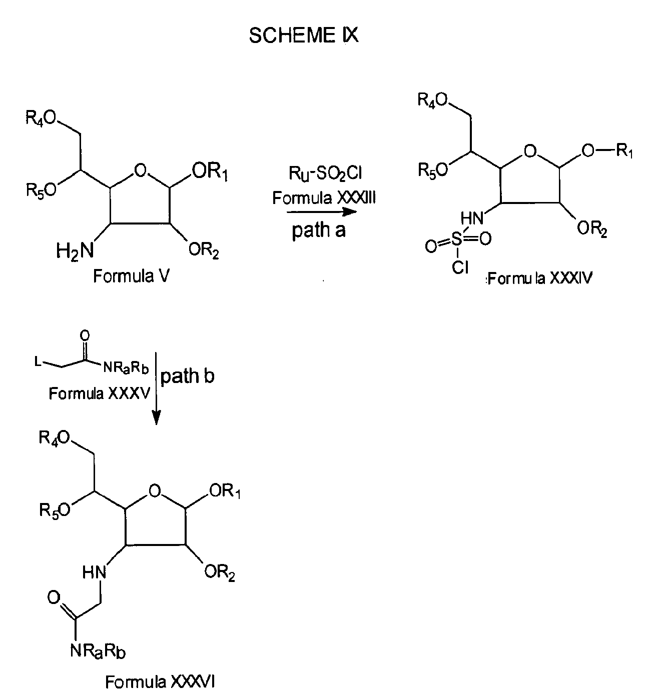

The compounds of the Formula XXXIV and the Formula XXXVI can be prepared, for example, by Scheme IX.
*Path a:* the compound of Formula V (wherein R₁, R₂, R₄ and R₅ are the same as defined earlier) can be reacted with a compound of Formula XXXIII (wherein Rᵤ is the same as defined earlier) to form a compound of Formula XXXIV.
*Path b:* the compound of the Formula V can be reacted with a compound of Formula XXXV (wherein L is a leaving group such as halogen) to form a compound of Formula XXXVI.

The compound of Formula V (path a) can be reacted with a compound of Formula XXXIII to form a compound XXXIV in an organic solvent such as dichloromethane, carbon tetrachloride, tetrahydrofuran or dimethylformamide, in the presence of an organic base such as triethylamine, pyridine, or diisopropoylethylamiine.

The compound of Formula V (path b) can be reacted with a compound of Formula XXXV in a organic solvent such as acetone, tetrahydrofuran, dimethylformamide, acetonitrile or dimethylsulphoxide, in the presence of a base such as potassium carbonate, sodium bicarbonate, triethylamine or pyridine.

Particular illustrative compounds which may be prepared following, for example, Scheme IX, path a include:
1,2;5,6-Di-O-isopropylidene-3-deoxy-3-{[4-fluoro-phenyl]sulphonyl}-amino-α-D-allofuranoside (Compound No. 52);
1,2;5,6-Di-O-isopropylidene-3-deoxy-3-{[4-methyl-phenyl]-sulphonyl}-amino-α-D-allofuranoside (Compound No. 53); and
1,2;5,6-Di-O-isopropylidene-3-deoxy-3-{[(4-[2-methoxy-2-oxo-ethyl]-phenyl)-amino]-carbonyl}-methylamino-α-D-allofuranoside (Compound No. 54).

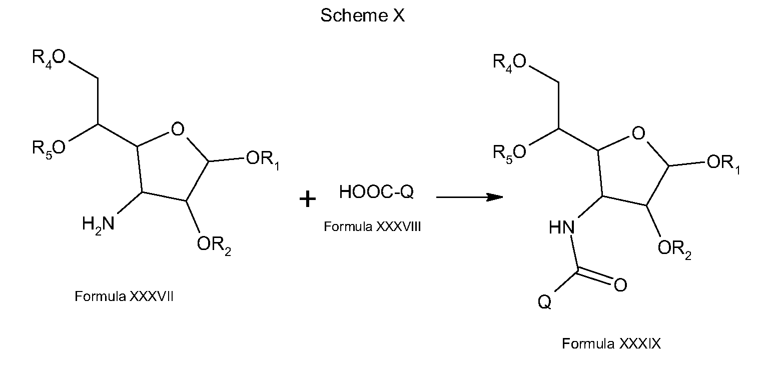

Compounds of Formula XXXIX can be prepared, for example, by Scheme X. Thus the compound of Formula XXXVII (wherein R₁, R₂, R₄ and R₅ are the same as defined earlier) can be reacted with a compound of Formula XXXVIII (wherein Q is substituted alkyl) to give a compound of Formula XXXIX.

The reaction of a compound of Formula XXXVII with a compound of Formula XXXVIII to give a compound of Formula XXXIX can be carried out in an organic solvent selected from dimethylformamide, dichloromethane, chloroform, tetrahydrofuran, dioxane or diethylether in presence of a base selected from N-methylmorpholine, triethylamine, diisopropylethylamine or pyridine with a condensing agent selected from 1-(3-dimethylaminopropyl)-3-ethylcarbodiimide hydrochloride (EDCI.HC1) or dicyclohexylcarbodiimide (DCC).

Particular illustrative compounds which can be prepared following, for example, Scheme X include:
1,2;5,6-Di-O-isopropylidene-3-deoxy-3-{[(3,5-difluorophenyl)-methyl]carbonyl}-amino-α-D-allofuranoside (Compound No. 57);
1,2;5,6-Di-O-isopropylidene-3-deoxy-3-{[(2,4-difluorophenyl)-methyl]carbonyl}-amino-α-D-allofuranoside (Compound No. 73);
1,2;5,6-Di-O-isopropylidene-3-deoxy-3-{[(3-methoxyphenyl)-methyl]carbonyl}-amino-α-D-allofuranoside (Compound No. 74);
1,2;5,6-Di-O-isopropylidene-3-deoxy-3-{[(2-methoxyphenyl)-methyl]carbonyl}-amino-α-D-allofuranoside (Compound No. 75);
1,2;5,6-Di-O-isopropylidene-3-deoxy-3-{[(2-chloro-4-fluorophenyl)-methyl]carbonyl}-amino-α-D-allofuranoside (Compound No. 76);
1,2;5,6-Di-O-isopropylidene-3-deoxy-3-{[(4-methylphenyl)-methyl]carbonyl}-amino-α-D-allofuranoside (Compound No. 77);
1,2;5,6-Di-O-isopropylidene-3-deoxy-3-{[(4-nitrophenyl)-methyl]carbonyl}-amino-α-D-allofuranoside (Compound No. 78);
1,2;5,6-Di-O-isopropylidene-3-deoxy-3-{[(2-nitrophenyl)-methyl]carbonyl}-amino-α-D-allofuranoside (Compound No. 79);
1,2;5,6-Di-O-isopropylidene-3-deoxy-3-{[(2-trifluoromethylphenyl)-methyl]carbonyl}-amino-α-D-allofuranoside (Compound No. 80);
1,2;5,6-Di-O-isopropylidene-3-deoxy-3-{[(2-trifluoromethoxyphenyl)-methyl]carbonyl}-amino-α-D-allofuranoside (Compound No. 81);
1,2;5,6-Di-O-isopropylidene-3-deoxy-3- {[(3-trifluoromethoxyphenyl)-methyl]carbonyl}-amino-α-D-allofuranoside (Compound No. 82);
1,2;5,6-Di-O-isopropylidene-3-deoxy-3-{[(4-isopropylphenyl)-methyl]carbonyl}-amino-α-D-allofuranoside (Compound No. 83);
1,2;5,6-Di-O-isopropylidene-3-deoxy-3-{[(3-chlorophenyl)-methyl]carbonyl}-amino-α-D-allofuranoside (Compound No. 84);
1,2;5,6-Di-O-isopropylidene-3-deoxy-3-{[(3-methylphenyl)-methyl]carbonyl}-amino-α-D-allofuranoside (Compound No. 85);
1,2;5,6- Di-O-isopropylidene-3-deoxy-3-{[(2,4-dichlorophenyl)-methyl]carbonyl}-amino-α-D-allofuranoside (Compound No. 86);
1,2;5,6-Di-O-isopropylidene-3-deoxy-3-{[(3-chlorophenyl)-methyl]carbonyl}-amino-α-D-allofuranoside (Compound No. 87);
1,2;5,6-Di-O-isopropylidene-3-deoxy-3-{[(3,4-dichlorophenyl)-methyl]carbonyl}-amino-α-D-allofuranoside (Compound No. 100);
1,2;5,6-Di-O-isopropylidene-3-deoxy-3-{[(4-(phenyl)phenyl)-methyl]carbonyl}-amino-α-D-allofuranoside (Compound No. 108);
1,2;5,6-Di-O-isopropylidene-3-deoxy-3-{[(2-naphthyl)-methyl]carbonyl}-amino-α-D-allofuranoside (Compound No. 109);
1,2;5,6-Di-O-isopropylidene-3-deoxy-3-{[(2-fluorophenyl)-methyl]carbonyl}-amino-α-D-allofuranoside (Compound No. 110);
1,2;5,6-Di-O-isopropylidene-3-deoxy-3-{[(2-chloro-6-fluorophenyl)-methyl]carbonyl}-amino-α-D-allofuranoside (Compound No. 111);
1,2;5,6-Di-O-isopropylidene-3-deoxy-3-{[(2,5-difluorophenyl)-methyl]carbonyl}-amino-α-D-allofuranoside (Compound No. 112);
1,2;5,6-Di-O-isopropylidene-3-deoxy-3-{[(4-fluorophenyl)-methyl]carbonyl}-amino-α-D-allofuranoside (Compound No. 113);
1,2;5,6-Di-O-isopropylidene-3-deoxy-3-{[(3-fluorophenyl)-methyl]carbonyl}-amino-α-D-allofuranoside (Compound No. 114);
1,2;5,6-Di-O-isopropylidene-3-deoxy-3-{[(3-bromophenyl)-methyl]carbonyl}-amino-α-D-allofuranoside (Compound No. 115); and
1,2;5,6-Di-O-isopropylidene-3-deoxy-3-{[(3,4-difluorophenyl)-methyl]carbonyl}-amino-α-D-allofuranoside (Compound No. 116).

Also, in all the above representative examples wherever esters are specified, one skilled in the art could optionally hydrolyze them to their respective acids, for example hydrolysis of alkyl esters (such as ethyl, methyl or benzyl ester) to their corresponding acids can be carried out in the presence of a base, for example, lithium hydroxide, sodium hydroxide or potassium hydroxide. Alternatively, hydrolysis of benzyl ester can be carried out hydrogenatically using catalysts, for example, palladium on carbon or platinum on carbon. Esters such as tert-butyl can be hydrolyzed to their corresponding acids in the presence of acid, for example, trifluoroacetic acid or hydrochloric acid.

In the above schemes, where specific bases, acids, solvents, condensing agents, hydrolyzing agents, etc., are mentioned, it is to be understood that other acids, bases, solvents, condensing agents, hydrolyzing agents, etc., may also be used. Similarly, the reaction temperature and duration of the reactions may be adjusted according to the requirements that arise during the process.

Examples set forth general synthetic procedures for the preparation of representative compounds. The examples are provided to illustrate particular aspect of the disclosure and do not be limit the scope of the present invention.

### EXAMPLES

### Example A: Synthesis of 1,2;5,6-Di-O-isopropylidene-3-deoxy-3-amino-α-D-allofuranoside

### Step a: Synthesis of 1,2;5,6-di-O-isopropylidene-3-oxo-α-D-glucofuranoside

To diacetoneglucose (25g) (commercially available) was added dimethyl sulphoxide (100ml) and acetic anhydride (50ml). The reaction mixture was stirred at 50-60°C for 24 hours. Dimethyl sulphoxide was evaporated under reduced pressure and water (2.5ml) was added with vigorous stirring followed by the addition of ether (10ml) and hexane. The mixture was kept in refrigerator for overnight. The solid thus separated was filtered to obtain the title compound (16g).

### Step b: Synthesis of 1,2;5,6-di-O-isopropylidene-3-deoxy-3-hydroxyimino-α-D-glucofuranoside

To a compound from *step a* above (12g), was added hydroxylamine hydrochloride (2.5g) pyridine (100ml) and anhydrous ethanol (100ml) at room temperature. The reaction mixture was stirred for half an hour .The temperature of the reaction was raised to 75°C and the reaction mixture was stirred for 24 hours. The solvents were evaporated off under reduced pressure and the residue thus obtained was poured into ice cold water. The organic product was extracted with ethyl acetate followed by washing with water, brine and dried over anhydrous sodium sulphate. The solvent was evaporated under reduced pressure and the product was purified by column chromatography using 25% ethyl acetate in hexane as eluent to furnish the title compound (8.5g).

### Step c: Synthesis of 1,2;5,6-Di-O-isopropylidene-3-deoxy-3-amino-α-D-allofuranoside

To a suspension of lithium aluminum hydride (8.4g) in tetrahydrofuran (50 ml) at 0°C, was added the compound obtained from *step b* above (8.5g in 50ml tetrahydrofuran) with constant stirring. After complete addition, the reaction mixture was allowed to attain room temperature and stirred for 8 hours. The excess of lithium aluminum hydride was decomposed by addition of ethyl acetate (100 ml) followed by the addition of water and sodium hydroxide solution (2ml, 15%) dropwise at 0°C .The reaction mixture was filtered off, washed with warm ethyl acetate and dried over anhydrous sodium sulphate. The solvent was evaporated under reduced pressure and the crude compound was purified by column chromatography using 50% methanol in ethyl acetate as eluent to furnish the title compound (7.0g).

### Example B: Synthesis of 1,2;S,6-Di-O-isopropylidene-3-deoxy-3-hydroxyethyl-∝-D allofuranoside

### Step a: Synthesis of 1,2;5,6-di-O-isopropylidene-3-oxo-α-D-glucofuranoside

To diacetone glucose (25g) (commercially available) was added dimethyl sulphoxide (100 ml) and acetic anhydride (50ml) .The reaction mixture was stirred at 50-60°C for 24 hours. Dimethyl sulphoxide was evaporated under reduced pressure and water was added with constant stirring followed by the addition of ether (10ml) and hexane. The mixture was kept in refrigerator for overnight and the solid thus separated was filtered to obtain the title compound (16g).

### Step b: Synthesis of 1,2,5,6-di-O-isopropylidene-3-deoxy-3-(methoxycarbonylmethylene)-α-D-glucofuranoside

The ice-cold solution of trimethyl phosphonoacetate (34ml) in dimethylformamide (34ml), was added potassium tert-butoxide (8.5g) and the reaction mixture was stirred at room temperature for 10 minutes. To it was added a solution of the compound (17g) obtained from the *step a* above in dimethyl formamide (34ml) and the reaction mixture was stirred for 1 hour at 0-10°C. The solvent was evaporated under reduced pressure and the residue was taken in water and extracted with ether followed by washing with water and brine. The mixture was dried over anhydrous sodium sulphate and the solvent was evaporated under reduced pressure. The crude compound thus obtained was taken in hexane and the mixture was kept in refrigerator for overnight. After trituration, the solid was separated out which was filtered and dried. The compound was purified by column chromatography using to furnish the title compound (11 g).

### Step c: Synthesis of 1,2;5,6-di-O-isopropylidene-3-deoxy-3-(methoxycarbonylmethyl)-α-D-allofuranoside

To a solution of a compound obtained from *step b* above (11g) in methanol (100ml) at 0-5°C, was added sodium borohydride (2.5g) in small portion with continuous stirring. The reaction mixture was stirred for one hour. The reaction mixture was allowed to come to room temperature followed by stirring for 24 hours. Acetone (10ml) was added to the reaction mixture to decompose excess of sodium borohydride. Methanol was removed under reduced pressure. The organic product was extracted with chloroform followed by washing with water, and brine. The reaction mixture was dried over anhydrous sodium sulphate. The solvent was evaporated under reduced pressure and the syrup thus obtained was taken in hexane, which on scratching gave solid product, which was filtered and dried. The crude compound was purified by column chromatography using 10% ethyl acetate in hexane as eluent (9g).

### Step d: Synthesis of 1,2;5,6-di-O-isopropylidene-3-(2-hydroxyethyl)-α-D-allofuranoside

To a suspension of lithium aluminum hydride (5.7g) in tetrahydrofuran (50ml) at 0°C, was added a solution of the compound (16g) obtained from *step c* above in tetrahydrofuran (50ml) dropwise with constant stirring. After complete addition the reaction mixture was allowed to attain room temperature and stirred for 8 hours. The excess of lithium aluminum hydride was decomposed by adding ethyl acetate (100ml) followed by the addition of water and aqueous sodium hydroxide solution (2ml, 15%) at 0°C. The reaction mixture was filtered, washed with warm ethyl acetate and dried over anhydrous sodium sulphate. The solvent was evaporated under reduced pressure and the crude product was purified by column chromatography using 20% methanol in ethyl acetate as eluent to furnish the title compound (1.1g).

### Example C: Synthesis of 1,2;5,6-di-O-isopropylidene-3-deoxy-3-ethylamino-α-D-allofuranoside

### Step a: Synthesis of 1,2;5,6-di-O-isopropylidene-3-deoxy-3-[2-(4-methyl-phenylsulphonyloxy)-ethyl]-α-D-allofuranoside

To a solution of 1,2;5,6-Di-O-isopropylidene-3-hydroxyethyl-α-D-allofuranoside (11g) in pyridine (15ml) was added a solution of p-toluenesulphonyl chloride (5.5g) in pyridine (15 ml) at 0°C with constant stirring .The reaction mixture was stirred for 6 hours. The temperature of the reaction mixture was gradually raised to 5°C and stirred for 12 hours. The reaction mixture was diluted with water. The solvents were evaporated off under reduced pressure and extracted with ethyl acetate followed by washing with water and brine and dried over anhydrous sodium sulphate. The solvent was evaporated under reduced pressure and the crude product was purified by column chromatography using 30% ethyl acetate in hexane as eluent to furnish the title compound. (9g).

### Step b: Synthesis of 1,2;5,6-di-O-isopropylidene-3-deoxy-3-[2-azidoethyl]-α-D-allofuranoside

To a suspension of the compound obtained from *step a* above (9g) in dimethylformamide (50 ml), was added sodium azide (1.58g). The reaction mixture was heated at 130°C for 8-10 hours. Dimethylformamide was evaporated under reduced pressure and the compound was extracted with ethyl acetate followed by washing with sodium bicarbonate, water, brine and dried over anhydrous sodium sulphate. The solvent was evaporated under reduced pressure to furnish the title product (3.5g).

### Step c: Synthesis of 1,2;5,6-di-O-isopropylidene-3-deoxy-3-ethylamino-α-D-allofuranoside

To a suspension of lithium aluminum hydride (1.0g) in tetrahydrofuran (50 ml) at 0°C was added a solution of the compound (4g) obtained from *step b* above in tetrahydrofuran (20ml) dropwise at 0°C with constant stirring. After complete addition, the reaction mixture was allowed to attain room temperature and stirred for 8 hours. The excess of lithium aluminum hydride was decomposed by adding ethyl acetate (100ml) followed by the addition of water and sodium hydroxide solution (2ml, 15%) at 0°C. The reaction mixture was filtered washed with warm ethyl acetate, the filtrate was dried over anhydrous sodium sulphate. The solvent was evaporated under reduced pressure and the product was purified by column chromatography using 20% methanol in ethyl acetate as eluent to give the desired product (2.8 g).

### Scheme I

### Example 1: Synthesis of 1,2;5,6-Di-O-isopropylidene-3-deoxy-3-[{(4-trifluoromethyl-phenyl)-amino)-carbonyl]-amino-α-D-allofuranoside (Compound No. 47)

4-Trifluoromethyl-phenyl isocyanate (144mg) was added slowly to a solution of 1,2;5,6-di-O-isopropylidene-3-deoxy-3-amino-α-D-allofuranoside (200mg) in dichloromethane (10 ml) at 0-5°C with constant stirring. After complete addition the reaction mixture was allowed to come to room temperature and stirred for 2 hours. The solvents were evaporated under reduced pressure and the crude product was purified by column chromatography using 50% ethyl acetate in hexane as eluent to furnish the title compound (340mg).
¹H NMR (CDCl₃) (300 MHz):δ 7.44-7.55(4H,m), 6.89(1H,s), 5.85(1H,s), 5.20(1H,d, 9Hz), 4.68(1H,m), 4.29(1H,m), 4.13(2H,m), 4.01(1H,m 3.89(1H,m), 1.55(3H,s), 1.45(3H,s), 1.35(6H,s).

Analogues of 1,2;5,6-Di-O-isopropylidene-3-deoxy-3-[{(4-trifluoromethyl-phenyl)-amino}-carbonyl]-amino-α-D-allofuranoside (Compound No.47) can be prepared by replacing appropriate isocyanate, respectively, as applicable in each case.
(3R or 3S)-1,2;5,6-Di-O-isopropylidene-3-deoxy-3-[{(4-chloro-phenyl)-amino}-carbonyl]-amino-α-D-allofuranoside (Compound No. 1);
1,2;5,6-Di-O-isopropylidene-3-deoxy-3-[{(4-methoxy-phenyl)-amino}-carbonyl]-amino-α-D-allofuranoside (Compound No. 3);
1,2;5,6-Di-O-isopropylidene-3-deoxy-3-[{phenyl-sulphonylamino}-carbonyl]-amino-α-D-allofuranoside (Compound No. 5);
1,2;5,6-Di-O-isopropylidene-3-deoxy-3-[{(4-methyl-phenyl)-sulphonylamino}-carbonyl]-amino-α-D-allofuranoside (Compound No. 7);
(3R or 3S)-1,2;5,6-Di-O-isopropylidene-3-deoxy-3-[{(4-nitro-phenyl)-amino}-carbonyl]-amino-α-D-allofuranoside (Compound No. 9);
(3R or 3S)-1,2;5,6-Di-O-isopropylidene-3-deoxy-3-[{(4-methyl-phenyl)-amino}-carbonyl]-amino-α-D-allofuranoside (Compound No. 11);
1,2;5,6-Di-O-isopropylidene-3-deoxy-3-{[(4-fluorophenyl)-amino]thiocarbonyl}-amino-α-D-allofuranoside (Compound No. 55);
1,2;5,6-Di-O-isopropylidene-3-deoxy-3-{[(4-trifluoromethoxyphenyl)-amino]thiocarbonyl}-amino-α-D-allofuranoside (Compound No. 56);
1,2;5,6-Di-O-isopropylidene-3-deoxy-3-{[(4-nitrophenyl)-amino]thiocarbonyl}-amino-α-D-allofuranoside (Compound No. 58);
1,2;5,6-Di-O-isopropylidene-3-deoxy-3-{[(2,4-difluorophenyl)-amino]thiocarbonyl}-amino-α-D-allofuranoside (Compound No. 59);
1,2;5,6-Di-O-isopropylidene-3-deoxy-3- {[(3,4-dichlorophenyl)-amino]thiocarbonyl}-amino-α-D-allofuranoside (Compound No. 60);
1,2;5,6-Di-O-isopropylidene-3-deoxy-3-{[(4-trifluoromethylphenyl)-amino]thiocarbonyl}-amino-α-D-allofuranoside (Compound No. 61);
1,2;5,6-Di-O-isopropylidene-3-deoxy-3- {[(4-methoxyphenyl)-amino]thiocarbonyl}-amino-α-D-allofuranoside (Compound No. 62);
1,2;5,6-Di-O-isopropylidene-3-deoxy-3-{[(4-benzyloxyphenyl)-amino]thiocarbonyl}-amino-α-D-allofuranoside (Compound No. 63);
1,2;5,6-Di-O-isopropylidene-3-deoxy-3-{[(3-nitrophenyl)-amino]thiocarbonyl}-amino-α-D-allofuranoside (Compound No. 64);
1,2;5,6-Di-O-isopropylidene-3-deoxy-3-{[(2-fluorophenyl)-amino]thiocarbonyl}-amino-α-D-allofuranoside (Compound No. 65);
1,2;5,6-Di-O-isopropylidene-3-deoxy-3-{[(2-trifluoromethylphenyl)-amino]thiocarbonyl}-amino-α-D-allofuranoside (Compound No. 66);
1,2;5,6-Di-O-isopropylidene-3-deoxy-3-{[(2-nitrophenyl)-amino]thiocarbonyl}-amino-α-D-allofuranoside (Compound No. 67);
1,2;5,6-Di-O-isopropylidene-3-deoxy-3-{[(4-cyanophenyl)-amino]thiocarbonyl}-amino-α-D-allofuranoside (Compound No. 68);
1,2;5,6-Di-O-isopropylidene-3-deoxy-3-{[(4-difluoromethoxyphenyl)-amino]thiocarbonyl}-amino-α-D-allofuranoside (Compound No. 69);
1,2;5,6-Di-O-isopropylidene-3-deoxy-3-{[(2-difluoromethoxyphenyl)-amino]thiocarbonyl}-amino-α-D-allofuranoside (Compound No. 70);
1,2;5,6-Di-O-isopropylidene-3-deoxy-3-{[(naphthyl)-amino]thiocarbonyl}-amino-α-D-allofuranoside (Compound No. 71);
1,2;5,6-Di-O-isopropylidene-3-deoxy-3- {[(4-thiomethylphenyl)-amino]thiocarbonyl}-amino-α-D-allofuranoside (Compound No. 72);
1,2;5,6-Di-O-isopropylidene-3-deoxy-3-{[(4-(4-propylcyclohexyl)phenyl)-amino]carbonyl}-amino-α-D-allofuranoside (Compound No. 88);
1,2;5,6-Di-O-isopropylidene-3-deoxy-3-{[(4-(4-hexylcyclohexyl)phenyl)-amino]carbonyl}-amino-α-D-allofuranoside (Compound No. 89);
1,2;5,6-Di-O-isopropylidene-3-deoxy-3-{[(pyridin-3-yl)-amino]thiocarbonyl}-amino-α-D-allofuranoside (Compound No. 90);
1,2;5,6-Di-O-isopropylidene-3-deoxy-3-{[(4-chlorophenyl)-amino]thiocarbonyl}-amino-α-D-allofuranoside (Compound No. 91);
1,2;5,6-Di-O-isopropylidene-3-deoxy-3-{[(2-chlorophenyl)-amino]thiocarbonyl}-amino-α-D-allofuranoside (Compound No. 92);
1,2;5,6-Di-O-isopropylidene-3-deoxy-3-{[(3-chlorophenyl)-amino]thiocarbonyl}-amino-α-D-allofuranoside (Compound No. 93);
1,2;5,6-Di-O-isopropylidene-3-deoxy-3-{[(2,6-dichlorophenyl)-amino]thiocarbonyl}-amino-α-D-allofuranoside (Compound No. 94);
1,2;5,6-Di-O-isopropylidene-3-deoxy-3- {[(2,6-dimethylphenyl)-amino]thiocarbonyl}-amino-α-D-allofuranoside (Compound No. 95);
1,2;5,6-Di-O-isopropylidene-3-deoxy-3-{[(2,5-difluorophenyl)-amino]thiocarbonyl}-amino-α-D-allofuranoside (Compound No. 96);
1,2;5,6-Di-O-isopropylidene-3-deoxy-3-{[(4-iodophenyl)-amino]thiocarbonyl}-amino-α-D-allofuranoside (Compound No. 97);
1,2;5,6-Di-O-isopropylidene-3-deoxy-3-{[(3-methoxyphenyl)-amino]thiocarbonyl}-amino-α-D-allofuranoside (Compound No. 98);
1,2;5,6-Di-O-isopropylidene-3-deoxy-3- {[(2-methoxyphenyl)-amino]thiocarbonyl}-amino-α-D-allofuranoside (Compound No. 99);
1,2;5,6-Di-O-isopropylidene-3-deoxy-3-{[(4-fluorophenyl)-amino]thiocarbonyl}-amino-α-D-glucofuranoside (Compound No. 101);
1,2;5,6-Di-O-isopropylidene-3-deoxy-3-{[(3-nitrophenyl)-amino]thiocarbonyl}-amino-α-D-glucofuranoside (Compound No. 102);
1,2;5,6-Di-O-isopropylidene-3-deoxy-3-{[(2-fluorophenyl)-amino]thiocarbonyl}-amino-α-D-glucofuranoside (Compound No. 103);
1,2;5,6-Di-O-isopropylidene-3-deoxy-3-{[(2-trifluoromethylphenyl)-amino]thiocarbonyl}-amino-α-D-glucofuranoside (Compound No. 104);
1,2;5,6-Di-O-isopropylidene-3-deoxy-3-{[(2,4-difluorophenyl)-amino]thiocarbonyl}-amino-α-D-glucofuranoside (Compound No. 105);
1,2;5,6-Di-O-isopropylidene-3-deoxy-3-{[(2-difluoromethoxyphenyl)-amino]thiocarbonyl}-amino-α-D-glucofuranoside (Compound No. 106); and
1,2;5,6-Di-O-isopropylidene-3-deoxy-3-{[(4-difluoromethoxyphenyl)-amino]thiocarbonyl}-amino-α-D-glucofuranoside (Compound No. 107).

### Example 2: Synthesis of 1,2;5,6-Di-O-isopropylidene-3-deoxy-3-{[(2-phenylethyl)-amino]thiocarbonyl}-amino-α-D-allofuranoside (Compound No. 49)

To a solution of the compound 1,2;5,6-Di-isopropylidene-3-deoxy-α-D-allofuranoside (100mg) in dichloromethane (5ml), was added triethylamine (0.06 ml) and 2-phenyl isocyanate (43mg) at room temperature. The reaction mixture was stirred for 4 hours at 55° C. Solvent was evaporated under reduced pressure. The crude compound thus obtained was purified with 50% ethyl acetate in hexane as eluent to furnish the title compound (100mg).
¹H NMR (CDCl₃) (300 MHz):δ 7.35-7.21(5H,m), 6.13(1H,bs), 5.92(1H,bs), 5.82(1H,d, 3.6Hz), 4.68(1H,t, 8.7Hz), 4.52(1H,bs), 4.27(1H,q, 10.5 Hz), 4.16(1H,t, 6.6Hz), 4.00-3.88(2H,m), 3.60-3.80(2H,m), 2.92(2H,t, 5.7Hz), 1.55(3H,s), 1.41(3H,s), 1.33(6H,s)

Analogues of 1,2;5,6-Di-O-isopropylidene-3-deoxy-3-{[(2-phenylethyl)-amino]-thio-carbonyl}-amino-α-D-allofuranoside (Compound No. 49) described below, can be prepared by replacing appropriate isothiocyanate group in place of 2-phenyl isocyanate, respectively, as applicable in each case.
1,2;5,6-Di-O-isopropylidene-3-deoxy-3-{[(4-fluorophenyl)-amino]-thio-carbonyl}-amino-α-D-allofuranoside (Compound No. 50).

### Scheme II

### Example 3: Synthesis of 1,2;5,6-Di-O-isopropylidene-3-deoxy-3-([{4-(2-methoxy-2-oxoethyl)-phenyl}-amino]-carbonyl)-amino-α-D-glucofuranoside (Compound No.4)

### Step a: Synthesis of 1,2;5,6-Di-O-isopropylidene-3-O-tosyl-a-D-glucofuranoside

To a solution of diacetoneglucose (8 g) (commercially available) in pyridine (20 ml) was added a solution of p-toluenesulphonyl chloride (4 g) in pyridine (20 ml) at 0°C. The reaction mixture was stirred for 8-10 hours. The solvent was evaporated under reduced pressure and the residue thus obtained was washed with hexane to obtain the title compound (8g).

### Step b: Synthesis of 1,2;5,6-Di-O-isopropylidene-3-deoxy-3-azido-α-D glucofuranoside

To a solution of a compound obtained from *step a* above (8 g) in dimethylformamide (50ml) was added sodium azide (1.5g). The reaction mixture was heated at 130°C for 10 hours, extracted with ethyl acetate followed by washing with water, brine and dried over anhydrous sodium sulphate. The solvent was evaporated under reduced pressure and the crude product was purified by column chromatography using 30% ethyl acetate in hexane as eluent to furnish the title compound (4 g).

### Step c: Synthesis of 1,2;5,6-Di-O-isopropylidene-3-deoxy-3-amino-α-D-glucofuranoside

To a solution of lithium aluminum hydride (1.1 g) in tetrahydrofuran (20ml) at 0°C, was added the compound obtained from step b (3.5 g) in tetrahydrofuran (10ml). The reaction mixture was stirred at this temperature for 15 minutes, and then was allowed to attain at room temperature and stirred for 10 hours. The reaction mixture was extracted with ethyl acetate followed by washing with aqueous sodium bicarbonate, water and brine and dried over anhydrous sodium sulphate. The solvent was evaporated under reduced pressure and the crude residue was purified by column chromatography using 30% ethyl acetate in hexane as an eluent to furnish the title compound (2 g).

### Step d: Synthesis of 1,2;5,6-D-O-isopropylidene-3-deoxy-3-([{4-(2-methoxy-2-oxoethyl)-phenyl}-amino]-carbonyl)-amino-α,D-glucofuranoside

To a solution of the compound (1 g) obtained from step c above in dichloromethane (10 ml) at 0°C was added methyl 4-isocyanatophenyl acetate (0.71 g). The reaction mixture was stirred for 15 minutes at the same temperature. The reaction mixture was allowed to attain room temperature and stirred for 24 hours. The solvent was evaporated under reduced pressure and the crude product was purified by column chromatography using 15% ethyl acetate in hexane as eluent furnish the title compound (1.3 g).
¹H NMR (CDCl₃) (300 MHz):δ 7.35(1H,s), 7.60-7.14(4H,m), 5.95(1H,d,7.5Hz), 5.81(1H,d, 3.3Hz), 4.60(1H,d,3.48Hz), 4.3-4.23(2H,m), 4.16-4.07(2H,m), 3.96(1H,m), 3.68(3H,s), 3.56(2H,s), 1.51(3H,s), 1.4(3H,s), 1.32(3H,s), 1.27(3H,s),

Analogues of 1,2; 5,6-Di-O-isopropylidene-3-deoxy-3-([{4-(2-methoxy-2-oxoethyl)-phenyl}-amino]-carbonyl)-amino-α-D-glucofuranoside (Compound No. 4) can be prepared by using appropriate isocyanate in place of methyl-4-isocyanatophenyl acetate.
(3R or 3S)-1,2;5,6-Di-O-isopropylidene-3-deoxy-3-[{(4-methyl-phenyl)-amino}-carbonyl]-amino-α-D-glucofuranoside (Compound No. 8);
(3R or 3S)-1,2;5,6-Di-O-isopropylidene-3-deoxy-3-[{(4-chloro-phenyl)-amino}-carbonyl]-amino-α-D-glucofuranoside (Compound No. 10);
1,2;5,6-Di-O-isopropylidene-3-deoxy-3-[phenyl-amino-carbonyl]-amino-α-D-glucofuranoside (Compound No. 12);
(3R or 3S)-1,2;5,6-Di-O-isopropylidene-3-deoxy-3-[{(4-nitro-phenyl)-amino}-carbonyl]-amino-α-D-glucofuranoside (Compound No. 14); and
1,2;5,6-Di-O-isopropylidene-3-deoxy-3-{[(4-fluoro-phenyl)-amino]-carbonyl}-amino-α-D-allofuranoside (Compound No. 51).

### Example 4: Synthesis of 1,2;5,6-Di-O-isopropylidene-3-deoxy-3-([{4-(2-hydroxy-2-oxoethyl)-phenyl}-aminol]-carbonyl)-amino-α-D-glucofuranoside (Compound No.6)

To a solution of the compound No. 4 (1 g, Example 2) in methanol (100 ml), was added sodium hydroxide (20 ml, IN). The reaction mixture was stirred for 6 hour at 50°C. The solvent was evaporated under reduced pressure. The aqueous layer was neutralized with dilute hydrochloric acid. The solid thus obtained was extracted with ethyl acetate followed by washing with water, brine and dried over anhydrous sodium sulphate. The solvent was evaporated under reduced pressure and the crude compound was purified by column chromatography using 10% ethyl acetate in hexane as eluent to furnish the title compound (500mg).
¹H NMR (CDCl₃) (300 MHz):δ 7.57(1H,s), 7.17(3H,s), 6.08(1H,bs), 5.85(1H,d, 3.3Hz), 4.63(1H,d, 3.24Hz), 4.24(2H,m), 4.14(3H,m), 4.10(1H,m), 3.56(2H,s), 1.51(3H,s), 1.40(3H,s), 1.33(3H,s), 1.29(3H,s).

### Scheme III

### Example 5: Synthesis of 1,2;5,6-Di-O-isopropylidene-3-O-[(4-fluoro-phenyl)-amino]-carbonyll-α-D-glucofuranoside (Compound No. 48)

To a solution of diacetoneglucose (260mg) in dichloromethane (10ml) at 0°C, was added 4-fluorophenyl isocyanate (105mg) and triethylamine (40mg) and then stirred at room temperature for 3-4hours, followed by refluxing for 3 hours. The volatiles were evaporated under reduced pressure and the crude residue was purified by column chromatography using 20% ethyl acetate in hexane as eluent to furnish the title compound (170mg).
¹H NMR (CDCl₃) (300mHz): δ 7.35(2H,bs), 7.02(2H,t, 8.4Hz), 6.65(1H,NH), 5.89(1H,d, 3.3Hz), 5.25(1H,s), 4.65(1H,d, 3.6Hz), 4.20-4.25(2H,m), 4.03-4.12(2H,m), 1.53(3H,s), 1.43(3H,s), 1.33(3H,s) and 1.32(3H,s).

Analogues of 1,2; 5,6-Di-O-isopropylidene-3-O-[(4-fluoro-phenyl)-amino]-carbonyl]-α-D-allofuranoside (Compound No. 13) can be prepared by using appropriate isocyanate in place of 4-fluoro-phenyl isocyanate respectively, as applicable in each case.
1,2;5,6-Di-O-isopropylidene-3-O-[(4-methyl-phenyl)-amino]-carbonyl]-α-D-glucofuranoside (Compound No. 13);
1,2;5,6-Di-O-isopropylidene-3-O-[(4-methoxy-phenyl)-amino]-carbonyl-α-D-glucofuranoside (Compound No. 15); and
1,2;5,6-Di-O-isopropylidene-3-O-[(4-chloro-phenyl)-amino]-carbonyl-α-D-glucofuranoside (Compound No. 16).

### Scheme IV

### Example 6: Synthesis of 1,2,5,6-Di-O-isopropylidene-3-deoxy-3-[2-{3-(4-chloro-phenyl)-ureido}-ethyl]-α-D-allofuranoside (Compound No. 2)

To a solution of the compound 1,2,5,6-di-O-isopropylidene-3-deoxy-3-ethylamino-α-D-allofuranoside (200 mg) in dichloromethane (20 ml) at 0-5°C, was added a solution ofp-chlorophenyl isocyanate (0.12 g) in dichloromethane (10 ml) with constant stirring and the reaction mixture was stirred for 15 minutes. The reaction mixture was allowed to attain room temperature and stirred for 8 hours. The solvent was evaporated under reduced pressure and the crude product was purified by column chromatography using 50% ethyl acetate as eluent to furnish the title compound (150 mg).
¹H NMR (CDCl₃) (300 MHz):δ 7.74(1H,s), 7.35(2H,m), 7.20(2H,m), 7.35(1H,d, 3Hz), 7.20(2H,m), 5.73(1H,d, 3Hz), 5.59(1H,d, 4.8Hz), 4.72(1H,t, 3.93Hz), 4.07-3.92(3H,m), 3.75(1H,t, 7.4Hz), 3.42-3.3(2H,m), 1.90(3H,m), 1.49(3H,s), 1.4(3H,s), 1.33(3H,s), &1.30(3H,s).

Analogues of 1,2;5,6-Di-O-isopropylidene-3-deoxy-3-[2- {3-(4-chloro-phenyl)-ureido}-ethyl]-α-D-allofuranoside (Compound No. 2) can be prepared by using appropriate isocyanate in place of p-chlorophenyl isocyanate, respectively, as applicable in each case.
1,2;5,6-Di-O-isopropylidene-3-deoxy-3-[2-{3-(4-methyl-phenyl)-ureido}-ethyl]-α-D-allofuranoside (Compound No. 17); and
1,2;5,6-Di-O-isopropylidene-3-deoxy-3-{2-[3-(4-methoxy-phenyl)-ureido]-ethyl}-α-D-allofuranoside (Compound No. 18).

### Scheme V

### Example 7: Synthesis of 2,3;5,6-Di-O-isopropylidene-1-O-{2-(1-piperazinyl)-ethyl}-α-D-mannofuranoside (Compound No. 36)

### Step a: Synthesis of 1-O-(2-chloroethyl)-2,3;5,6-di-O-isopropylidene-α-D-mannofuranoside

A suspension of mannose (500 mg) in anhydrous acetone (20ml) was cooled to 0°C followed by the addition of sulphuric acid (0.05 ml) and chloroethanol (558 mg). The reaction mixture was refluxed till the reaction showed completion (TLC). The reaction mixture was neutralized with triethylamine and excess of acetone was evaporated under reduced pressure. The residue thus obtained was dissolved in ethyl acetate followed by washing with aqueous sodium bicarbonate and dried over anhydrous sodium sulphate. The solvent was evaporated under reduced pressure and the crude product was purified by column chromatography using 10% ethyl acetate as eluent to furnish the title compound (200mg).

### Step b: Synthesis of 2,3;5,6-Di-O-isopropylidene-1-0-{2-(1-piperazinyl)-ethyl}-α-D-mannofuranoside (Compound No. 36)

To the compound obtained from the above *step a* (100 mg) was added a solution of potassium iodide (52mg) in dimethylformamide (5ml). The reaction mixture was stirred at 80°C for one hour followed by the addition of piperazine (133mg) and potassium carbonate (86mg). After completion of reaction (TLC) the reaction mixture was poured in cold water and extracted with ethyl acetate .The organic layer was dried over anhydrous sodium sulphate, filtered and evaporated under reduced pressure. The crude residue was purified by column chromatography using 10% methanol in ethyl acetate as eluent to furnish the title compound (60 mg).
¹H NMR (CDCl₃, 300MHz):δ 4.99(1H, s), 4.78-4.75(1H, m), 4.60(1H, m), 4.39(1H, m), 4.09-4.03(2H, m), 4.95-4.92(1H, m), 3.74(1H, m), 3.55-3.53(1H, m), 2.91(3H, t, 4.89Hz), 2.56(2H, t, 5.8Hz), 2.26(6H, bs), 1.45(6H, s), 1.37(3H, s), 1.31(3H, s).

Analogues of 2,3;5,6-Di-O-isopropylidene-1-O-{2-(1-piperazinyl)-ethyl}-α-D-mannofuranoside (Compound No. 36) can be prepared by replacing appropriate amine in place of piperazine and appropriate sugar moiety respectively, as applicable in each case.
2,3;5,6-Di-O-isopropylidene-1-O-{2-[1-(4-[4-methoxy-phenyl]-piperazinyl)]-ethyl}-α-D-mannofuranoside (Compound No. 31);
2,3;5,6-Di-O-isopropylidene-1-O-{2-[1-(4-[2-pyrimidinyl]-piperazinyl)]-ethyl}-α-D-mannofuranoside (Compound No. 32) ;
2,3;5,6-Di-O-isopropylidene-1-O-{2-[1-(4-benzyl-piperazinyl)]-ethyl}-α-D-mannofuranoside (Compound No. 34);
2,3;5,6-Di-O-isopropylidene-1-O-{2-[4-morpholinyl]-ethyl}-α-D-mannofuranoside (Compound No. 33);
2,3;5,6-Di-O-isopropylidene-1-O-{2-[1-(4-[{4-chloro-phenyl}-aminocarbonyl]-piperazinyl)]-ethyl}-α-D-mannofuranoside (Compound No. 35);
2,3;5,6-Di-O-isopropylidene-1-O-{3-[1-(4-[3-chloro-phenyl]-piperazinyl)]-propyl}-α-D-mannofuranoside (Compound No. 29); and
2,3;5,6-Di-O-isopropylidene-1-O-{2-[1-(4-[4-chloro-phenyl]-piperazinyl)]-ethyl}-α-D-mannofuranoside (Compound No. 30).

### Scheme VI, (path a)

### Example 8: Synthesis of 2,3;5,6-Di-O-isopropylidene-1-O-{2-[1-(4-[2-thienyl-methyl-carbonyl]-piperazinl)]-ethyl}-α-D-mannofuranoside (Compound No. 42)

To a solution of the compound No. 36 (200 mg) in dichloromethane (10ml) cooled at 0°C, was added triethylamine (82mg) and chloroacetylthiophene (104 mg) slowly. The reaction mixture was stirred till the reaction showed completion (TLC). The reaction mixture was poured into ice-cold water and the product was extracted with dichloromethane. The organic layer was dried over anhydrous sodium sulphate and the solvent was evaporated under reduced pressure. The crude product was purified by column chromatography using 90% ethyl acetate in hexane as eluent to furnish the title compound (90 mg).
¹H NMR (CDCl₃) (300 MHz):δ 7.21(1H,d 4.5Hz), 6.96(1H,dd, 4.5Hz), 6.91 (1H,d, 3Hz), 4.99(1H,s), 4.77(1H,t, 4Hz), 4.60(1H,d, 5.8Hz), 4.4 (1H,m), 4.11-4.03(2H,m), 3.95(3H,m), 3.73(1H,m), 3.67(2H,bs), 3.53(3H,bs), 2.57(2H,bs), 2.47-2.39(4H,m), 1.48(3H,s), 1.46(3H,s), 1.39(3H,s), 1.33(3H,s).

Analogues of 2,3,5,6-Di-O-isopropylidene-1-O-{2-[1-(4-[2-thienyl-methyl-carbonyl]-piperazinyl)]-ethyl}-α-D-mannofuranoside (Compound No. 42) can be prepared by using appropriate acyl halide group in place of chloroacetylthiophene, respectively, as applicable in each case.
2,3;5,6-Di-O-isopropylidene-1-O-{2-[1-(4-[4-fluoro-phenyl-carbonyl]-piperazinyl)]-ethyl}-α-D-mannofuranoside (Compound No. 43).

### Example 9: Synthesis of 2,3;5,6-Di-O-isopropylidene-1-O-{2-[1-(4-{4-methyl-phenylsulphonyl}-piperazinyl)]-ethyl}-α-D-mannofuranoside (Compound No. 40)

To a solution of the Compound No.36 (200 mg) in pyridine (5ml), was added p-toluenesulphonyl chloride (122 mg) the reaction mixture was stirred for 2 hours. The reaction mixture was poured into cold water and was extracted with ethyl acetate followed by washing with water, brine and dried over anhydrous sodium sulphate. The solvent was evaporated under reduced pressure and the crude product was purified by column chromatography using 50% ethyl acetate in hexane as eluent to furnish the title compound (130 mg).
¹H NMR (CDCl₃) (300 MHz):δ 7.64(2H,d, 8Hz), 7.32(2H,d), 4.94(1H,s), 4.74 (1H, 3.4Hz), 4.56(1H,d, 5.9Hz), 4.38(1H,m), 4.06(1H,m), 3.99 (1H,m), 3.89(1H,m), 3.87(1H,m), 3.69(1H,m), 3.48(1H,m), 3.02(4H, s), 2.55(6H,s), 2.43(3H,s), 1.44(3H,s), 1.42(3H,s), 1.3(3H,s), 1.26(3H,s).

### Scheme VI, (path b)

### Example 10: Synthesis of 2,3;5,6-Di-O-isopropylidene-1-O-{2-[1-(4-{(1-naphthyl)-amino-carbonyl}-piperazinyl)]-ethyl}-α-D-mannofuranoside (Compound No. 39)

To a solution of the Compound No. 36 (200 mg) in acetonitrile (5ml) was added naphthyl isocyanate (109mg). The reaction mixture was stirred at room temperature till the reaction shown completion (TLC). The solvent was evaporated under reduced pressure. The crude product was purified by column chromatography using 90% ethyl acetate in hexane as eluent to furnish the title compound (100 mg).
¹H NMR (CDCl₃) (300 MHz):δ 7.88(2H,m), 7.68(2H,m), 7.54-7.47(3H,m), 6.67(1H,s), 5.04(1H,s), 4.80(1H,t, 2.31Hz), 4.64(1H,d, 5.8Hz), 4.40(1H,m), 4.13-4.08(2H,m) 4.00(1H,m), 3.80(1H,m), 3.60(5H,t), 2.66(2H,m), 2.57(4H,t), 1.49(3H,s), 1.48(3H,s), 1.40(3H,s), 1.35(3H,s).

### Example 11: Synthesis of 2,3;5,6-Di-O-isopropylidene-1-O-{2-[1-(4-[isopropylaminothiocarbonyl]-piperazinyl)]-ethyl}-α-D-mannofuranoside(Compound No. 38)

To a solution of the Compound No.36 (200 mg) in acetonitrile (5ml) was added p-chlorophenyl isothiocyanate (65mg). The reaction mixture was stirred at room temperature till the reaction showed completion (TLC). The solvent was evaporated under reduced pressure. The crude product was purified by column chromatography using 50% ethyl acetate in hexane as eluent to furnish the title compound (140 mg).
¹H NMR (CDCl₃) (300 MHz):δ 5.23-5.20(1H,bs), 4.99(1H,s), 4.78-4.75(1H,m), 4.60-4.59(2H,m), 4.41-4.39(1H,m), 4.09-4.03(2H,m), 3.95-3.92(1H,m), 3.80-3.74(5H,m), 3.57-3.55(1H,m) ,2.61-2.51(6H,m), 1.46(6H,s), 1.37(3H,s), 1.32(3H,s), 1.25(3H,s), 1.24(3H,s).

### Scheme VI, (path c)

### Example 12: Synthesis of 2,3;5,6-Di-O-isopropylidene-1-O-{2-[1-(4-{[3,3']-bithiophenyl-5-yl- methyl}-piperazinyl)]-ethyl}-α-D-mannofuranoside (Compound No. 37)

To a solution of 5-chloromethyl-[3,3']-bithiophenyl (200 mg) in dimethylformamide (5ml) and potassium carbonate (111mg) was slowly added the Compound No. 36 (138mg). The reaction mixture was stirred for 3 hours at room temperature and then the reaction mixture was poured into ice-cold water and the product was extracted with ethyl acetate. The organic layer was dried over anhydrous sodium sulphate and evaporated under reduced pressure. The crude product was purified by column chromatography to furnish the title compound (90 mg).
¹H NMR (CDCl₃) (300 MHz): δ 7.34-7.26(4H,m), 7.15(1H,s), 4.99(1H,s), 4.76(1H,t. 5.61Hz), 4.60(1H,d, 5.8Hz), 4.40 (1H,m), 4.10-3.92 (3H,m), 3.78-3.72 (3H,m), 3.56 (1H,m), 2.58 (10H,bs), 2.04 (1H, s), 1.46(3H,s), 1.45 (3H,s), 1.37 (3H,s), 1.32 (3H,s).

Analogues of 2,3;5,6-Di-O-isopropylidene-1-O-{2-[1-(4-{[3,3']-bithiophenyl-5-yl-methyl}-piperazinyl)]-ethyl}-α-D-mannofuranoside (Compound No. 37) can be prepared by replacing appropriate alkyl halide group in place of 5-chloromethyl-[3, 3¹]-bithiophenyl, respectively, as applicable in each case.
2,3;5,6-Di-O-isopropylidene-1-O-{2-[1-(4-[(2-chloro-3,4-methylenedioxyphenyl)-methyl]-piperazinyl)]-ethyl}-α-D-mannofuranoside (Compound No. 45).

### Scheme VII, (path a)

### Example 13: Synthesis of 2,3;5,6-Di-O-isopropylidene-1-O-{2-[1-(4-{2-(1-[1H-1,2,4-triazolyl])-acetyl}-piperazinyl)]-ethyl}-α-D-mannofuranoside (Compound No. 44)

### Step a: Synthesis of 2,3 ;5,6- Di-O-isopropylidene-1-O-{2-(1-[4-chloroacetyl]-piperazinyl)-ethyl}-α-D-mannofuranoside

To a solution of the Compound No. 36 (2 g) in dichloromethane (35 ml) cooled at 0°C, was added triethylamine (1.13 gm) and chloroacetyl chloride (0.51 ml). The reaction mixture was poured into ice-cold water and the product was extracted with dichloromethane. The solvent was evaporated under reduced pressure. The crude product was purified by column chromatography using 50% ethyl acetate in hexane as eluent to furnish the title compound (1.6 g).

### Step b: Synthesis of 2,3,5,6-Di-O-isopropylidene-1-O-{2-[1-(4-{2-(1-[1H-1,2,4-triazolyl])acetyl}-piperazinyl]-ethyl}-α-D-mannofuranoside

To a solution of the compound obtained from step *a* above (200 mg) in dimethylformamide (5ml), was added 1*H*-[1,2,4]-triazole (37 mg) and cesium carbonate (145mg). The reaction mixture was stirred at room temperature till the reaction showed completion (TLC). The reaction mixture was poured into ice-cold water and extracted with ethyl acetate. The organic layer was dried over anhydrous sodium sulphate and filtered. The solvent was evaporated under reduced pressure. The crude compound was purified by column chromatography using 90% ethyl acetate in hexane as eluent to furnish the title compound (12mg).
¹H NMR (CDCl₃) (300 MHz):δ 8.26(1H,s), 7.98(1H,s), 5.06(2H,s), 5.01(1H,s), 4.79(1H,t, 3.2Hz), 4.62(1H,d,5.9Hz), 4.40(1H,m), 4.12-4.06(2H,m), 3.97(1H,m), 3.80(1H,m), 3.68(2H,bs), 3.57(3H,bs), 2.64-2.60(2H,m), 2.56-2.50(4H,m), 1.49(3H,s), 1.48(3H,s), 1.48(3H,s), 1.40(3H,s), 1.34(3H,s).

Analogues of 2,3,5,6-Di-O-isopropylidene-1-O-{[1-(4-{2-(1-[1H-1,2,4-triazolyl])-acetyl}-piperazinyl]-ethyl}-α-D-mannofuranoside (Compound No. 44) can be prepared by replacing appropriate amine group in place of 1*H*-[1,2,4]-triazole, respectively, as applicable in each case.
2,3;5,6-Di-O-isopropylidene-1-O- {2-[1-(4-[2-(2,6-dioxo-1-piperidinyl)-acetyl]-piperazinyl)]-ethyl}-α-D-mannofuranoside (Compound No. 41).

### Scheme VII, (path b)

### Example 14: Synthesis of 2,3;5,6-Di-O-isopropylidene-1-O-{2-[1-(4-{2-[4-chlorophenoxy]-acetyl}-piperazinyl)]-ethyl}-α-D-mannofuranoside (Compound No. 46)

To a solution of a compound obtained from step *a* of Example 12 above (200 mg) in dimethylformamide (5 ml), was added cesium carbonate (145 mg) and 4-chlorophenol (69mg). The reaction mixture was stirred at 60°C till the reaction completion (TLC). The solvent was evaporated under reduced pressure and the crude product was purified by column chromatography using 50% ethyl acetate in hexane as eluent to furnish the title compound (160 mg).
¹H NMR (CDCl₃) (300 MHz):δ 7.27(2H,d, 9Hz), 6.90(2H,d, 9Hz), 5.01(1H,s), 4.78(1H,m), 4.68(2H,s), 4.61(1H,d, 5.8Hz), 4.43 (1H,m), 4.12-4.08(2H,m) 3.97(1H,m), 3.75-3.65(1H,m), 3.59-3.54(5H,m), 2.59(2H,t, 5.8Hz), 2.48(4H,bs), 1.49(3H,s), 1.47(3H,s), 1.40(3H,s), 1.34(3H,s).

### Scheme VIII

### Example 15: Synthesis of 1,2-O-isopropylidene-3-deoxy-3-{2-[3-(4-{2-methoxy-2-oxoethyl}-phenyl)-ureido]-ethyl}-α-D-allofuranoside (Compound No. 26)

To a solution of the compound No. 4 (prepared following Scheme II) (4.0 g) in tetrahydrofuran (4 ml) was added aqueous perchloric acid (30%, 4.0 ml) at 0°C and stirred the reaction mixture at this temperature for 6 hours. The solvent was evaporated under reduced pressure and the crude compound was purified by column chromatography using 70% ethyl acetate in hexane as eluent to furnish the title compound (2.0 g).
¹H NMR (CDCl₃, 300 MHz):δ 7.27(2H,d, 7.59Hz), 7.17 (3H,d, 7.62Hz), 5.71(1H, 3.12Hz), 5.44(1H,s), 4.62(1H,d, 3.4Hz), 3.72(8H,m), 3.57(2H,s), 3.36(3H,m), 1.99(1H, m), 1.78(2H,bs), 1.45(3H,s), 1.26(3H,s).

Analogues of 1,2-O-Isopropylidene-3-deoxy-3-{2-[3-(4-{2-methoxy-2-oxoethyl}-ureido]-ethyl}-α-O-allofuranoside (Compound No. 26) can be prepared by using appropriate sugar derivative in place of compound No. 4, respectively as applicable in each case.
1,2-O-Isopropylidene-3-O-[(4-methoxy-phenyl)-amino]-carbonyl-α-D-allofuranoside (Compound No. 19);
1,2-O-Isopropylidene-3-deoxy-3-{2-[3-(4-methoxy-phenyl)-ureido]-ethyl}-α-D-glucofuranoside (Compound No. 20);
1,2-O-Isopropylidene-3-O-{(4-chloro-phenyl)-amino}-carbonyl-α-D-glucofuranoside (Compound No. 21);
1,2-O-Isopropylidene-3-deoxy-3-{[(4-nitro-phenyl)-amino]-carbonyl}-amino-α-D-allofuranoside (Compound No. 22);
1,2-O-Isopropylidene-3-deoxy-3-{[(4-chloro-phenyl)-amino]-carbonyl}-amino-α-D-allofuranoside (Compound No. 23);
1,2-O-Isopropylidene-3-O-{(4-methyl-phenyl)-amino}-carbonyl-α-D-glucofuranoside (Compound No. 24);
1,2-O-Isopropylidene-3-deoxy-3-[2-{3-(4-methyl-phenyl)-ureido}-ethyl]-α-D-allofuranoside (Compound No. 25);
1,2-O-Isopropylidene-3-deoxy-3-{[(4- methyl-phenyl)-amino]-carbonyl}-amino-α-D-allofuranoside (Compound No. 27); and
1,2-O-Isopropylidene-3-deoxy-3-{2-[3-(4-{2-hydroxy-2-oxo-ethyl}-phenyl)-ureido]-ethyl}-α-D-allofuranoside (Compound No. 28).

### Scheme IX, (path a)

### Example 16: Synthesis of 1,2;5,6-Di-O-isopropylidene-3-deoxy-3-{[4-fluoro-phenyl]-sulphoeyl}-amino-α -D -allofuranoside (Compound No. 52)

To a solution of 1,2;5,6-Di-O-isopropylidene-3-deoxy-3-amino-α-D-allofuranoside (100mg) in dichloromethane (5ml), was added 4-fluorobenzenesulphonyl chloride (75mg) at 0°C and stirred the reaction mixture for 2 hours. Solvent was evaporated under reduced pressure. The reaction mixture was taken into water, extracted with ethyl acetate, and the combined organic extracts were washed with brine and dried over anhydrous sodium sulphate. Solvent was evaporated under reduced pressure and the residue thus obtained was purified with 40% ethyl acetate in hexane as eluent to furnish the title compound (77mg).
¹H NMR (CDCl₃, 300 MHz):δ 7.92-7.97(2H,m), 7.23-7.18(3 H, m), 5.72(1 H, d, 3Hz), 5.15(1 H, d, 9Hz), 4.23-4.17(2 H, m), 3.98-3.95(2 H, m), 3.84-3.88(1 H, m), 3.56-3.20(1 H, m), 1.51(3H,s), 1.42(3 H, s), 1.32(3 H, s), 1.25(3 H, s).

Analogues of 1,2;5,6-Di-O-isopropylidene-3-deoxy-3- {[4-fluoro-phenyl]-sulphonyl}-amino-α-D-allofuranoside (compound no. 52) described below can be prepared by replacing appropriate sulphonyl group in place of 4-fluoro-benzenesulphonyl chloride , respectively, as applicable in each case.
1,2;5,6-Di-O-isopropylidene-3-deoxy-3-{[4-methyl-phenyl]-sulphonyl}-amino-α-D-allofuranoside (Compound No. 53).

### Scheme IX, (path b)

### Example 17: Synthesis of 1,2;5,6-Di-O-isopropylidene-3-deoxy-3-{[(4-methoxy-2-oxoethyl)aminol-carboEyl}-methyl amino-α-D-allofuranoside (Compound No. 54)

To a solution of 1,2;5,6-Di-O-isopropylidene-3-deoxy-3-amino-α-D-allofuranoside (100mg) in dry acetone (10ml), at room temperature, was added potassium carbonate (172mg) followed by the addition of 4-(2-chloro-acetyl amino)-phenyl acetic acid methyl ester (100mg) after 5-10 minutes. The reaction mixture was stirred for 3 hours at room temperature followed by refluxing for overnight. Solvent was evaporated under reduced pressure .The reaction mixture was taken into water and extracted with ethyl acetate. The combined organic layer was washed with water, brine and dried over anhydrous sodium sulphate. The solvent was evaporated under reduced pressure and the residue thus obtained was purified by column chromatography using 60% ethyl acetate in hexane to furnish the title compound (46 mg).
¹H (CDC1₃, 300MHz): δ 9.61(1H,s), 7.61(2H,d, 9Hz), 7.26-7.22(4H,m), 5.77(1H,s), 4.64(1H,d, 9Hz), 4.24-2.84(12H,m), 1.57(3 H, s), 1.46(3 H, s), 1.25(6H,s).

### Scheme X:

### Example 18: Synthesis of 1,2;5,6-Di-O-isopropylidene-3-deoxy-3-{[(2-trifluoromethylphenyl)-methyl]carbonyl}-amino-α-D-allofuranoside (Compound No. 80)

N-methylmorpholine (0.200 g, 0.772 mmol) was added to a mixture of 1,2;5,6-Di-O-isopropylidene-3-deoxy-3-amino-α-D-allofuranoside (0.200 g, 0.772 mmol), 2-trifluoromethylphenylacetic acid (0.189 g, 0.926 mmol), N-hydroxybenzotriazole (0.208 g, 1.544 mmol) in dry dimethylformamide). The reaction mixture was stirred at OC for 1 hour followed by the addition of 1-(3-dimethylaminopropyl)-3-ethylcarbodiimide hydrochloride (0.296 g, 1.544 mmol) at the same temperature. The reaction mixture was stirred at room temperature for 10 hours. The mixture was poured into water, extracted with ethyl acetate, washed with water and brine, dried over anhydrous sodium sulphate and concentrated under reduced pressure. The residue thus obtained was purified by column chromatography using 50% ethyl acetate in hexane solvent mixture as an eluent to furnish the title compound. Yield: 0.130g.
¹H NMR (CDCl₃, 400 MHz):δ 7.28-7.24 (m, 2H, Ar-H), 6.95-6.90 (m, 2H, Ar-H), 5.98-5.83 (brm, 1H, -NH), 5.84 (d, 1H, J = 3.00Hz, -CH), 4.62 (m, 1H, -CH), 4.20-4.05 (m, 3H, 3x-CH), 3.93-3.82 (m, 2H, 2x-CH), 3.65 (s, 2H, -CH₂Ph) and 1.48-1.32 (m, 12H, 4x-CH₃)

Analogues of 1,2;5,6-Di-O-isopropylidene-3-deoxy-3-{[(2-trinuoromethylphenyl)-methyl]carbonyl}-amino-α-D-allofuranoside (Compound No. 80) can be prepared by reacting an appropriate acid with amine respectively, as applicable in each case.
1,2;5,6-Di-O-isopropylidene-3-deoxy-3-{[(3,5-difluorophenyl)-methyl]carbonyl}-amino-α-D-allofuranoside (Compound No. 57);
1,2;5,6-Di-O-isopropylidene-3-deoxy-3-{[(2,4-difluorophenyl)-methyl]carbonyl}-amino-α-D-allofuranoside (Compound No. 73);
1,2;5,6-Di-O-isopropylidene-3-deoxy-3-{[(3-methoxyphenyl)-methyl]carbonyl}-amino-α-D-allofuranoside (Compound No. 74);
1,2;5,6-Di-O-isopropylidene-3-deoxy-3-{[(2-methoxyphenyl)-methyl]carbonyl}-amino-α-D-allofuranoside (Compound No. 75);
1,2;5,6-Di-O-isopropylidene-3-deoxy-3-{[(2-chloro-4-fluorophenyl)-methyl]carbonyl}-amino-α-D-allofuranoside (Compound No. 76);
1,2;5,6-Di-O-isopropylidene-3-deoxy-3-{[(4-methylphenyl)-methyl]carbonyl}-amino-α-D-allofuranoside (Compound No. 77);
1,2;5,6-Di-O-isopropylidene-3-deoxy-3-{[(4-nitrophenyl)-methyl]carbonyl}-amino-α-D-allofuranoside (Compound No. 78);
1,2;5,6-Di-O-isopropylidene-3-deoxy-3-{[(2-nitrophenyl)-methyl]carbonyl}-amino-α-D-allofuranoside (Compound No. 79);
1,2;5,6-Di-O-isopropylidene-3-deoxy-3-{[(2-trifluoromethoxyphenyl)-methyl]carbonyl}-amino-α-D-allofuranoside (Compound No. 81);
1,2;5,6-Di-O-isopropylidene-3-deoxy-3-{[(3-trifluoromethoxyphenyl)-methyl]carbonyl}-amino-α-D-allofuranoside (Compound No. 82);
1,2;5,6-Di-O-isopropylidene-3-deoxy-3-{[(4-isopropylphenyl)-methyl]carbonyl}-amino-α-D-allofuranoside (Compound No. 83);
1,2;5,6-Di-O-isopropylidene-3-deoxy-3-{[(3-chlorophenyl)-methyl]carbonyl}-amino-α-D-allofuranoside (Compound No. 84);
1,2;5,6-Di-O-isopropylidene-3-deoxy-3-{[(3-methylphenyl)-methyl]carbonyl}-amino-α-D-allofuranoside (Compound No. 85);
1,2;5,6-Di-O-isopropylidene-3-deoxy-3-{[(2,4-dichlorophenyl)-methyl]carbonyl}-amino-α-D-allofuranoside (Compound No. 86);
1,2;5,6-Di-O-isopropylidene-3-deoxy-3-{[(3-chlorophenyl)-methyl]carbonyl}-amino-α-D-allofuranoside (Compound No. 87);
1,2;5,6-Di-O-isopropylidene-3-deoxy-3-{[(3,4-dichlorophenyl)-methyl]carbonyl}-amino-α-D-allofuranoside (Compound No. 100);
1,2;5,6-Di-O-isopropylidene-3-deoxy-3-{[(4-(phenyl)phenyl)-methyl]carbonyl}-amino-α-D-allofuranoside (Compound No. 108);
1,2;5,6-Di-O-isopropylidene-3-deoxy-3-{[(2-naphthyl)-methyl]carbonyl}-amino-α-D-allofuranoside (Compound No. 109);
1,2;5,6-Di-O-isopropylidene-3-deoxy-3-{[(2-fluorophenyl)-methyl]carbonyl}-amino-α-D-allofuranoside (Compound No. 110);
1,2;5,6-Di-O-isopropylidene-3-deoxy-3-{[(2-chloro-6-nuorophenyl)-methyl]carbonyl}-amino-α-D-allofuranoside (Compound No. 111);
1,2;5,6-Di-O-isopropylidene-3-deoxy-3- {[(2,5-difluorophenyl)-methyl]carbonyl}-amino-α-D-allofuranoside (Compound No. 112);
1,2;5,6-Di-O-isopropylidene-3-deoxy-3-{[(4-fluorophenyl)-methyl]carbonyl}-amino-α-D-allofuranoside (Compound No. 113);
1,2;5,6-Di-O-isopropylidene-3-deoxy-3-{[(3-fluorophenyl)-methyl]carbonyl}-amino-α-D-allofuranoside (Compound No. 114);
1,2;5,6-Di-O-isopropylidene-3-deoxy-3-{[(3-bromophenyl)-methyl]carbonyl}-amino-α-D-allofuranoside (Compound No. 115); and
1,2;5,6-Di-O-isopropylidene-3-deoxy-3-{[(3,4-difluorophenyl)-methyl]carbonyl}-amino-α-D-allofuranoside (Compound No. 116).

### Pharmacological activity

The compounds of the present invention are tested in one or both of the assays described herein. Standard assays are used to evaluate activity of compounds in present invention on inflammatory cells. Attenuation of agonist-induced release of lipid mediator of neutrophil chemotaxis, leukotriene B4 (LTB4), is used to evaluate inhibitory effect on neutrophils.

### A23187 induced LTB₄ release

Venous blood was collected from healthy human donors using heparin as an anticoagulant. Neutrophils were isolated from freshly drawn blood after dextran sedimentation and ficoll separation *(*Eur J Biochem. 169, 175, 1987). 180 µl of the of neutrophil suspension (0.2x10⁶ cells/ml) was taken and added 19µL of Hank's Buffer salt solution along with 1µL of the test drug (200 times concentrated) in a 24 well plate and incubated at 37°C for 1hour. 3 minutes before the end of test compound incubation, 0.25 mM Ca⁺⁺/Mg⁺⁺were added. Then, 0.3 µg/ml A23187 (Sigma Chem, USA) was added and incubated for further 10 min at 37°C. The reaction was stopped by adding 80 µL of cold methanol and centrifuged to remove cell debris (J Pharmacol Exp Ther. 297:267, 2001). The samples were analysed for LTB₄ release using LTB₄ ELISA kits (Assay Design Inc., USA). The amount of LTB₄ released was quantified and percent inhibition of LTB₄ release was calculated with respect to the difference between the A23187 stimulated and negative control cells, to compute IC₅₀ values.

For compounds, tested (49, 50, 55, 58, 71, 78, 80-84, 91, 92, 100, 101, 103 and 105), the A123187 induced LTB4 release showed
IC 50 of about 0.13 µM to about 30 µM, for example from about 0.13µM to about 1.6 µM, or from about 0.13 µM to about 0.6 µM.

### Assay for 5-Lipoxygenase Activity

In a 96 well UV-plate, 100 µl of phosphate buffer saline (PBS) containing DTT (200 µM), ATP (100 µM) and calcium chloride (100 µM) was added. To each well 0.5 µl of test drug (200 times concentrated) or vehicle was added, followed by 4 µl of recombinant 5-Lox (3 units/µl) and was incubated at 37°C for 5 min. The reaction was initiated by adding 1 µl of 1mM freshly prepared arachidonic acid and increase in absorbance was monitored at 236 nm for 10 min. *(*J Biol. Chem. 261:11512, 1986) A plot of absorbance verses time curve was prepared and area under curve (AUC) was computed for each well. Percent inhibition of AUC for different treatments was calculated with respect to the difference between the Arachidonic acid stimulated and negative control values, to compute IC₅₀ values.

For compounds tested (55, 60, 63, 67, 68, 80, 84, 100, 101, and 103) the 5-1; poxygenase inhibition showed IC₅₀ of from about 0.3 µM to about 10 µM, or from about 0.3 µM to about 5 µM, or from about 0.3 µM to about 1.5 µM.

## Claims

1. The compound of Formula I 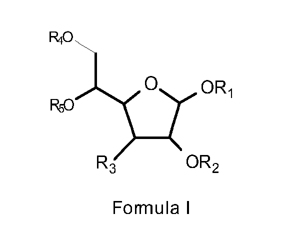 wherein
R₁ and R₂ together form a five-membered acetal, wherein the carbon atom joining the oxygens can be substituted with R_{L} and Rₘ [wherein R_{L} and Rₘ are independently selected from hydrogen, alkyl, alkenyl, alkynyl, cycloalkyl, aryl, or aralkyl; or R_{L} and Rₘ can together join to form a 3-8 membered ring, wherein the ring may optionally contain one or more heteroatoms selected from O, N or S, and the ring may be optionally substituted with one or more of alkyl, alkenyl, alkynyl, amino, substituted amino, cycloalkyl, oxo, hydroxy, carboxy, -COQR₆ (wherein Q is O or NH and R₆ is selected from alkyl, alkenyl, alkynyl, aryl, aralkyl, and cycloalkyl), alkoxy, aryloxy, halogen (F,Cl, Br, I), aryl, aralkyl, heteroaryl, heterocyclyl, heteroarylalkyl, or heterocyclylalkyl; or R_{L} and Rₘ can together join to form an oxo group];
R₃ is selected from
A) -(CH₂)ₙG wherein n is an integer from 0-5 and G is selected from
1) ORₑ {wherein Rₑ is selected from
a) acyl (with the proviso that n cannot be 0), and
b) -C(=O)NR_{f}R_{q} [wherein R_{f} and R_{q} can be independently selected from hydrogen, hydroxy (with the restriction that both R_{f} and R_{q} cannot both be hydroxy), alkyl, alkenyl, alkynyl, cycloalkyl, aryl, aralkyl, heteroaryl, heterocyclyl, and S(O)₂R₇ (wherein R₇ is selected from alkyl, alkenyl, alkynyl, cycloalkyl, aryl, aralkyl, heteroaryl, heterocyclyl, heterocyclylalkyl, heteroarylalkyl, and optionally substituted amino)]; and R_{f} and R_{q} may also together join to form a heterocyclyl ring; also, when n is zero, then R_{f} and Rq cannot be hydrogen, alkyl, alkenyl, alkynyl, cycloalkyl and R_{f} and Rq together cannot join to form a heterocyclyl ring};
2) -NRⱼC(=O)ORs (wherein Rⱼ is selected from hydrogen, lower (C₁-C₆) alkyl, lower (C₂-C₆) alkenyl, lower (C₂-C₆) alkynyl, lower (C₃-C₆) cycloalkyl, aryl, heteroaryl (with the proviso that the heteroaryl ring is not linked through a heteroatom), aralkyl (C₁-C₄), heteroarylalkyl (C₁-C₄), and heterocyclylalkyl (C₁-C₄), and Rₛ is selected from alkyl, alkenyl, alkynyl, cycloalkyl, aryl, aralkyl, heterocyclylalkyl, or heteroarylalkyl);
3) NRⱼYRᵤ (wherein Rⱼ is the same as defined above and Y is -C(=O), - C(=S) or SO₂ and Rᵤ is selected from alkyl, alkenyl, alkynyl, cycloalkyl, aryl, aralkyl, heteroaryl, heterocyclyl, heteroarylalkyl, and heterocyclylalkyl; and when n is 0 then Y cannot be -C(=O));
4) -NRⱼC(=T)NRₜRₓ (wherein Rₜ is OH or Rₓ and T is O, S, -N(CN), - N(NO₂) -CH(NO₂) Rⱼ is the same as defined above and Rₓ is selected from hydrogen, alkyl, alkenyl, alkynyl, cycloalkyl, aryl, aralkyl, heteroaryl, heterocyclyl, heteroarylalkyl, heterocyclylalkyl, and S(O)₂R₇ wherein R₇ is the same as defined above);
5) heterocyclyl;
6) heteroaryl; and
7) -(C=O)NRₐR_{b} (wherein Rₐ and R_{b} are independently selected from hydrogen, and Rᵤ wherein Rᵤ is same as defined earlier, also, Rₐ and R_{b} together with the nitrogen atom carrying them can be the N-terminus of an amino acid or di-tetrapeptide or Rₐ and R_{b} may together join to form a heterocyclyl ring);
R₃ is alternately selected from
B) -NRⱼRₘ (wherein Rⱼ is the same as defined above and Rₘ is selected from alkyl, cycloalkyl, aryl, aralkyl, heteroaryl, heterocyclyl, heteroarylalkyl, and heterocyclylalkyl);
C) -O(CH₂)_{w}G₁ [wherein w is an integer from 1-5 (and G₁ is selected from ORₑ (wherein Rₑ is the same as defined above), -NRⱼC(=O)ORₛ (wherein Rⱼ and Rₛ are the same as defined above), -NRⱼC(=T)NRₜRₓ (wherein Rⱼ, T, Rₜ and Rₓ are the same as defined above), -NRⱼYRᵤ (wherein Y, Rᵤ and Rⱼ are the same as defined above), heterocyclyl, and heteroaryl)];
D) -NRⱼ(CH₂)_{w}G₁ (wherein w, Rⱼ and G₁ are the same as defined above);
E) -O(CH₂)_{w}G₂ [wherein w is the same as defined above (and G₂ is selected from -C(=O)NRₐR_{b} (wherein Rₐ and R_{b} are the same as defined above), and -C(=O)ORₖ (wherein Rₖ is H or R₆ and R₆ is the same as defined above); or
F) -NRⱼ(CH₂)_{w}G₂ (wherein w is as defined above, Rⱼ and G₂ are the same as defined above))];
further, when R₃ is ORₑ then R₂ and Rₑ may together join to form a five membered acetal wherein the carbon linking the two oxygens is substituted with R_{L} and Rₘ (wherein R_{L} and Rₘ are the same as defined earlier, and R₁ is independently selected from
a) -(CH₂)ₜG₁ (wherein t is an integer from 2-4 and G₁ are the same as defined above and also when G₁ is heterocyclylalkyl group then the said group cannot be 4-(1-pyrrolidinyl) butyl),
b) -(CH₂)_{w}G₂ (wherein w and G₂ are the same as defined above),
c) aryl,
d) aralkyl (with the proviso that aralkyl cannot be phenylpropyl),
e) heteroaryl, and
f) heterocyclyl (wherein the heteroaryl and heterocyclyl rings are not linked through a heteroatom), and cycloalkyl (with the proviso that cycloalkyl cannot be cyclooctyl); and
R₄ and R₅ are independently selected from hydrogen, lower (C₁-C₆) alkyl, lower (C₂-C₆) alkenyl, lower (C₂-C₆) alkynyl, lower (C₃-C₈) cycloalkyl, aryl, acyl, heterocyclyl, heteroaryl, lower (C₁-C₄) heterocyclylalkyl, and lower (C₁-C₄) heteroarylalkyl; or R₄ and R₅ may together form a five-membered acetal wherein the carbon linking the two oxygens is substituted with R_{L} and Rₘ (wherein R_{L} and Rₘ are the same as defined earlier) with the proviso that when R₃ is ORₑ then the acetal must be isopropylidene acetal.

2. A compound selected from the group consisting of:
(3R or 3S)-1,2;5,6-Di-O-isopropylidene-3-deoxy-3-[{(4-chloro-phenyl)-amino}-carbonyl]-amino-α-D-allofuranoside (Compound No. 1);
1,2;5,6-Di-O-isopropylidene-3-deoxy-3-[2-{3-(4-chloro-phenyl)-ureido}-ethyl]-α-D-allofuranoside (Compound No. 2);
1,2;5,6-Di-O-isopropylidene-3-deoxy-3-[{(4-methoxy-phenyl)-amino}-carbonyl]-amino-α-D-allofuranoside (Compound No. 3);
1,2;5,6-Di-O-isopropylidene-3-deoxy-3-([{4-(2-methoxy-2-oxoethyl)-phenyl}-amino]- carbonyl)-amino-α-D-glucofuranoside (Compound No. 4);
1,2;5,6-Di-O-isopropylidene-3-deoxy-3-[{phenyl-sulphonylamino}-carbonyl]-amino-α-D-allofuranoside (Compound No. 5);
1,2;5,6-Di-O-isopropylidene-3-deoxy-3-([{4-(2-hydroxy-2-oxoethyl)-phenyl}-amino]-carbonyl)-amino-α-D-glucofuranoside (Compound No. 6);
1,2;5,6-Di-O-isopropylidene-3-deoxy-3-[{(4-methyl-phenyl)-sulphonylamino}-carbonyl]-amino-α-D-allofuranoside (Compound No. 7);
(3R or 3S)-1,2;5,6-Di-O-isopropylidene-3-deoxy-3-[{(4-methyl-phenyl)-amino}-carbonyl]-amino-α-D-glucofuranoside (Compound No. 8);
(3R or 3S)-1,2;5,6-Di-O-isopropylidene-3-deoxy-3-[{(4-nitro-phenyl)-amino}-carbonyl]-amino-α-D-allofuranoside (Compound No. 9);
(3R or 3S)-1,2;5,6-Di-O-isopropylidene-3-deoxy-3-[{(4-chloro-phenyl)-amino}-carbonyl]-amino-α-D- glucofuranoside (Compound No. 10);
(3R or 3S)-1,2;5,6-Di-O-isopropylidene-3-deoxy-3-[ {4-methyl-phenyl}-amino}-carbonyl]-amino-α-D-allofuranoside (Compound No. 11);
1,2;5,6-Di-O-isopropylidene-3-deoxy-3-[phenyl-amino-carbonyl]-amino-α-D-glucofuranoside (Compound No. 12);
1,2;5,6-Di-O-isopropylidene-3-O-[(4-methyl-phenyl)-amino]-carbonyl]-α-D-glucofuranoside (Compound No. 13);
(3R or 3S)-1,2;5,6-Di-O-isopropylidene-3-deoxy-3-[{(4-nitro-phenyl)-amino}-carbonyl]-amino-α-D-glucofuranoside (Compound No. 14);
1,2;5,6-Di-O-isopropylidene-3-O-[(4-methoxy-phenyl)-amino]-carbonyl-α-D-glucofuranoside (Compound No. 15);
1,2;5,6-Di-O-isopropylidene-3-O-[(4-chloro-phenyl)-amino]-carbonyl-α-D-glucofuranoside (Compound No. 16);
1,2;5,6-Di-O-isopropylidene-3-deoxy-3-[2-{3-(4-methyl-phenyl)-ureido}-ethyl]-α-D-allofuranoside (Compound No. 17);
1,2;5,6-Di-O-isopropylidene-3-deoxy-3-{2-[3-(4-methoxy-phenyl)-ureido]-ethyl}-α-D-allofuranoside (Compound No. 18);
1,2-O-Isopropylidene-3-deoxy-3-{[(4-methoxy-phenyl)-amino]-carbonyl}-amino-α-D-allofuranoside (Compound No. 19);
1,2-O-Isopropylidene-3-deoxy-3-12-[3-(4-methoxy-phenyl)-ureido]-ethyl}-α-D-allofuranoside (Compound No. 20);
1,2-O-Isopropylidene-3-O-{(4-chloro-phenyl)-amino}-carbonyl-α-D-allofuranoside (Compound No. 21);
1,2-O-Isopropylidene-3-deoxy-3-{[(4-nitro-phenyl)-amino]-carbonyl}-amino-α-D-allofuranoside (Compound No. 22);
1,2-O-Isopropylidene-3-deoxy-3-{[(4-chloro-phenyl)-amino]-carbonyl}-amino-α-D-allofuranoside (Compound No. 23);
1,2-O-Isopropylidene-3-O-{(4-methyl-phenyl)-amino}-carbonyl-α-D-allofuranoside (Compound No. 24);
1,2-O-Isopropylidene-3-deoxy-3-[2-{3-(4-methyl-phenyl)-ureido}-ethyl]-α-D-allofuranoside (Compound No. 25);
1,2-O-Isopropylidene-3-deoxy-3-{2-[3-(4-{2-methoxy-2-oxo-ethyl}-phenyl)-ureido]-ethyl}-α-D-allofuranoside (Compound No. 26);
1,2-O-Isopropylidene-3-deoxy-3-{2-[(4-methyl-phenyl)-amino]-carbonyl}-amino-α-D-allofuranoside (Compound No. 27);
1,2-O-Isopropylidene-3-deoxy-3-{2-[3-(4-{2-hydroxy-2-oxo-ethyl}-phenyl)-ureido]-ethyl}-α-D-allofuranoside (Compound No. 28);
2,3;5,6-Di-O-isopropylidene-1-O-{3-[1-(4-[3-chloro-phenyl]-piperazinyl)]-propyl}-α-D-mannofuranoside (Compound No. 29);
2,3;5,6-Di-O-isopropylidene-1-O-{2-[1-(4-[4-chloro-phenyl]-piperazinyl)]-ethyl}-α-D-mannofuranoside (Compound No. 30);
2,3;5,6-Di-O-isopropylidene-1-O-{2-[1-(4-[4-methoxy-phenyl]-piperazinyl)]-ethyl}-α-D-mannofuranoside (Compound No. 31);
2,3,5,6-D i-O-isopropylidene-1-O-{2-[1-(4-[2-pyrimidinyl]-piperazinyl)]-ethyl}-α-D-mannofuranoside (Compound No. 32);
2,3,5,6-Di-O-isopropylidene-1-O-{2-[4-morpholinyl]-ethyl}-α-D-mannofuranoside (Compound No. 33);
2,3;5,6-Di-O-isopropylidene-1-O-{2-[1-(4-benzyl-piperazinyl)]-ethyl}-α-D-mannofuranoside (Compound No. 34);
2,3;5,6-Di-O-isopropylidene-1-O-{2-[1-(4-[4-chloro-phenyl-amino-carbonyl]-piperazinyl)]-ethyl}-α-D-mannomranoside (Compound No. 35);
2,3;5,6-Di-O-isopropylidene-1-O-{2-(1-piperazinyl)-ethyl}-α-D-mannofuranoside (Compound No. 36);
2,3;5,6-Di-O-isopropylidene-1-O-{2-[1-(4-{[3,3']-bithiophenyl-5-ylmethyl}-piperazinyl)]-ethyl}-α-D-mannofuranoside (Compound No. 37);
2,3;5,6-Di-O-isopropylidene-1-O-{2-[1-(4-[isopropylamino-thiocarbonyl]-piperazinyl)]-ethyl}-α-D-mannofuranoside (Compound No. 38);
2,3;5,6-Di-O-isopropylidene-1-O-{2-[1-(4-{(1-naphthyl)-amino-carbonyl}-piperazinyl)]-ethyl}-α-D-mannofuranoside (Compound No. 39);
2,3;5,6-Di-O-isopropylidene-1-O-{2-[1-(4-{4-methyl-phenyl-sulphonyl}-piperazinyl)]-ethyl}-α-D-mannofuranoside (Compound No. 40);
2,3;5,6-Di-O-isopropylidene-1-O-{2-[1-(4-[2-(2,6-dioxo-1-piperidinyl)-acetyl]-piperazinyl)]-ethyl}-α-D-mannofuranoside (Compound No. 41);
2,3;5,6-Di-O-isopropylidene-1-O-{2-[1-(4-[2-thienyl-methyl-carbonyl]-piperazinyl)]-ethyl}-α-D-mannofuranoside (Compound No. 42);
2,3;5,6-Di-O-isopropylidene-1-O-{2-[1-(4-[4-fluoro-phenyl-carbonyl]-piperazinyl)]-ethyl}-α-D-mannofuranoside (Compound No. 43);
2,3;5,6-Di-O-isopropylidene-1-O-{2-[1-(4-{2-(1-[1*H*-1,2,4-triazolyl])-acetyl}-piperazinyl]-ethyl}-α-D-mannomranoside (Compound No. 44);
2,3;5,6-Di-O-isopropylidene-1-O-{2-[1-(4-[(2-chloro-3,4-methylenedioxyphenyl)-methyl]-piperazinyl)]-ethyl}-α-D-mannofuranoside (Compound No. 45);
2,3;5,6-Di-O-isopropylidene-1-O-{2-[1-(4-{2-[4-chloro-phenoxy]-acetyl}-piperazinyl)]-ethyl}-α-D-mannofuranoside (Compound No. 46);
1,2;5,6-Di-O-isopropylidene-3-deoxy-3-[{(4-trifluoromethyl-phenyl)-amino}-carbonyl]-amino-α-D-allofuranoside (Compound No. 47);
1,2;5,6-Di-O-isopropylidene-3-O-[(4-fluoro-phenyl)-amino]-carbonyl]-α-D-glucofuranoside (Compound No. 48);
1,2;5,6-Di-O-isopropylidene-3-deoxy-3-{[(2-phenylethyl)-amino]thiocarbonyl}-amino-α-D-allofuranoside (Compound No. 49);
1,2;5,6-Di-O-isopropylidene-3-deoxy-3-{[(4-fluorophenyl)-amino]thiocarbonyl}-amino-α-D-allofuranoside (Compound No. 50);
1,2;5,6-Di-O-isopropylidene-3-deoxy-3-[{(4-fluoro-phenyl)-amino}-carbonyl]-amino-α-D-allofuranoside (Compound No. 51);
1,2;5,6-Di-O-isopropylidene-3-deoxy-3-{[4-fluoro-phenyl]sulphonyl}-amino-α-D-allofuranoside (Compound No. 52);
1,2;5,6-Di-O-isopropylidene-3-deoxy-3-{[4-methyl-phenyl]-sulphonyl}-amino-α-D-allofuranoside (Compound No. 53);
1,2;5,6-Di-O-isopropylidene-3-deoxy-3- {[(4-[2-methoxy-2-oxo-ethyl]-phenyl)-amino]-carbonyl}-methylamino-α-D-allofuranoside (Compound No. 54);
1,2;5,6-Di-O-isopropylidene-3-deoxy-3-{[(4-fluorophenyl)-amino]thiocarbonyl}-amino-α-D-allofuranoside (Compound No. 55);
1,2;5,6-Di-O-isopropylidene-3-deoxy-3-{[(4-trifluoromethoxyphenyl)-amino]thiocarbonyl}-amino-α-D-allofuranoside (Compound No. 56);
1,2;5,6-Di-O-isopropylidene-3-deoxy-3-{[(3,5-difluorophenyl)-methyl]carbonyl}-amino-α-D-allofuranoside (Compound No. 57);
1,2;5,6-Di-O-isopropylidene-3-deoxy-3-{[(4-nitrophenyl)-amino]thiocarbonyl}-amino-α-D-allofuranoside (Compound No. 58);
1,2;5,6-Di-O-isopropylidene-3-deoxy-3-{[(2,4-difluorophenyl)-amino]thiocarbonyl}-amino-α-D-allofuranoside (Compound No. 59);
1,2;5,6-Di-O-isopropylidene-3-deoxy-3-{[(3,4-dichlorophenyl)-amino]thiocarbonyl}-amino-α-D-allofuranoside (Compound No. 60);
1,2;5,6-Di-O-isopropylidene-3-deoxy-3-{[(4-trifluoromethylphenyl)-amino]thiocarbonyl}-amino-α-D-allofuranoside (Compound No. 61);
1,2;5,6-Di-O-isopropylidene-3-deoxy-3- {[(4-methoxyphenyl)-amino]thiocarbonyl}-amino-α-D-allofuranoside (Compound No. 62);
1,2;5,6-Di-O-isopropylidene-3-deoxy-3- {[(4-benzyloxyphenyl)-amino]thiocarbonyl}-amino-α-D-allofuranoside (Compound No. 63);
1,2;5,6-Di-O-isopropylidene-3-deoxy-3-{[(3-nitrophenyl)-amino]thiocarbonyl}-amino-α-D-allofuranoside (Compound No. 64);
1,2;5,6-Di-O-isopropylidene-3-deoxy-3-{[(2-fluorophenyl)-amino]thiocarbonyl}-amino-α-D-allofuranoside (Compound No. 65);
1,2;5,6-Di-O-isopropylidene-3-deoxy-3-{[(2-trifluoromethylphenyl)-amino]thiocarbonyl}-amino-α-D-allofuranoside (Compound No. 66);
1,2;5,6-Di-O-isopropylidene-3-deoxy-3-{[(2-nitrophenyl)-amino]thiocarbonyl}-amino-α-D-allofuranoside (Compound No. 67);
1,2;5,6-Di-O-isopropylidene-3-deoxy-3-{[(4-cyanophenyl)-amino]thiocarbonyl}-amino-α-D-allofuranoside (Compound No. 68);
1,2;5,6-Di-O-isopropylidene-3-deoxy-3-{[(4-difluoromethoxyphenyl)-amino]thiocarbonyl}-amino-α-D-allofuranoside (Compound No. 69);
1,2;5,6-Di-O-isopropylidene-3-deoxy-3-{[(2-difluoromethoxyphenyl)-amino]thiocarbonyl}-amino-α-D-allofuranoside (Compound No. 70);
1,2;5,6-Di-O-isopropylidene-3-deoxy-3-{[(naphthyl)-amino]thiocarbonyl)-amino-α-D-allofuranoside (Compound No. 71);
1,2;5,6-Di-O-isopropylidene-3-deoxy-3- {[(4-thiomethylphenyl)-amino]thiocarbonyl}-amino-α-D-allofuranoside (Compound No. 72);
1,2;5,6-Di-O-isopropylidene-3-deoxy-3-{[(2,4-difluorophenyl)-methyl]carbonyl}-amino-α-D-allofuranoside (Compound No. 73);
1,2;5,6-Di-O-isopropylidene-3-deoxy-3-{[(3-methoxyphenyl)-methyl]carbonyl}-amino-α-D-allofuranoside (Compound No. 74);
1,2;5,6-Di-O-isopropylidene-3-deoxy-3-{[(2-methoxyphenyl)-methyl]carbonyl}-amino-a-D-allofuranoside (Compound No. 75);
1,2;5,6-Di-O-isopropylidene-3-deoxy-3-{[(2-chloro-4-Quorophenyl)-methyl]carbonyl}-amino-α-D-allofuranoside (Compound No. 76);
1,2;5,6-Di-O-isopropylidene-3-deoxy-3-{[(4-methylphenyl)-methyl]carbonyl}-amino-α-D-allofuranoside (Compound No. 77);
1,2;5,6-Di-O-isopropylidene-3-deoxy-3-{[(4-nitrophenyl)-methyl]carbonyl}-amino-α-D-allofuranoside (Compound No. 78);
1,2;5,6-Di-O-isopropylidene-3-deoxy-3-{[(2-nitrophenyl)-methyl]carbonyl}-amino-α-D-allofuranoside (Compound No. 79);
1,2;5,6-Di-O-isopropylidene-3-deoxy-3-{[(2-trifluoromethylphenyl)-methyl]carbonyl}-amino-α-D-allofuranoside (Compound No. 80);
1,2;5,6-Di-O-isopropylidene-3-deoxy-3-{[(2-trifluoromethoxyphenyl)-methyl]carbonyl}-amino-α-D-allofuranoside (Compound No. 81);
1,2;5,6-Di-O-isopropylidene-3-deoxy-3-{[(3-trifluoromethoxyphenyl)-methyl]carbonyl}-amino-α-D-allofuranoside (Compound No. 82);
1,2;5,6-Di-O-isopropylidene-3-deoxy-3-{[(4-isopropylphenyl)-methyl]carbonyl}-amino-α-D-allofuranoside (Compound No. 83);
1,2;5,6-Di-O-isopropylidene-3-deoxy-3-{[(3-chlorophenyl)-methyl]carbonyl}-amino-α-D-allofuranoside (Compound No. 84);
1,2;5,6-Di-O-isopropylidene-3-deoxy-3-{[(3-methylphenyl)-methyl]carbonyl}-amino-α-D-allofuranoside (Compound No. 85);
1,2;5,6-Di-O-isopropylidene-3-deoxy-3-{[(2,4-dichlorophenyl)-methyl]carbonyl}-amino-α-D-allofuranoside (Compound No. 86);
1,2;5,6-Di-O-isopropylidene-3-deoxy-3-{[(3-chlorophenyl)-methyl]carbonyl}-amino-α-D-allofuranoside (Compound No. 87);
1,2;5,6-Di-O-isopropylidene-3-deoxy-3-{[(4-(4-propylcyclohexyl)phenyl)-amino]carbonyl}-amino-α-D-allofuranoside (Compound No. 88);
1,2;5,6-Di-O-isopropylidene-3-deoxy-3-{[(4-(4-hexylcyclohexyl)phenyl)-amino]carbonyl}-amino-α-D-allofuranoside (Compound No. 89);
1,2;5,6-Di-O-isopropylidene-3-deoxy-3- {[(pyridin-3-yl)-amino]thiocarbonyl}-amino-α-D-allofuranoside (Compound No. 90);
1,2;5,6-Di-O-isopropylidene-3-deoxy-3-{[(4-chlorophenyl)-amino]thiocarbonyl}-amino-α-D-allofuranoside (Compound No. 91);
1,2;5,6-Di-O-isopropylidene-3-deoxy-3-{[(2-chlorophenyl)-amino]thiocarbonyl}-amino-α-D-allofuranoside (Compound No. 92);
1,2;5,6-Di-O-isopropylidene-3-deoxy-3-{[(3-chlorophenyl)-amino]thiocarbonyl}-amino-α-D-allofuranoside (Compound No. 93);
1,2;5,6-Di-O-isopropylidene-3-deoxy-3-{[(2,6-dichlorophenyl)-amino]thiocarbonyl}-amino-α-D-allofuranoside (Compound No. 94);
1,2;5,6-Di-O-isopropylidene-3-deoxy-3- {[(2,6-dimethylphenyl)-amino]thiocarbonyl}-amino-α-D-allofuranoside (Compound No. 95);
1,2;5,6-Di-O-isopropylidene-3-deoxy-3-{[(2,5-difluorophenyl)-amino]thiocarbonyl}-amino-α-D-allofuranoside (Compound No. 96);
1,2;5,6-Di-O-isopropylidene-3-deoxy-3-{[(4-iodophenyl)-amino]thiocarbonyl}-amino-α-D-allofuranoside (Compound No. 97);
1,2;5,6-Di-O-isopropylidene-3-deoxy-3-{[(3-methoxyphenyl)-amino]thiocarbonyl}-amino-α-D-allofuranoside (Compound No. 98);
1,2;5,6-Di-O-isopropylidene-3-deoxy-3-{[(2-methoxyphenyl)-amino]thiocarbonyl}-amino-α-D-allofuranoside (Compound No. 99);
1,2;5,6-Di-O-isopropylidene-3-deoxy-3-{[(3,4-dichlorophenyl)-methyl]carbonyl}-amino-α-D-allofuranoside (Compound No. 100);
1,2;5,6-Di-O-isopropylidene-3-deoxy-3-{[(4-fluorophenyl)-amino]thiocarbonyl}-amino-α-D-glucofuranoside (Compound No. 101);
1,2;5,6-Di-O-isopropylidene-3-deoxy-3-{[(3-nitrophenyl)-amino]thiocarbonyl}-amino-α-D-glucofuranoside (Compound No. 102);
1,2;5,6-Di-O-isopropylidene-3-deoxy-3-{[(2-fluorophenyl)-amino]thiocarbonyl}-amino-α-D-glucofuranoside (Compound No. 103);
1,2;5,6-Di-O-isopropylidene-3-deoxy-3-{[(2-trifluoromethylphenyl)-amino]thiocarbonyl}-amino-α-D-glucofuranoside (Compound No. 104);
1,2;5,6-Di-O-isopropylidene-3-deoxy-3-{[(2,4-difluorophenyl)-amino]thiocarbonyl}-amino-α-D-glucofuranoside (Compound No. 105);
1,2;5,6-Di-O-isopropylidene-3-deoxy-3-{[(2-difluoromethoxyphenyl)-amino]thiocarbonyl}-amino-α-D-glucofuranoside (Compound No. 106);
1,2;5,6-Di-O-isopropylidene-3-deoxy-3-{[(4-difluoromethoxyphenyl)-amino]thiocarbonyl}-amino-α-D-glucofuranoside (Compound No. 107).
1,2;5,6-Di-O-isopropylidene-3-deoxy-3-{[(4-(phenyl)phenyl)-methyl]carbonyl}-amino-α-D-allofuranoside (Compound No. 108);
1,2;5,6-Di-O-isopropylidene-3-deoxy-3-{[(2-naphthyl)-methyl]carbonyl}-amino-α-D-allofuranoside (Compound No. 109);
1,2;5,6-Di-O-isopropylidene-3-deoxy-3-{[(2-fluorophenyl)-methyl]carbonyl}-amino-α-D-allofuranoside (Compound No. 110);
1,2;5,6-Di-O-isopropylidene-3-deoxy-3-{[(2-chloro-6-fluorophenyl)-methyl]carbonyl}-amino-α-D-allofuranoside (Compound No. 111);
1,2;5,6-Di-O-isopropylidene-3-deoxy-3-{[(2,5-difluorophenyl)-methyl]carbonyl}-amino-α-D-allofuranoside (Compound No. 112);
1,2;5,6-Di-O-isopropylidene-3-deoxy-3-{[(4-fluorophenyl)-methyl]carbonyl}-amino-α-D-allofuranoside (Compound No. 113);
1,2;5,6-Di-O-isopropylidene-3-deoxy-3-{[(3-fluorophenyl)-methyl]carbonyl}-amino-α-D-allofuranoside (Compound No. 114);
1,2;5,6-Di-O-isopropylidene-3-deoxy-3-{[(3-bromophenyl)-methyl]carbonyl}-amino-α-D-allofuranoside (Compound No. 115);
1,2;5,6-Di-O-isopropylidene-3-deoxy-3-{[(3,4-difluorophenyl)-methyl]carbonyl}-amino-α-D-allofuranoside (Compound No. 116).

3. A method of making compounds of Formula VII 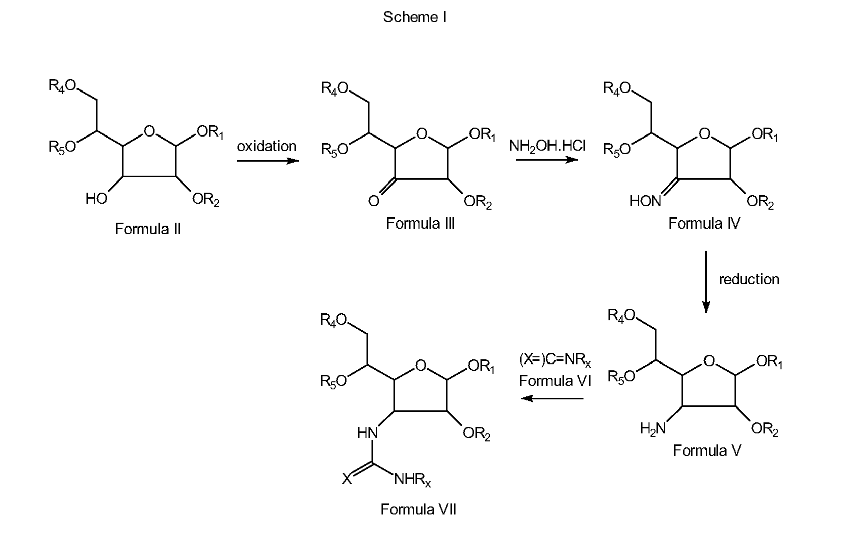 wherein
R₁ and R₂ together form a five-membered acetal, wherein the carbon atom joining the oxygens can be substituted with R_{L} and Rₘ [wherein R_{L} and Rₘ are independently selected from hydrogen, alkyl, alkenyl, alkynyl, cycloalkyl, aryl, or aralkyl; or R_{L} and Rₘ can together join to form a 3-8 membered ring, wherein the ring may optionally contain one or more heteroatoms selected from O, N or S, and the ring may be optionally substituted with one or more of alkyl, alkenyl, alkynyl, amino, substituted amino, cycloalkyl, oxo, hydroxy, carboxy, -COQR₆ (wherein Q is O or NH and R₆ is selected from alkyl, alkenyl, alkynyl, aryl, aralkyl, and cycloalkyl), alkoxy, aryloxy, halogen (F,Cl, Br, I), aryl, aralkyl, heteroaryl, heterocyclyl, heteroarylalkyl, or heterocyclylalkyl; or R_{L} and Rₘ can together join to form an oxo group]; and
R₄ and R₅ are independently selected from hydrogen, lower (C₁-C₆) alkyl, lower (C₂-C₆) alkenyl, lower (C₂-C₆) alkynyl, lower (C₃-C₈) cycloalkyl, aryl, acyl, heterocyclyl, heteroaryl, lower (C₁-C₄) heterocyclylalkyl, and lower (C₁-C₄) heteroarylalkyl; or R₄ and R₅ may together form a five-membered acetal wherein the carbon linking the two oxygens is substituted with R_{L} and Rₘ (wherein R_{L} and Rₘ are the same as defined earlier),
the method comprising
oxidizing a compound of Formula II to give a compound of Formula III; reacting the compound of Formula III with hydroxylamine hydrochloride to form a compound of Formula IV;
reducing the compound of Formula IV to form a compound of Formula V; and reacting the compound of Formula V with a compound of Formula VI (wherein X is S or O, and Rₓ is selected from hydrogen, alkyl, alkenyl, alkynyl, cycloalkyl, aryl, aralkyl, heteroaryl, heterocyclyl, heteroarylalkyl, heterocyclylalkyl, and S(O)₂R₇ wherein R₇ is selected from alkyl, alkenyl, alkynyl, cycloalkyl, aryl, aralkyl, heteroaryl, heterocyclyl, heterocyclylalkyl, heteroarylalkyl, and optionally substituted amino) to give a compound of Formula VII.

4. A method of making a compound of Formula VII 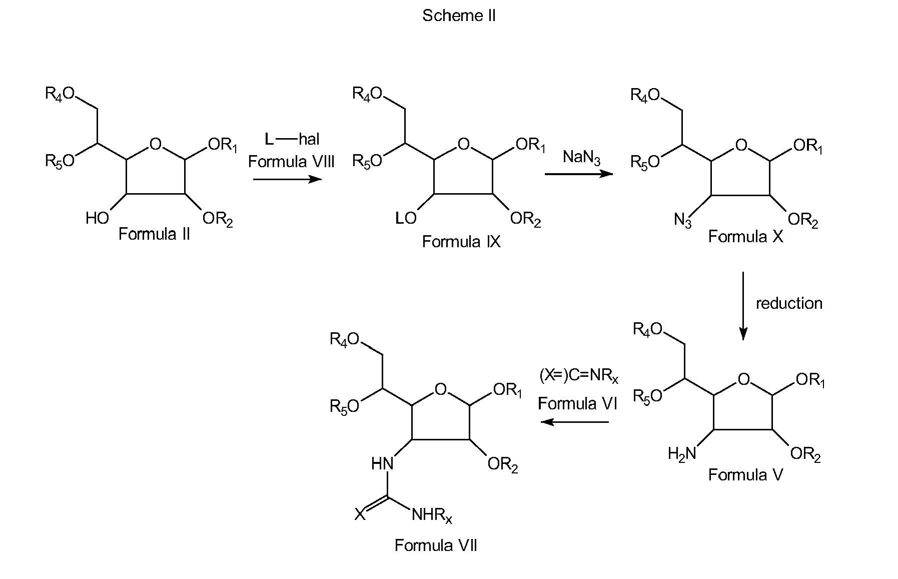 wherein
R₁ and R₂ together form a five-membered acetal, wherein the carbon atom joining the oxygens can be substituted with R_{L} and Rₘ [wherein R_{L} and Rₘ are independently selected from hydrogen, alkyl, alkenyl, alkynyl, cycloalkyl, aryl, or aralkyl; or R_{L} and Rₘ can together join to form a 3-8 membered ring, wherein the ring may optionally contain one or more heteroatoms selected from O, N or S, and the ring may be optionally substituted with one or more of alkyl, alkenyl, alkynyl, amino, substituted amino, cycloalkyl, oxo, hydroxy, carboxy, -COQR₆ (wherein Q is O or NH and R₆ is selected from alkyl, alkenyl, alkynyl, aryl, aralkyl, and cycloalkyl), alkoxy, aryloxy, halogen (F,Cl, Br, I), aryl, aralkyl, heteroaryl, heterocyclyl, heteroarylalkyl, or heterocyclylalkyl; or R_{L} and Rₘ can together join to form an oxo group]; and
R₄ and R₅ are independently selected from hydrogen, lower(C₁-C₆) alkyl, lower (C₂-C₆) alkenyl, lower (C₂-C₆) alkynyl, lower (C₃-C₈) cycloalkyl, aryl, acyl, heterocyclyl, heteroaryl, lower(C₁-C₄) heterocyclylalkyl, and lower(C₁-C₄) heteroarylalkyl; or R₄ and R₅ may together form a five-membered acetal wherein the carbon linking the two oxygens is substituted with R_{L} and Rₘ (wherein R_{L} and Rₘ are the same as defined earlier),
the method comprising
reacting a compound of Formula II with a compound of Formula VIII (wherein L is a leaving group and hal is halogen) to form a compound of Formula IX;
reacting the compound of Formula IX with sodium azide to form a compound of Formula X;
reducing the compound of Formula X to form a compound of Formula V, and reacting the compound of Formula V with a compound of Formula VI (wherein X is S or O, and Rₓ is selected from hydrogen, alkyl, alkenyl, alkynyl, cycloalkyl, aryl, aralkyl, heteroaryl, heterocyclyl, heteroarylalkyl, heterocyclylalkyl, and S(O)₂R₇ wherein R₇ is selected from alkyl, alkenyl, alkynyl, cycloalkyl, aryl, aralkyl, heteroaryl, heterocyclyl, heterocyclylalkyl, heteroarylalkyl, and optionally substituted amino) to give a compound of Formula VII.

5. A method of making a compound of Formula XI 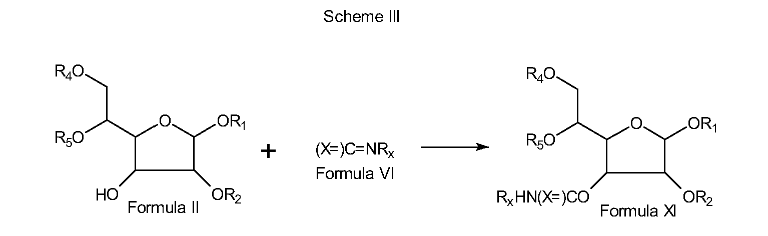 wherein
R₁ and R₂ together form a five-membered acetal, wherein the carbon atom joining the oxygens can be substituted with R_{L} and Rₘ [wherein R_{L} and Rₘ are independently selected from hydrogen, alkyl, alkenyl, alkynyl, cycloalkyl, aryl, or aralkyl; or R_{L} and Rₘ can together join to form a 3-8 membered ring, wherein the ring may optionally contain one or more heteroatoms selected from O, N or S, and the ring may be optionally substituted with one or more of alkyl, alkenyl, alkynyl, amino, substituted amino, cycloalkyl, oxo, hydroxy, carboxy, -COQR₆ (wherein Q is O or NH and R₆ is selected from alkyl, alkenyl, alkynyl, aryl, aralkyl, and cycloalkyl), alkoxy, aryloxy, halogen (F,Cl, Br, I), aryl, aralkyl, heteroaryl, heterocyclyl, heteroarylalkyl, or heterocyclylalkyl; or R_{L} and Rₘ can together join to form an oxo group]; and
R₄ and R₅ are independently selected from hydrogen, lower (C₁-C₆) alkyl, lower (C₂-C₆) alkenyl, lower (C₂-C₆) alkynyl, lower (C₃-C₈) cycloalkyl, aryl, acyl, heterocyclyl, heteroaryl, lower (C₁-C₄) heterocyclylalkyl, and lower (C₁-C₄) heteroarylalkyl; or R₄ and R₅ may together form a five-membered acetal wherein the carbon linking the two oxygens is substituted with R_{L} and Rₘ (wherein R_{L} and Rₘ are the same as defined earlier),
the method comprising
reacting a compound of Formula II with a compound of Formula VI VI (wherein X is S or O, and Rx is selected from hydrogen, alkyl, alkenyl, alkynyl, cycloalkyl, aryl, aralkyl, heteroaryl, heterocyclyl, heteroarylalkyl, heterocyclylalkyl, and S(O)₂R₇ (wherein R₇ is selected from alkyl, alkenyl, alkynyl, cycloalkyl, aryl, aralkyl, heteroaryl, heterocyclyl, heterocyclylalkyl, heteroarylalkyl, and optionally substituted amino) to give a compound of Formula XI.

6. A method of making a compound of Formula XVI 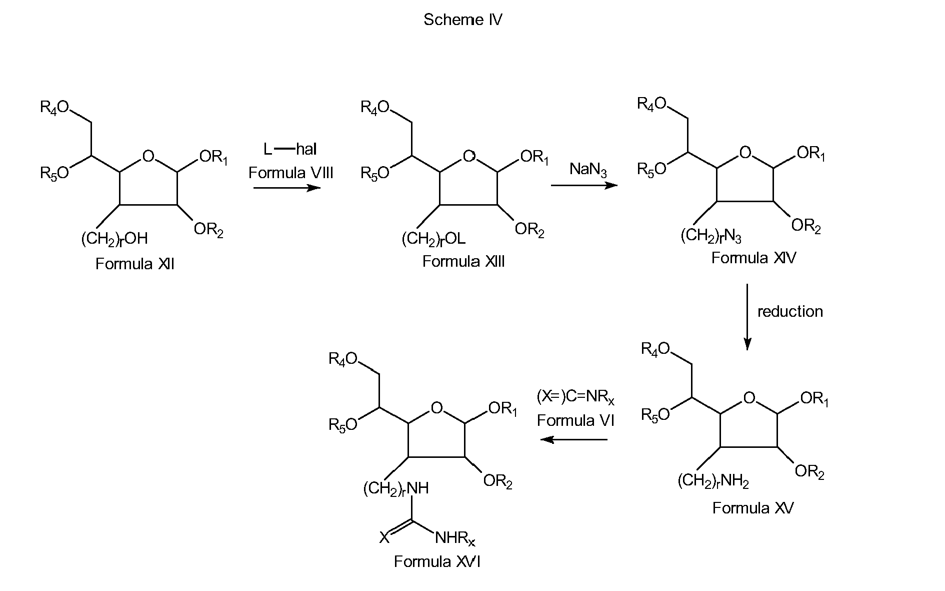 wherein
R₁ and R₂ together form a five-membered acetal, wherein the carbon atom joining the oxygens can be substituted with R_{L} and Rₘ [wherein R_{L} and Rₘ are independently selected from hydrogen, alkyl, alkenyl, alkynyl, cycloalkyl, aryl, or aralkyl; or R_{L} and Rₘ can together join to form a 3-8 membered ring, wherein the ring may optionally contain one or more heteroatoms selected from O, N or S, and the ring may be optionally substituted with one or more of alkyl, alkenyl, alkynyl, amino, substituted amino, cycloalkyl, oxo, hydroxy, carboxy, -COQR₆ (wherein Q is O or NH and R₆ is selected from alkyl, alkenyl, alkynyl, aryl, aralkyl, and cycloalkyl), alkoxy, aryloxy, halogen (F,Cl, Br, I), aryl, aralkyl, heteroaryl, heterocyclyl, heteroarylalkyl, or heterocyclylalkyl; or R_{L} and Rₘ can together join to form an oxo group]; and
R₄ and R₅ are independently selected from hydrogen, lower(C₁-C₆) alkyl, lower(C₂-C₆) alkenyl, lower (C₂-C₆) alkynyl, lower (C₃-C₈) cycloalkyl, aryl, acyl, heterocyclyl, heteroaryl, lower (C₁-C₄) heterocyclylalkyl, and lower(C₁-C₄) heteroarylalkyl; or R₄ and R₅ may together form a five-membered acetal wherein the carbon linking the two oxygens is substituted with R_{L} and Rₘ (wherein R_{L} and Rₘ are the same as defined earlier),
the method comprising
reacting a compound of Formula XI with a compound of Formula VIII (wherein L is a leaving group and hal is halogen) to form a compound of Formula XIII;
reacting the compound of Formula XIII with sodium azide to form a compound of Formula XIV;
reducing the compound of Formula XIV to form a compound of Formula XV; and
reacting the compound of Formula XV with a compound of Formula VI (wherein X is S or O, and Rx is selected from hydrogen, alkyl, alkenyl, alkynyl, cycloalkyl, aryl, aralkyl, heteroaryl, heterocyclyl, heteroarylalkyl, heterocyclylalkyl, and S(O)₂R₇ (wherein R₇ is selected from alkyl, alkenyl, alkynyl, cycloalkyl, aryl, aralkyl, heteroaryl, heterocyclyl, heterocyclylalkyl, heteroarylalkyl, and optionally substituted amino) to give a compound of Formula XVI.

7. A method of making a compound of Formula XIX 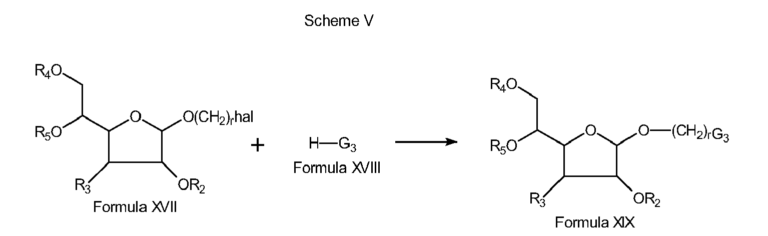 wherein
R₂ and R₃ form an acetal, wherein the carbon linking the two oxygens is substituted with R_{L} and Rₘ (wherein R_{L} and Rₘ are independently selected from hydrogen, alkyl, alkenyl, alkynyl, cycloalkyl, aryl, or aralkyl; or R_{L} and Rₘ can together join to form a 3-8 membered ring, wherein the ring may optionally contain one or more heteroatoms selected from O, N or S, and the ring may be optionally substituted with one or more of alkyl, alkenyl, alkynyl, amino, substituted amino, cycloalkyl, oxo, hydroxy, carboxy, -COQR₆ (wherein Q is O or NH and R₆ is selected from alkyl, alkenyl, alkynyl, aryl, aralkyl, and cycloalkyl), alkoxy, aryloxy, halogen (F,Cl, Br, I), aryl, aralkyl, heteroaryl, heterocyclyl, heteroarylalkyl, or heterocyclylalkyl; or R_{L} and Rₘ can together join to form an oxo group);
R₃ is -(CH₂)ₙG wherein n is an integer from 0-5 and G is ORₑ {wherein Rₑ is selected from
a) acyl (with the proviso that n cannot be 0), and
b) -C(=O)NR_{f}R_{q} [wherein R_{f} and Rq can be independently selected from hydrogen, hydroxy (with the restriction that both R_{f} and Rq cannot both be hydroxy), alkyl, alkenyl, alkynyl, cycloalkyl, aryl, aralkyl, heteroaryl, heterocyclyl, and S(O)₂R₇ (wherein R₇ is selected from alkyl, alkenyl, alkynyl, cycloalkyl, aryl, aralkyl, heteroaryl, heterocyclyl, heterocyclylalkyl, heteroarylalkyl, and optionally substituted amino)]; and R_{f} and R_{q} may also together join to form a heterocyclyl ring; also, when n is zero, then R_{f} and Rq cannot be hydrogen, alkyl, alkenyl, alkynyl, cycloalkyl and R_{f} and Rq together cannot join to form a heterocyclyl ring}; and
R₄ and R₅ are independently selected from hydrogen, lower (C₁-C₆) alkyl, lower (C₂-C₆) alkenyl, lower (C₂-C₆) alkynyl, lower (C₃-C₈) cycloalkyl, aryl, acyl, heterocyclyl, heteroaryl, lower (C₁-C₄) heterocyclylalkyl, and lower (C₁-C₄) heteroarylalkyl; or R₄ and R₅ may together form isopropylidene acetal, the method comprising
reacting a compound of Formula XVII (wherein r is an integer from 1-3 and hal is halogen) with a compound of Formula XVIII (wherein G₃ is a heterocyclyl ring attached to H through N) to form a compound of Formula XIX.

8. A method of making a compound of Formula XXII 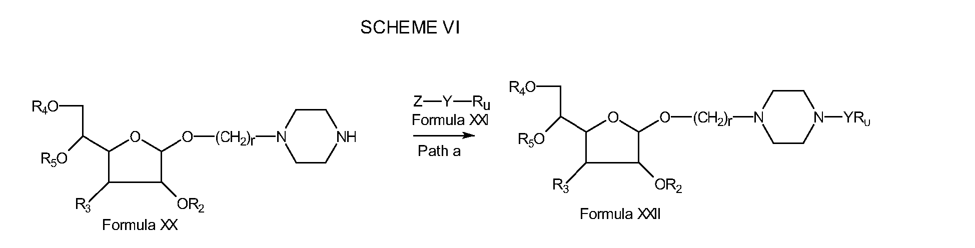 wherein
R₂ and R₃ form an acetal, wherein the carbon linking the two oxygens is substituted with R_{L} and Rₘ (wherein R_{L} and Rₘ are independently selected from hydrogen, alkyl, alkenyl, alkynyl, cycloalkyl, aryl, or aralkyl; or R_{L} and Rₘ can together join to form a 3-8 membered ring, wherein the ring may optionally contain one or more heteroatoms selected from O, N or S, and the ring may be optionally substituted with one or more of alkyl, alkenyl, alkynyl, amino, substituted amino, cycloalkyl, oxo, hydroxy, carboxy, -COQR₆ (wherein Q is O or NH and R₆ is selected from alkyl, alkenyl, alkynyl, aryl, aralkyl, and cycloalkyl), alkoxy, aryloxy, halogen (F,Cl, Br, I), aryl, aralkyl, heteroaryl, heterocyclyl, heteroarylalkyl, or heterocyclylalkyl; or R_{L} and Rₘ can together join to form an oxo group);
R₃ is -(CH₂)ₙG wherein n is an integer from 0-5 and G is ORₑ {wherein Rₑ is selected from
a) acyl (with the proviso that n cannot be 0), and
b) -C(=O)NR_{f}R_{q} [wherein R_{f} and Rq can be independently selected from hydrogen, hydroxy (with the restriction that both R_{f} and R_{q} cannot both be hydroxy), alkyl, alkenyl, alkynyl, cycloalkyl, aryl, aralkyl, heteroaryl, heterocyclyl, and S(O)₂R₇ (wherein R₇ is selected from alkyl, alkenyl, alkynyl, cycloalkyl, aryl, aralkyl, heteroaryl, heterocyclyl, heterocyclylalkyl, heteroarylalkyl, and optionally substituted amino)]; and R_{f} and R_{q} may also together join to form a heterocyclyl ring; also, when n is zero, then R_{f} and Rq cannot be hydrogen, alkyl, alkenyl, alkynyl, cycloalkyl and R_{f} and Rq together cannot join to form a heterocyclyl ring}; and
R₄ and R₅ are independently selected from hydrogen, lower (C₁-C₆) alkyl, lower (C₂-C₆) alkenyl, lower (C₂-C₆) alkynyl, lower (C₃-C₈) cycloalkyl, aryl, acyl, heterocyclyl, heteroaryl, lower (C₁-C₄) heterocyclylalkyl, and lower (C₁-C₄) heteroarylalkyl; or R₄ and R₅ may together form isopropylidene acetal,
the method comprising
reacting a compound of Formula XX with a compound of Formula XXI (wherein Z is halogen or OH; -C(=O), -C(=S) or SO₂ and Rᵤ is selected from alkyl, alkenyl, alkynyl, cycloalkyl, aryl, aralkyl, heteroaryl, heterocyclyl, heteroarylalkyl, and heterocyclylalkyl; and when n is 0 then Y cannot be -C(=O)) to give a compound of Formula XXII.

9. A method of making a compound of Formula XXIII 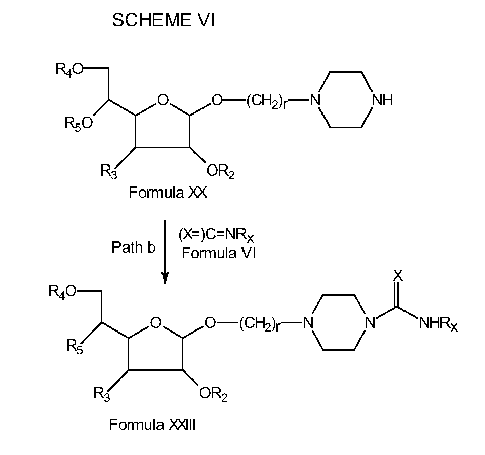 wherein
R₂ and R₃ form an acetal, wherein the carbon linking the two oxygens is substituted with R_{L} and Rₘ (wherein R_{L} and Rₘ are independently selected from hydrogen, alkyl, alkenyl, alkynyl, cycloalkyl, aryl, or aralkyl; or R_{L} and Rₘ can together join to form a 3-8 membered ring, wherein the ring may optionally contain one or more heteroatoms selected from O, N or S, and the ring may be optionally substituted with one or more of alkyl, alkenyl, alkynyl, amino, substituted amino, cycloalkyl, oxo, hydroxy, carboxy, -COQR₆ (wherein Q is O or NH and R₆ is selected from alkyl, alkenyl, alkynyl, aryl, aralkyl, and cycloalkyl), alkoxy, aryloxy, halogen (F,Cl, Br, I), aryl, aralkyl, heteroaryl, heterocyclyl, heteroarylalkyl, or heterocyclylalkyl; or R_{L} and Rₘ can together join to form an oxo group);
R₃ is -(CH₂)ₙG wherein n is an integer from 0-5 and G is ORₑ {wherein Rₑ is selected from
a) acyl (with the proviso that n cannot be 0), and
b) -C(=O)NR_{f}R_{q} [wherein R_{f} and R_{q} can be independently selected from hydrogen, hydroxy (with the restriction that both R_{f} and R_{q} cannot both be hydroxy), alkyl, alkenyl, alkynyl, cycloalkyl, aryl, aralkyl, heteroaryl, heterocyclyl, and S(O)₂R₇ (wherein R₇ is selected from alkyl, alkenyl, alkynyl, cycloalkyl, aryl, aralkyl, heteroaryl, heterocyclyl, heterocyclylalkyl, heteroarylalkyl, and optionally substituted amino)]; and R_{f} and Rqmay also together join to form a heterocyclyl ring; also, when n is zero, then R_{f} and R_{q} cannot be hydrogen, alkyl, alkenyl, alkynyl, cycloalkyl and R_{f} and Rq together cannot join to form a heterocyclyl ring}; and
R₄ and R₅ are independently selected from hydrogen, lower (C₁-C₆) alkyl, lower (C₂-C₆) alkenyl, lower (C₂-C₆) alkynyl, lower (C₃-C₈) cycloalkyl, aryl, acyl, heterocyclyl, heteroaryl, lower (C₁-C₄) heterocyclylalkyl, and lower (C₁-C₄) heteroarylalkyl; or R₄ and R₅ may together form isopropylidene acetal,
the method comprising
reacting a compound of Formula XX with a compound of Formula VI (wherein X is S or O, and Rₓ is selected from hydrogen, alkyl, alkenyl, alkynyl, cycloalkyl, aryl, aralkyl, heteroaryl, heterocyclyl, heteroarylalkyl, heterocyclylalkyl, and S(O)₂R₇ wherein R₇ is alkyl, alkenyl, alkynyl, cycloalkyl, aryl, aralkyl, heteroaryl, heterocyclyl, heterocyclylalkyl, heteroarylalkyl, and optionally substituted amino) to give a compound of Formula XXIII.

10. A method of making a compound of Formula XXV 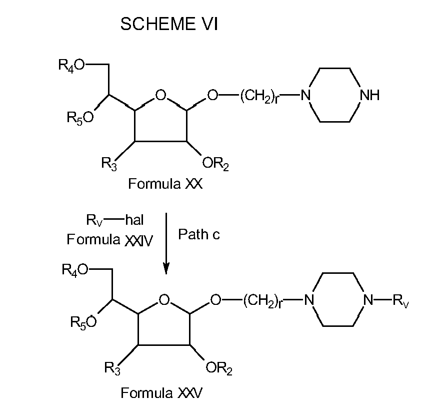 wherein
R₂ and R₃ form an acetal, wherein the carbon linking the two oxygens is substituted with R_{L} and Rₘ (wherein R_{L} and Rₘ are independently selected from hydrogen, alkyl, alkenyl, alkynyl, cycloalkyl, aryl, or aralkyl; or R_{L} and Rₘ can together join to form a 3-8 membered ring, wherein the ring may optionally contain one or more heteroatoms selected from O, N or S, and the ring may be optionally substituted with one or more of alkyl, alkenyl, alkynyl, amino, substituted amino, cycloalkyl, oxo, hydroxy, carboxy, -COQR₆ (wherein Q is O or NH and R₆ is selected from alkyl, alkenyl, alkynyl, aryl, aralkyl, and cycloalkyl), alkoxy, aryloxy, halogen (F,Cl, Br, I), aryl, aralkyl, heteroaryl, heterocyclyl, heteroarylalkyl, or heterocyclylalkyl; or R_{L} and Rₘ can together join to form an oxo group);
R₃ is -(CH₂)ₙG wherein n is an integer from 0-5 and G is ORₑ {wherein Rₑ is selected from
a) acyl (with the proviso that n cannot be 0), and
b) -C(=O)NR_{f}R_{q} [wherein R_{f} and Rq can be independently selected from hydrogen, hydroxy (with the restriction that both R_{f} and Rq cannot both be hydroxy), alkyl, alkenyl, alkynyl, cycloalkyl, aryl, aralkyl, heteroaryl, heterocyclyl, and S(O)₂R₇ (wherein R₇ is selected from alkyl, alkenyl, alkynyl, cycloalkyl, aryl, aralkyl, heteroaryl, heterocyclyl, heterocyclylalkyl, heteroarylalkyl, and optionally substituted amino)]; and R_{f} and R_{q} may also together join to form a heterocyclyl ring; also, when n is zero, then R_{f} and Rq cannot be hydrogen, alkyl, alkenyl, alkynyl, cycloalkyl and R_{f} and Rq together cannot join to form a heterocyclyl ring}; and
R₄ and R₅ are independently selected from hydrogen, lower (C₁-C₆) alkyl, lower (C₂-C₆) alkenyl, lower (C₂-C₆) alkynyl, lower (C₃-C₈) cycloalkyl, aryl, acyl, heterocyclyl, heteroaryl, lower (C₁-C₄) heterocyclylalkyl, and lower (C₁-C₄) heteroarylalkyl; or R₄ and R₅ may together form isopropylidene acetal,
the method comprising
reacting a compound of Formula XX with a compound of Formula XXIV (wherein R_{y} is alkyl and hal is halogen) to form a compound of Formula XXV.

11. A method of making a compound of Formula XXVIII 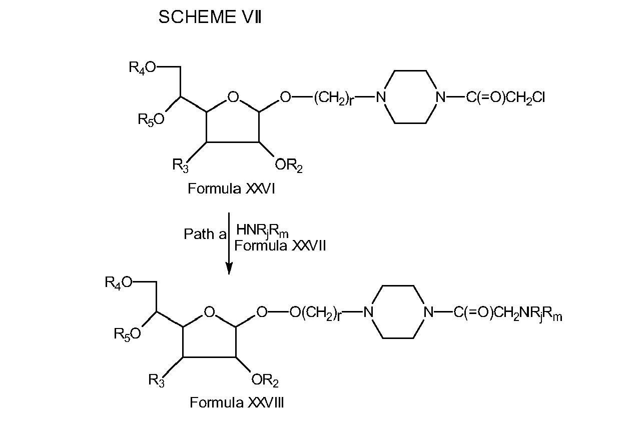 wherein
R₂ and R₃ form an acetal, wherein the carbon linking the two oxygens is substituted with R_{L} and Rₘ (wherein R_{L} and Rₘ are independently selected from hydrogen, alkyl, alkenyl, alkynyl, cycloalkyl, aryl, or aralkyl; or R_{L} and Rₘ can together join to form a 3-8 membered ring, wherein the ring may optionally contain one or more heteroatoms selected from O, N or S, and the ring may be optionally substituted with one or more of alkyl, alkenyl, alkynyl, amino, substituted amino, cycloalkyl, oxo, hydroxy, carboxy, -COQR₆ (wherein Q is O or NH and R₆ is selected from alkyl, alkenyl, alkynyl, aryl, aralkyl, and cycloalkyl), alkoxy, aryloxy, halogen (F,Cl, Br, I), aryl, aralkyl, heteroaryl, heterocyclyl, heteroarylalkyl, or heterocyclylalkyl; or R_{L} and Rₘ can together join to form an oxo group);
R₃ is -(CH₂)ₙG wherein n is an integer from 0-5 and G is ORₑ {wherein Rₑ is selected from
a) acyl (with the proviso that n cannot be 0), and
b) -C(=O)NR_{f}R_{q} [wherein R_{f} and R_{q} can be independently selected from hydrogen, hydroxy (with the restriction that both R_{f} and R_{q} cannot both be hydroxy), alkyl, alkenyl, alkynyl, cycloalkyl, aryl, aralkyl, heteroaryl, heterocyclyl, and S(O)₂R₇ (wherein R₇ is selected from alkyl, alkenyl, alkynyl, cycloalkyl, aryl, aralkyl, heteroaryl, heterocyclyl, heterocyclylalkyl, heteroarylalkyl, and optionally substituted amino)]; and R_{f} and Rqmay also together join to form a heterocyclyl ring; also, when n is zero, then R_{f} and R_{q} cannot be hydrogen, alkyl, alkenyl, alkynyl, cycloalkyl and R_{f} and Rq together cannot join to form a heterocyclyl ring}; and
R₄ and R₅ are independently selected from hydrogen, lower (C₁-C₆) alkyl, lower (C₂-C₆) alkenyl, lower (C₂-C₆) alkynyl, lower (C₃-C₈) cycloalkyl, aryl, acyl, heterocyclyl, heteroaryl, lower (C₁-C₄) heterocyclylalkyl, and lower (C₁-C₄) heteroarylalkyl; or R₄ and R₅ may together form isopropylidene acetal,
the method comprising
reacting a compound of Formula XXVI (wherein r is an integer from 1-3) with a compound of Formula XXVII (wherein Rⱼ is selected from hydrogen, lower (C₁-C₆) alkyl, lower (C₂-C₆) alkenyl, lower (C₂-C₆) alkynyl, lower (C₃-C₆) cycloalkyl, aryl, heteroaryl (with the proviso that the heteroaryl ring is not linked through a heteroatom), aralkyl (C₁-C₄), heteroarylalkyl (C₁-C₄), and heterocyclylalkyl (C₁-C₄) and Rₘ is as defined above) to give a compound of Formula XXVIII.

12. A method of making a compound of Formula XXX 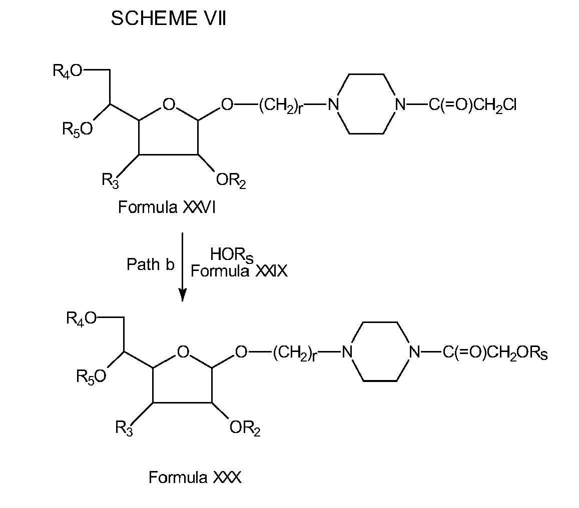 wherein
R₂ and R₃ form an acetal, wherein the carbon linking the two oxygens is substituted with R_{L} and Rₘ (wherein R_{L} and Rₘ are independently selected from hydrogen, alkyl, alkenyl, alkynyl, cycloalkyl, aryl, or aralkyl; or R_{L} and Rₘ can together join to form a 3-8 membered ring, wherein the ring may optionally contain one or more heteroatoms selected from O, N or S, and the ring may be optionally substituted with one or more of alkyl, alkenyl, alkynyl, amino, substituted amino, cycloalkyl, oxo, hydroxy, carboxy, -COQR₆ (wherein Q is O or NH and R₆ is selected from alkyl, alkenyl, alkynyl, aryl, aralkyl, and cycloalkyl), alkoxy, aryloxy, halogen (F,Cl, Br, I), aryl, aralkyl, heteroaryl, heterocyclyl, heteroarylalkyl, or heterocyclylalkyl; or R_{L} and Rₘ can together join to form an oxo group);
R₃ is -(CH₂)ₙG wherein n is an integer from 0-5 and G is ORₑ {wherein Rₑ is selected from
a) acyl (with the proviso that n cannot be 0), and
b) -C(=O)NR_{f}R_{q} [wherein R_{f} and Rq can be independently selected from hydrogen, hydroxy (with the restriction that both R_{f} and Rq cannot both be hydroxy), alkyl, alkenyl, alkynyl, cycloalkyl, aryl, aralkyl, heteroaryl, heterocyclyl, and S(O)₂R₇ (wherein R₇ is selected from alkyl, alkenyl, alkynyl, cycloalkyl, aryl, aralkyl, heteroaryl, heterocyclyl, heterocyclylalkyl, heteroarylalkyl, and optionally substituted amino)]; and R_{f} and Rq may also together join to form a heterocyclyl ring; also, when n is zero, then R_{f} and Rq cannot be hydrogen, alkyl, alkenyl, alkynyl, cycloalkyl and R_{f} and Rq together cannot join to form a heterocyclyl ring}; and
R₄ and R₅ are independently selected from hydrogen, lower (C₁-C₆) alkyl, lower (C₂-C₆) alkenyl, lower (C₂-C₆) alkynyl, lower (C₃-C₈) cycloalkyl, aryl, acyl, heterocyclyl, heteroaryl, lower (C₁-C₄) heterocyclylalkyl, and lower (C₁-C₄) heteroarylalkyl; or R₄ and R₅ may together form isopropylidene acetal,
the method comprising
reacting a compound of Formula XXVI (wherein r is an integer from 1-3) with a compound of Formula XXIX (wherein Rs is selected from alkyl, alkenyl, alkynyl, cycloalkyl, aryl, aralkyl, heterocyclylalkyl, or heteroarylalkyl) to form a compound of Formula XXX.

13. A method of making a compound of Formula XXXII 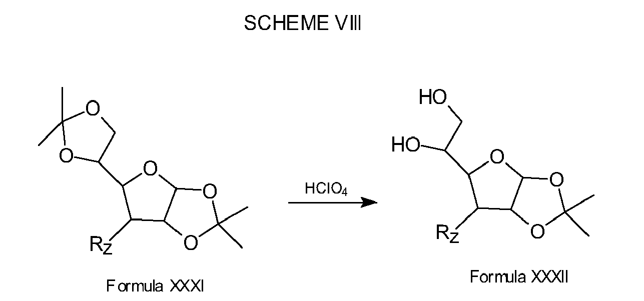 wherein R_{z} is hydrogen, alkyl, aralkyl, or heteroaryl alkyl,
the method comprising
reacting a compound of Formula XXXI with perchloric acid to form a compound of Formula XXXII.

14. A method of making a compound of Formula XXXIV 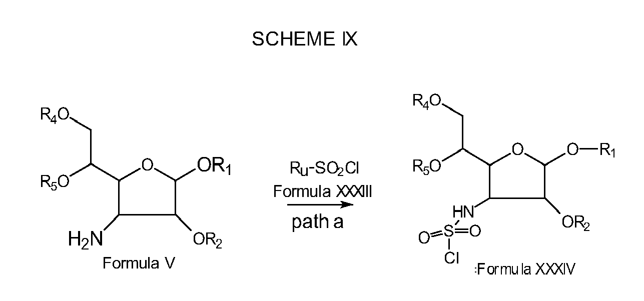 wherein
R₁ and R₂ together form a five-membered acetal, wherein the carbon atom joining the oxygens can be substituted with R_{L} and Rₘ [wherein R_{L} and Rₘ are independently selected from hydrogen, alkyl, alkenyl, alkynyl, cycloalkyl, aryl, or aralkyl; or R_{L} and Rₘ can together join to form a 3-8 membered ring, wherein the ring may optionally contain one or more heteroatoms selected from O, N or S, and the ring may be optionally substituted with one or more of alkyl, alkenyl, alkynyl, amino, substituted amino, cycloalkyl, oxo, hydroxy, carboxy, -COQR₆ (wherein Q is O or NH and R₆ is selected from alkyl, alkenyl, alkynyl, aryl, aralkyl, and cycloalkyl), alkoxy, aryloxy, halogen (F,Cl, Br, I), aryl, aralkyl, heteroaryl, heterocyclyl, heteroarylalkyl, or heterocyclylalkyl; or R_{L} and Rₘ can together join to form an oxo group]; and
R₄ and R₅ are independently selected from hydrogen, lower (C₁-C₆) alkyl, lower (C₂-C₆) alkenyl, lower (C₂-C₆) alkynyl, lower (C₃-C₈) cycloalkyl, aryl, acyl, heterocyclyl, heteroaryl, lower (C₁-C₄) heterocyclylalkyl, and lower (C₁-C₄) heteroarylalkyl; or R₄ and R₅ may together form a five-membered acetal wherein the carbon linking the two oxygens is substituted with R_{L} and Rₘ (wherein R_{L} and Rₘ are the same as defined earlier),
the method comprising
reacting a compound of Formula V with a compound of Formula XXXIII (wherein Ru is selected from alkyl, alkenyl, alkynyl, cycloalkyl, aryl, aralkyl, heteroaryl, heterocyclyl, heteroarylalkyl, and heterocyclylalkyl) to form a compound of Formula XXXIV.

15. A method of making a compound of Formula XXXVI 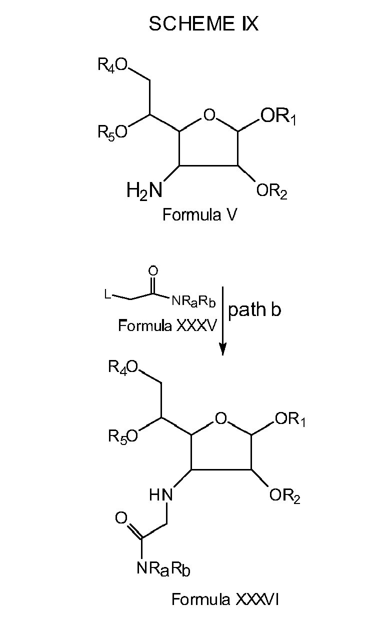 wherein
R₁ and R₂ together form a five-membered acetal, wherein the carbon atom joining the oxygens can be substituted with R_{L} and Rₘ [wherein R_{L} and Rₘ are independently selected from hydrogen, alkyl, alkenyl, alkynyl, cycloalkyl, aryl, or aralkyl; or R_{L} and Rₘ can together join to form a 3-8 membered ring, wherein the ring may optionally contain one or more heteroatoms selected from O, N or S, and the ring may be optionally substituted with one or more of alkyl, alkenyl, alkynyl, amino, substituted amino, cycloalkyl, oxo, hydroxy, carboxy, -COQR₆ (wherein Q is O or NH and R₆ is selected from alkyl, alkenyl, alkynyl, aryl, aralkyl, and cycloalkyl), alkoxy, aryloxy, halogen (F,Cl, Br, I), aryl, aralkyl, heteroaryl, heterocyclyl, heteroarylalkyl, or heterocyclylalkyl; or R_{L} and Rₘ can together join to form an oxo group]; and
R₄ and R₅ are independently selected from hydrogen, lower (C₁-C₆) alkyl, lower (C₂-C₆) alkenyl, lower (C₂-C₆) alkynyl, lower (C₃-C₈) cycloalkyl, aryl, acyl, heterocyclyl, heteroaryl, lower (C₁-C₄) heterocyclylalkyl, and lower (C₁-C₄) heteroarylalkyl; or R₄ and R₅ may together form a five-membered acetal wherein the carbon linking the two oxygens is substituted with R_{L} and Rₘ (wherein R_{L} and Rₘ are the same as defined earlier),
the method comprising
reacting a compound of Formula V with a compound of Formula XXXV (wherein L is a leaving group, and Rₐ and R_{b} together with the nitrogen atom carrying them are the N-terminus of an amino acid or di-tetrapeptide or Rₐ and R_{b} together join to form a heterocyclyl ring) to form a compound of Formula XXXVI.

16. A method of making compounds of Formula XXXIX 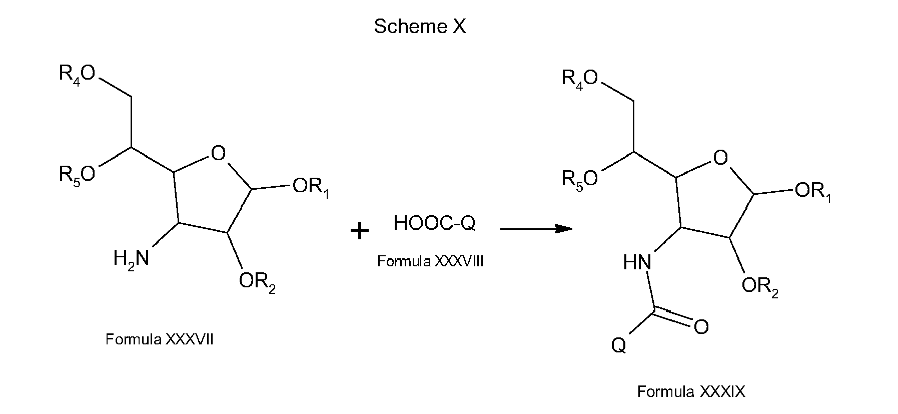 wherein
R₁ and R₂ together form a five-membered acetal, wherein the carbon atom joining the oxygens can be substituted with R_{L} and Rₘ [wherein R_{L} and Rₘ are independently selected from hydrogen, alkyl, alkenyl, alkynyl, cycloalkyl, aryl, or aralkyl; or R_{L} and Rₘ can together join to form a 3-8 membered ring, wherein the ring may optionally contain one or more heteroatoms selected from O, N or S, and the ring may be optionally substituted with one or more of alkyl, alkenyl, alkynyl, amino, substituted amino, cycloalkyl, oxo, hydroxy, carboxy, -COQR₆ (wherein Q is O or NH and R₆ is selected from alkyl, alkenyl, alkynyl, aryl, aralkyl, and cycloalkyl), alkoxy, aryloxy, halogen (F,Cl, Br, I), aryl, aralkyl, heteroaryl, heterocyclyl, heteroarylalkyl, or heterocyclylalkyl; or R_{L} and Rₘ can together join to form an oxo group]; and
R₄ and R₅ are independently selected from hydrogen, lower (C₁-C₆) alkyl, lower (C₂-C₆) alkenyl, lower (C₂-C₆) alkynyl, lower (C₃-C₈) cycloalkyl, aryl, acyl, heterocyclyl, heteroaryl, lower (C₁-C₄) heterocyclylalkyl, and lower (C₁-C₄) heteroarylalkyl; or R₄ and R₅ may together form a five-membered acetal wherein the carbon linking the two oxygens is substituted with R_{L} and Rₘ (wherein R_{L} and Rₘ are the same as defined earlier),
Q is substituted alkyl;
the method comprising
reacting a compound of Formula XXXVII with a compound of Formula XXXVIII to give a compound of Formula XXXIX.

17. A pharmaceutical composition comprising a compound of claim 1 and a pharmaceutically acceptable carrier.

18. The use of compounds according to claim 1 for the manufacture of medicament for treating or preventing inflammation, cardiovascular, cancer or autoimmune diseases in mammal.

19. The use of compounds according to claim 1 for the manufacture of medicament for treating or preventing bronchial asthma, chronic obstructive pulmonary disorder, rheumatoid arthritis, type I diabetes, multiple sclerosis, allograft rejection, cancer, inflammatory bowel disease, ulcerative colitis, psoriasis, acne, atherosclerosis, pruritis, allergic rhinitis in mammal.

20. The use of compounds according to claim 1 for the manufacture of medicament for treating or preventing disease or disorder which is mediated through 5-lipooxygenase in mammal.

21. The use of pharmaceutical composition according to claim 17 for the manufacture of medicament for treating or preventing inflammation, cardiovascular, cancer or autoimmune diseases in mammal.

22. The use of pharmaceutical composition according to claim 17 for the manufacture of medicament for treating or preventing bronchial asthma, chronic obstructive pulmonary disorder, rheumatoid arthritis, type I diabetes, multiple sclerosis, allograft rejection, cancer, inflammatory bowel disease, ulcerative colitis, psoriasis, acne, atherosclerosis, pruritis, allergic rhinitis in mammal.

23. The use of pharmaceutical composition according to claim 17 for the manufacture of medicament for treating or preventing disease or disorder which is mediated through 5-lipooxygenase in mammal.
